# EUROPEAN PATENT SPECIFICATION

(11) **EP 1 454 921 B1**
(45) Date of publication and mention of the grant of the patent: **28.07.2021**
(21) Application number: 02779885.9
(22) Date of filing: 20.09.2002
(51) Int. Cl.: A61K 6/30, C09J 4/00, C08F 4/68

(54) **RADICAL POLYMERIZATION CATALYSTS AND ADHESIVE KIT FOR DENTAL USE**
RADIKALPOLYMERISATIONSKATALYSATOREN UND KLEBEKIT FÜR DIE ZAHNÄRZTLICHE VERWENDUNG
CATALYSEURS DE POLYMERISATION RADICALAIRE ET ENSEMBLE ADHESIF D'USAGE DENTAIRE

(30) Priority: 21.09.2001 JP 2001289846; 23.05.2002 JP 2002148921
(43) Date of publication of application: 08.09.2004
(73) Proprietor: Tokuyama Corporation, Shunan-shi, Yamaguchi-ken 745-8648 (JP); Tokuyama Dental Corporation, Tokyo 110-0016 (JP)
(72) Inventor: OGURI, Makoto, c/o Tokuyama Dental Corporation, Tsukuba-shi, Ibaraki 300-4247 (JP); KAZAMA, Hideki, c/o Tokuyama Dental Corporation, Tsukuba-shi, Ibaraki 300-4247 (JP); KIMURA, Mikio, c/o Tokuyama Dental Corporation, Tsukuba-shi, Ibaraki 300-4247 (JP); IBARAGI, Kazuya, c/o Tokuyama Dental Corporation, Tsukuba-shi, Ibaraki 300-4247 (JP); FUZINAMI Kyouichi, c/o Tokuyama Dental Corporation, Tsukuba-shi, Ibaraki 300-4247 (JP); SATOH, Takeshi, c/o Tokuyama Dental Corporation, Tsukuba-shi, Ibaraki 300-4247 (JP)
(74) Representative: Hoffmann Eitle
(86) International application number: PCT/JP2002/009708
(87) International publication number: WO 2003/027153

(56) References cited:
- EP-A2- 0 415 704
- WO-A1-00/47586
- WO-A2-00/35968
- JP-A- 9 227 325
- JP-A- 9 227 325
- JP-A- 9 309 811
- JP-A- 9 309 811
- JP-A- 2000 169 535
- JP-A- 2000 169 535
- JP-A- 2000 187 322
- JP-A- 2001 011 113
- JP-A- 2002 187 907
- US-A- 5 866 631
- US-A- 6 133 338

## Description

### Technical Field

The present invention relates to a radical polymerization catalyst suitably used in e.g. paints, inks and medical materials, particularly in dental materials, as well as to compositions using the radical polymerization catalyst, such as curable composition.

The above compositions include dental adhesive materials used in dental treatments for adhering a dental restorative material (which is composed of, for example, a metal, an organic polymer substance and a ceramic, or a composite material thereof) to teeth.

### Background Art

Azo compounds and peroxides are generally known as radical polymerization catalysts (also referred to as radical polymerization initiators) for curing a radical-polymerizable monomer. For example, a radical polymerization catalyst comprising an organic peroxide, a vanadium compound, an acidic phosphoric acid ester and α-hydroxycarbonyl compound is disclosed in JP-A-9-53051.

Further, US 5,866,631 provides a dental primer composition capable of obtaining high adhesive strength to both dentin and enamel, as a pretreatment material for a chemically polymerizable adhesive. This composition is a dental primer composition comprising a polymerizable monomer containing an acidic group, water, aryl borate and transition metal compound.

Radical polymerization catalysts are in wide used also in dental field. Specifically explaining, they are compounded in dental adhesives, composite resins, self-curing dental acryric resins and dental pretreatment agents.

In these curable compositions, an appropriate radical polymerization catalyst is selected and used so as to match the formulations, application purposes and required properties of the compositions. Radical polymerization catalysts include a photopolymerization catalyst which generates a radical when exposed to a light, and a chemical polymerization catalyst used in chemical reactions represented by redox reaction. Use of a chemical polymerization catalyst is necessary when a curable composition contains a large amount of, for example, a filler where light transmission is difficult, or when a curable composition is used in an application where light irradiation is difficult. For a curable composition to be polymerized and cured sufficiently even in the innermost portion, the light amount (light intensity) supplied from a light irradiator needs be sufficient. However, there is a case when the light amount from the light irradiator decreases owing to, for example, the deterioration of the irradiator. In such a case, the decrease in light amount is difficult to know unless the light amount is measured by the use of, for example, a special actinometer exclusively used for the purpose. There is a case when the exact light amount supplied from a light irradiator is insufficient even if the light irradiation by the operator seems sufficient, making insufficient the curing of a curable composition. Further, there is a case when the polymerization catalyst compounded in a dental adhesive or a dental pretreatment agent need be a chemical polymerization catalyst depending upon the kind of the dental material to be adhered. Furthermore, to cure a curable composition using a photopolymerization catalyst, light irradiation need be made using a special light irradiator exclusively used for the purpose, which is inconvenient. Therefore, use of a chemical polymerization catalyst is desired in some cases for simplified operation.

For restoration of a tooth which has been damaged owing to, for example, caries or accident, there is a restoration method (a direct restoration method) which comprises filling a dental restorative material directly in the cavity of the tooth to make a shape and then curing the restorative material. The dental restorative material is a paste typified by a composite resin or a compomer.

In this restoration method, the dental restorative material is adhered to a tooth which has been subjected to a required pretreatment, using an adhesive ordinarily called a bonding agent (a dental adhesive for direct restoration) composed mainly of a radical-polymerizable monomer (this includes an acidic group-containing radical-polymerizable monomer).

In recent years, there has been developed, as the bonding agent, a material needing no pretreatment operation and enabling a simplified operation (for example, JP-A-9-263604 and JP-A-10-245525). However, this bonding agent contains a particular photopolymerization catalyst; when this bonding agent is used in combination with a dental restorative material of chemical polymerization type, the unreacted acidic group-containing radical-polymerizable monomer which remains in the bonding agent, reacts with an amine component in an organic peroxide/the tertiary amine type catalyst, ordinarily used as chemical polymerization catalyst components in the dental restorative material. As a result, the adhesion strength between the tooth to be repaired and the dental restorative material containing a chemical polymerization catalyst becomes insufficient. Therefore, there is a problem that the dental restorative material usable in combination with a bonding agent containing a photopolymerization catalyst is limited to a photo-curing type. Further, it is required to omit the operation of light irradiation to simplify the adhesion operation.

There is a restoration method (an indirect restoration method) which comprises adhering to a tooth a dental repair material such as inlay or crown, which has beforehand been produced outside a mouth made of e.g. a metal or a ceramic material. In this restoration method, there is used, as an adhesive, a dental adhesive cement such as CR (composite resin) type resin cement or resin-reinforced type glass ionomer cement, which is composed mainly of a radical-polymerizable monomer (this includes an acidic group-containing radical-polymerizable monomer) and an organic or inorganic filler. These cements ordinarily contain a large amount of a filler and accordingly are low in light transmissibility and there are cases when no light reaches the innermost portion of the cement applied that the innermost portion is not cured. Further, the inlay, the crown may be made of e.g. a light-impermeable metal material and, in such cases, the cement is not cured even by light irradiation. Thus, when a cement is used, it is often impossible to use a photopolymerization catalyst and it is necessary to use a chemical polymerization catalyst which is polymerizable in the dark at ambient temperature.

As to the chemical polymerization catalyst, various proposals have been made. There are currently known, for example, (1) a system using a trialkyl boron or a partial oxidation product thereof (for example, JP-A-57-108102), (2) a redox type self-curing catalyst, for example, a system which is a combination of an organic peroxide and a cobalt salt or a manganese salt, a system which is a combination of an organic peroxide and a tertiary amine (for example, JP-A-51-92884), or a system which is a combination of hydrogen peroxide and a Fe²⁺ compound, (3) a system composed of barbituric acid, a Cu²⁺ compound and a Cl⁻ compound (JP-A-5-295013) and (4) a chemical polymerization catalyst using an aryl borate compound, for example, a system which is a combination of an aryl borate compound and an acidic compound (JP-A-9-309811), or a system which is a combination of an aryl borate compound, an acidic compound and a transition metal compound (JP-A-9-227325). Some of them have been put into practical use.

In the previously-mentioned radical polymerization catalyst system comprising an organic peroxide, a vanadium compound, an acidic phosphoric acid ester and an α-hydroxycarbonyl compound, the vanadium compound need be used in a large amount for the system to exhibit a sufficient polymerization activity when used in dental applications. A dental adhesive containing such a catalyst, however, has a problem of low storage stability.

The above-mentioned system (1) using a trialkyl boron or a partial oxidation product thereof has a very high polymerization activity and is excellent as a chemical polymerization catalyst compounded in a dental adhesive. However, being extremely unstable chemically, the system need be stored in a package separately from other components and taken out by an appropriate amount right before the use, for mixing with other components such as monomer. This incurs a complicated operation.

Of the catalyst systems (2) and (3), the chemical polymerization catalyst which is a combination of an organic peroxide and a tertiary amine and the barbituric acid type chemical polymerization catalyst are being used most generally in dental materials for the low harm to living body and availability. However, the following problems are being pointed out for each of these chemical polymerization catalysts.

That is, for the chemical polymerization catalyst which is a combination of an organic peroxide and a tertiary amine, there are pointed out a problem of the initial color or discoloration of a cured material caused by the oxidation of the amine compound, and a serious problem of polymerization inhibition caused by oxygen or acidic component (the acidic component reacts with the tertiary amine to form a quaternary salt having no reducing power). The problem of the initial color or discoloration incurs, for example in the case of, a dental repair material typified by a composite resin, color difference between natural teeth and cured restorative material, which gives a reduced appearance. The problem of polymerization inhibition means no exhibition of sufficient polymerization activity, in polymerization of, for example, a dental adhesive composed of an acidic group-containing radical-polymerizable monomer. For the barbituric acid type chemical polymerization catalyst, there are being pointed out problems of, for example, difficulty of curing time control and low storage stability in dental adhesive.

The chemical polymerization catalyst system (4) using an aryl borate is easy to handle, shows no initial color or discoloration of cured material, and has excellent storage stability. However, its polymerization activity is not fully satisfactory and a higher polymerization activity is required.

As mentioned previously, a restorative material of filling type (which is ordinarily called a composite resin) is in use for restoration of teeth damaged owing to e.g. caries. Particularly, for initial or middle stage caries having a relatively small cavity, direct restoration using a photo-curing type composite resin which has a good appearance and enables simple and rapid operation, occupies a very important position.

In restoration of caries, adhesivity between the tooth to be restored and the restorative material used, such as composite resin is very important. Since the composite resin per se has no adhesivity, an adhesive is ordinarily used in adhesion of the tooth and the restorative material. As such an adhesive, a photo-curing type adhesive is used ordinarily because a high adhesivity is obtainable, curing can be made at any desired timing and the operation is simple.

However, since teeth is constituted by an enamel composed mostly of inorganic substances and a dentin high in contents of organic substances and water, it is ordinarily difficult for the adhesive alone to show a sufficient adhesion strength to both the enamel and the dentin. Therefore, a pretreatment of tooth using a pretreatment agent is conducted prior to the application of the adhesive.

As the pretreatment, there has been conducted a total three-steps treatment, i.e. two-steps treatment of applying of aqueous acid solution to tooth/water washing/drying and applying of primer/drying and subsequent application of adhesive. This three-steps treatment operation is complicated and moreover is likely to invite operational mistakes, and a simplified operation has been required. To respond to this requirement, there have been proposed, in recent years, various simplified treatment systems (hereinafter referred to also as two-steps treatment systems) wherein an acidic group-containing radical-polymerizable monomer (called adhesive monomer) is compounded in a primer to omit applying of aqueous acid solution/water washing/drying; and some of these systems have been put into practical application. In such adhesion systems, it is conducted in some cases to compound an acidic group-containing radical-polymerizable monomer also in an adhesive to obtain a high adhesivity.

A high adhesivity is obtained in the above adhesion systems of two-steps treatment. However, requirements for accuracy and reliability are becoming increasingly high and a system capable of exhibiting an even higher adhesivity is being required.

To respond to such a requirement, the present inventors proposed, in JP-A-2001-14779, (1) a pretreatment agent which contains an acid group-containing radical-polymerizable monomer, an aryl borate compound, an organic peroxide, water and, as necessary, a metal compound but contains no amine compound. Further, the present inventors found out that a high adhesivity to tooth is exhibited by an adhesive kit constituted by a pretreatment agent containing an acidic group-containing radical-polymerizable monomer and water and an adhesive containing an acidic group-containing radical-polymerizable monomer and an aryl borate compound, wherein a + tetravalent and/or + pentavalent vanadium compound is compounded in either of the pretreatment agent and the adhesive; and this adhesive kit was proposed in JP-A-2002-289846.

In the pretreatment material (1), the acidic group-containing radical-polymerizable monomer and the aryl borate compound are essential; however, since the aryl borate compound is decomposed very easily by an acid, the two substances are packed separately in respective packages so that they come into no contact, until right before their use.

Also in the adhesive kit (2), the adhesive is packed in two packages for the same reason. In the pretreatment agent and adhesive kit (system), a mixing operation is necessary for the pretreatment agent or the adhesive, right before their use. Therefore, they are not satisfactory for the ever-increasing requirement for simpler operation.

In restoration of teeth having heavy damage such as crown disintegration, a treatment called abutment building is conducted in some cases (this is not conducted for initial or middle stage caries having a relatively small cavity). As a material for this abutment building, there is being increasingly used a composite resin which hardly invites breakage of root of tooth and which has a good appearance.

In the abutment building, however, the composite resin is often applied to a site which a light is difficult to reach, such as root of tooth. Therefore, the adhesive used is highly required to cure sufficiently with no light irradiation and give a high adhesion strength. Hence, a chemical curing type adhesive is preferred as the adhesive used for abutment building.

The chemical curing type adhesive begins curing by mixing two or more components with each other. Therefore, in the chemical curing type adhesive, unlike in the above-mentioned photo-curing type adhesive, it is necessary that the adhesive components are packed in two or more packages and are mixed with each other right before the use.

In actual dental treatment, a photopolymerization type adhesive kit of easy operation is required for initial or middle stage caries having a relatively small cavity, and a chemical polymerization type adhesive kit needing no light irradiation for curing is required for abutment building.

As described above, the photopolymerization type adhesive kit is constituted by a pretreatment agent consisting ordinarily of two packages and a photopolymerization type adhesive consisting of one package. Also, the chemical polymerization type adhesive kit is constituted by a pretreatment agent consisting ordinarily of two packages and a chemical polymerization type adhesive consisting of two packages. This necessitates management of at least total seven packages in dental treatment.

However, purchase or storage of kits constituted by such many packages is extremely complicated. Further, different kits may be needed for different dental cases and it may happen that a wrong adhesive kit or wrong packages are selected to use in dental treatment.

Hence, there is required a photopolymerization type adhesive kit which enables a two-steps treatment and wherein each of the pretreatment agent and the adhesive can be stably stored in one package, adhesion is made effective by photopolymerization, and operation is simple. It is further required to use common packages in the photopolymerization type adhesive kit and the chemical polymerization type adhesive kit.

### Disclosure of the Invention

The present inventors made an intensive study to achieve the above-mentioned requirements. As a result, the present inventors found out that a combination of an aryl borate compound, an acidic compound and a particular vanadium compound, when used as a polymerization catalyst, has a strikingly high polymerization activity and that a curable composition containing such a radical polymerization catalyst has excellent characteristics as a dental adhesive, a dental pretreatment agent and a dental restorative material. The present invention has been completed based on the above finding.

That is, the present invention provides a dental adhesive containing a chemical radical polymerization catalyst comprising
(i) an aryl borate compound,
(ii) a (+)-tetra- and/or -pentavalent vanadium compound selected from divanadium tetroxide (tetravalent), vanadium oxide acetylacetonate (tetravalent), vanadyl oxalate (tetravalent), vanadyl sulfate (tetravalent), oxobis(1-phenyl-1,3-butanedionate) vanadium (tetravalent), bis(maltolato) oxovanadium (tetravalent), vanadium pentoxide (pentavalent), sodium metavanadate (pentavalent) and ammonium metavanadate (pentavalent); and
(iii) an acidic group-containing radical-polymerizable monomer which, when present in form of a 10 wt.-% aqueous solution or suspension, has a pH of ≤ 4.5, and which is a compound of formula (2): 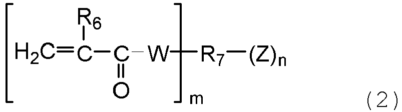 wherein
   - R⁶: is H or methyl;
   - R⁷: is a di- to hexavalent C₁₋₃₀-organic residue;
   - W: is O, S or NH;
   - Z: is -COOH, -SO₃H, -O-P(=O)(OH)₂, -P(=O)(OH)₂ or -O-P(=O)(OH)(OR⁸), wherein R⁸ is C₁₋₁₀-alkyl or aryl having 6-14 ring carbon atoms, and the alkyl group or the aryl group may be substituted by halogen, hydroxyl, cyano, nitro, C₁₋₅-alkyl, C₂₋₅-alkenyl, C₂₋₅-alkynyl, C₁₋₅-alkoxyl, C₂₋₅-acyl or C₂₋₅-acyloxy;
   - m: is an integer of 1-5;
   - n: is an integer of 1-5; and (m+n) corresponds to the valence of R⁷.

Also, the present invention provides a dental restorative material comprising the present dental adhesive and further (iv) a radical-polymerizable monomer not containing an acidic group; and (v) a filler; and further provides a dental pretreatment agent comprising the present dental adhesive and (vi) water.

Yet further, the present invention provides a dental adhesive kit constituted by
(1) a dental pretreatment agent containing an acidic group-containing radical-polymerizable monomer (iii) as defined above and (vi) water, and
(2) a dental adhesive containing (i) an aryl borate compound and an acidic group-containing radical-polymerizable monomer (iii) as defined above;
   and at least one of (1) and (2) contains a (+)-tetra- and/or -pentavalent vanadium compound (ii) as defined above.
   Even further, the present invention provides a dental adhesive kit constituted by
   (1') a pretreatment agent containing a (+)-tetra- and/or - pentavalent vanadium compound (ii) as defined above,
   an acidic group-containing radical-polymerizable monomer (iii) as defined above, and (vi) water, and
(3) an adhesive containing (i) an aryl borate compound,
   (iv) a radical-polymerizable monomer not containing an acidic group, (vii) an organic peroxide and (viii) a photopolymerization catalyst.

Preferred embodiments of the invention are as defined in the appended dependent claims and/or on the following detailed description.

The polymerization catalyst of the present invention has high chemical stability by itself, is easy to handle, has a high polymerization activity, is hardly susceptible to polymerization inhibition, and does not color or discolor the cured material obtained by polymerization

The object of the present invention is to provide a curable composition containing the above radical polymerization catalyst and various dental materials utilizing the radical polymerization catalyst.

The present inventors proposed, in JP-A-2001-14779, a dental pretreatment agent containing an acidic group-containing radical-polymerizable monomer, an aryl borate compound, an organic peroxide and, as a metal compound, a + tetravalent and/or + pentavalent vanadium compound. As a result of a further study, the present inventors came to an idea of a dental adhesive kit constituted by a pretreatment agent containing an acidic group-containing radical-polymerizable monomer and a + tetravalent and/or + pentavalent vanadium compound and an adhesive containing an aryl borate compound and an organic peroxide. This dental adhesive kit was found to give an adhesion strength equal to or higher than the case proposed in JP-A-2001-14779, where all these components are contained in a pretreatment agent. Thereby, it was learned that it is not necessary for a pretreatment agent to contain an aryl borate compound (which can not be stored together with an acid group-containing radical-polymerizable monomer in the same package). As a result, single packaging of a pretreatment agent was made possible and the second dental adhesive kit has been completed.

Also in the dental adhesive, it was found out that when there is used, as a chemical curing type polymerization catalyst, the above-mentioned system comprising an acidic group-containing radical-polymerizable monomer, an aryl borate compound, an organic peroxide, water and a metal compound and when there is used, as the metal compound, a + tetravalent and/or + pentavalent vanadium compound, there arises no problem caused by use of an amine compound. It was further found out that use of an amine compound in a pretreatment agent further increases the storage stability of a pretreatment agent of single packaging without decreasing the adhesion strength.

That is, the second dental adhesive kit is constituted by (A) a pretreatment agent containing an acidic group-containing radical-polymerizable monomer, a + tetravalent and/or + pentavalent vanadium compound and water and (B) an adhesive containing an acidic group-free radical-polymerizable monomer, an aryl borate compound, an organic peroxide and a photopolymerization catalyst.

In the second adhesive kit of the present invention, the adhesive (B) may further contain an acidic compound and a + tetravalent and/or + pentavalent vanadium compound.

Also in the second adhesive kit of the present invention, the adhesive (B) may consist of (B1) a package containing an acidic group-free radical-polymerizable monomer, an aryl borate compound, an organic peroxide and a photopolymerization catalyst and (B2) a package containing an acidic compound and a + tetravalent and/or + pentavalent vanadium compound.

In the above adhesive kit, the pretreatment agent may contain an amine compound.

### Best Mode for Carrying out the Invention

### (Radical polymerization catalyst)

The radical polymerization catalyst of the present invention comprises an aryl borate compound, an acidic compound and a + tetravalent and/or + pentavalent vanadium compound.

### Aryl borate compound

As to the aryl borate compound used in the present radical polymerization catalyst, there is no particular restriction as long as it is a compound having at least one boron-aryl bond in the molecule, and a known compound can be used. A borate compound having no boron-aryl bond is extremely inferior in stability, easily reacts with the oxygen in the air and decomposes, and therefore is unusable practically.

As the aryl borate compound, there are preferred, for the storage stability and polymerization activity, borate compounds of formula (1) 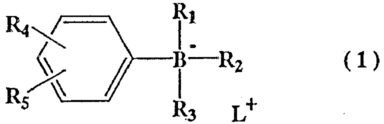
wherein R₁, R₂ and R₃ are each independently alkyl, aryl or alkenyl and each of these groups may have a substituent(s);
R₄ and R₅ are each independently H, halogen, nitro, optionally substituted alkyl or alkoxy, or optionally substituted phenyl; and L⁺ is a metal cation, a tertiary or quaternary ammonium ion, a quaternary pyridinium ion, a quaternary quinolinium ion or a quaternary phosphonium ion).

In the general formula (1), R₁, R₂ and R₃ are each independently alkyl, aryl or alkenyl and each of these groups may have a substituent(s).

As to the alkyl group, there is no particular restriction. It may be a straight chain or a branched chain but is preferably C₅₋₃₀-alkyl, more preferably straight chain C₄₋₂₀-alkyl g. Specifically, it can be exemplified by n-butyl grup, n-octyl, n-dodecyl and n-hexadecyl. The substituent of the alkyl group can be exemplified by halogen atoms such as F, Cl and Br; hydroxyl; nitro; cyano; C₆₋₁₀-aryl, such as phenyl, nitrophenyl and; C₁₋₅-alkoxy, such as methoxy, ethoxy and propoxy; and C₂₋₅-acyl, such as acetyl. There is no particular restriction, either, as to the number or position of the substituent.

As to the aryl group, there is no particular restriction, either. It may be a known aryl group but is preferably a substituted or unsubstituted aryl group having 6 to 14 carbon atoms [the carbon atoms exclude those of substituent(s)] which has a monocyclic structure or a two or three ring-condensed cyclic structure. As the substituent thereof, there can be mentioned the groups shown above as the substituents of the alkyl group, and C₁₋₅-alkyl such as methyl, ethyl and butyl.

As specific examples of the substituted or unsubstituted aryl group, there can be mentioned phenyl, 1- or 2-naphthyl, 1-, 2- or 9-anthryl, 1-, 2-, 3-, 4- or 9-phenanthryl, p-fluorophenyl, p-chlorophenyl, (3,5-bistrifluoromethyl)phenyl, 3,5-bis(1,1,1,3,3,3-hexafluoro-2-methoxy-2-propyl)phenyl, p-nitrophenyl, m-nitrophenyl, p-butylphenyl, m-butylphenyl, p-butyloxyphenyl, m-butyloxyphenyl, p-octyloxyphenyl and m-octyloxyphenyl.

The alkenyl group is not particularly restricted, either, but is preferably C₄₋₂₀-alkenyl, such as 3-hexenyl or 7-octenyl group. As the substituent thereof, there can be mentioned the groups shown above as the substituents of the alkyl group.

In the above general formula (1), R₄ and R₅ are each independently H, halogen, nitro, optionally substituted alkyl or alkoxy, or optionally substituted phenyl.

The optionally substituted alkyl or alkoxy group is not particularly restricted and may be a straight chain or a branched chain. It is preferably C₁₋₁₀-alkyl or C₁₋₁₀-alkoxy. As the substituent, there can be mentioned the groups shown above as the substituents of the alkyl groups represented by R₁-R₃. As specific examples of optionally substituted alkyl, there can be mentioned methyl, ethyl, n- or i-propyl, n-, i- or t-butyl, chloromethyl, trifluoromethyl, methoxymethyl and 1,1,1,3,3,3-hexafluoro-2-methoxy-2-propyl. As specific examples of optionally substituted alkoxy, there can be mentioned methoxy, ethoxy, 1- or 2-propoxy, 1- or 2-butoxy, 1-, 2- or 3-octyloxy and chloromethoxy.

The substituent bonding to the substituted or unsubstituted phenyl group is not particularly restricted, either. As specific examples thereof, there can be mentioned the groups shown as the substituents of the aryl groups represented by R₁-R₃.

In the above general formula (1), L⁺ is a metal cation, a tertiary or quaternary ammonium ion, a quaternary pyridinium ion, a quaternary quinolinium ion or a quaternary phosphonium ion.

The metal cation is preferably, for example, an alkali metal cation such as sodium, lithium or potassium ; or an alkaline earth metal cation such as magnesium . The tertiary or quaternary ammonium ion is exemplified by tetrabutylammonium, tetramethylammonium, tetraethylammonium, tributylammonium, triethanolammonium, p-tolyldimethylammonium, and ethoxycarbonylphenyldimethylammonium. The quaternary pyridinium ion is exemplified by methylquinolinium, ethyl quinolinium and butyl quinolinium. The quaternary phosphonium ion is exemplified by tetrabutylphosphonium and methyltriphenylphosphonium.

Specific examples of the aryl borate compound of formula (1) are shown below.

As the borate compound having one boron-aryl bond in the molecule, there can be mentioned, for example, sodium salt, lithium salt, potassium salt, magnesium salt, tetrabutylammonium salt, tetramethylammonium salt, tetraethylammonium salt, tributylammonium salt, triethanolammonium salt, dimethyl-p-toluidine salt, ethyl dimethylaminobenzoate salt, methylpyridinium salt, ethyl pyridinium salt, butylpyridinium salt, methylquinolinium salt, ethylquinolinium salt or butylquinolinium salt of trialkyl phenyl borate, trialkyl (p-chlorophenyl) borate, trialkyl (p-fluorophenyl) borate, trialkyl (3,5-bistrifluoromethyl)phenyl borate, trialkyl [3,5-bis(1,1,1,3,3,3-hexafluoro-2-methoxy-2-propyl)phenyl] borate, trialkyl (p-nitrophenyl) borate, trialkyl (m-nitrophenyl) borate, trialkyl (p-butylphenyl) borate, trialkyl (m-butylphenyl) borate, trialkyl (p-butyloxyphenyl) borate, trialkyl (m-butyloxyphenyl) borate, trialkyl (p-octyloxyphenyl) borate or trialkyl (m-octyloxyphenyl) borate (in each of these borate compounds, "alkyl" refers to either of n-butyl, n-octyl and n-dodecyl).

As the borate compound having two boron-aryl bonds in the molecule, there can be mentioned, for example, sodium salt, lithium salt, potassium salt, magnesium salt, tetrabutylammonium salt, tetramethylammonium salt, tetraethylammonium salt, tributylammonium salt, triethanolammonium salt, dimethyl-p-toluidine salt, ethyl dimethylaminobenzoate salt, methylpyridinium salt, ethyl pyridinium salt, butylpyridinium salt, methylquinolinium salt, ethylquinolinium salt or butylquinolinium salt of dialkyl diphenyl borate, dialkyl di(p-chlorophenyl) borate, dialkyl di(p-fluorophenyl) borate, dialkyl di(3,5-bistrifluoromethyl)phenyl borate, dialkyl di[3,5-bis(1,1,1,3,3,3-hexafluoro-2-methoxy-2-propyl)phenyl] borate, dialkyl (p-nitrophenyl) borate, dialkyl di(m-nitrophenyl) borate, dialkyl di(p-butylphenyl) borate, dialkyl di(m-butylphenyl) borate, dialkyl di(p-butyloxyphenyl) borate, dialkyl di(m-butyloxyphenyl) borate, dialkyl di(p-octyloxyphenyl) borate or dialkyl di(m-octyloxyphenyl) borate (in each of these borate compounds, "alkyl" refers to either of n-butyl, n-octyl and n-dodecyl).

As the borate compound having three boron-aryl bonds in the molecule, there can be mentioned, for example, sodium salt, lithium salt, potassium salt, magnesium salt, tetrabutylammonium salt, tetramethylammonium salt, tetraethylammonium salt, tributylammonium salt, triethanolammonium salt, dimethyl-p-toluidine salt, ethyl dimethylaminobenzoate salt, methylpyridinium salt, ethyl pyridinium salt, butylpyridinium salt, methylquinolinium salt, ethylquinolinium salt or butylquinolinium salt of monoalkyl triphenyl borate, monoalkyl tris(p-chlorophenyl) borate, monoalkyl tris(p-fluorophenyl) borate, monoalkyl tris(3,5-bistrifluoromethyl)phenyl borate, monoalkyl tris[3,5-bis(1,1,1,3,3,3-hexafluoro-2-methoxy-2-propyl)phenyl] borate, monoalkyl tris(p-nitrophenyl) borate, monoalkyl tris(m-nitrophenyl) borate, monoalkyl tris(p-butylphenyl) borate, monoalkyl tris(m-butylphenyl) borate, monoalkyl tris(p-butyloxyphenyl) borate, monoalkyl tris(m-butyloxyphenyl) borate, monoalkyl tris(p-octyloxyphenyl) borate or monoalkyl tris(m-octyloxyphenyl) borate (in each of these borate compounds, "alkyl" refers to either of n-butyl, n-octyl and n-dodecyl).

As the borate compound having four boron-aryl bonds in the molecule, there can be mentioned, for example, sodium salt, lithium salt, potassium salt, magnesium salt, tetrabutylammonium salt, tetramethylammonium salt, tetraethylammonium salt, tributylammonium salt, triethanolammonium salt, dimethyl-p-toluidine salt, dimethylaminobenzoate salt, methylpyridinium salt, ethyl pyridinium salt, butylpyridinium salt, methylquinolinium salt, ethylquinolinium salt or butylquinolinium salt of tetraphenyl borate, tetrakis(p-chlorophenyl) borate, tetrakis(p-fluorophenyl) borate, tetrakis(3,5-bistrifluoromethyl)phenyl borate, tetrakis[3,5-bis(1,1,1,3,3,3-hexafluoro-2-methoxy-2-propyl)phenyl] borate, tetrakis(p-nitrophenyl) borate, tetrakis(m-nitrophenyl) borate, tetrakis(p-butylphenyl) borate, tetrakis(m-butylphenyl) borate, tetrakis(p-butyloxyphenyl) borate, tetrakis(m-butyloxyphenyl) borate, tetrakis(p-octyloxyphenyl) borate or tetrakis(m-octyloxyphenyl) borate (in each of these borate compounds, "alkyl" refers to either of n-butyl, n-octyl and n-dodecyl).

Of these aryl borate compounds, preferred are aryl borate compounds having three or four boron-aryl bonds in the molecule, in view of the storage stability. More preferred are aryl borate compounds having four boron-aryl bonds in the molecule, from the standpoints of easy handling, easy synthesis and availability. Particularly preferred are aryl borate compounds of formula (1) wherein R₁, R₂, R₃ and the group of the formula 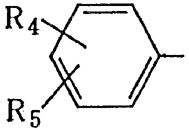 are the same, that is, a boron atom has four same aryl groups as the substituents.

As L+, preferred is a tertiary or quaternary ammonium ion and more preferred is a tertiary ammonium ion.

These aryl borate compounds may be used singly or in admixture of two or more kinds.

### Acidic compound

The acidic compound used in the present radical polymerization catalyst is mass% an acidic group-containing radical-polymerizable monomer (hereinafter may be referred to as "acidic monomer") of a general formula (2) described later herein.

### Vanadium compound

The third component of the present radical polymerization catalyst is a + tetravalent and/or + pentavalent vanadium compound. There are vanadium compounds whose oxidation numbers range from - mono-valency to + penta-valency. The vanadium compound used in the present invention has + tetra-valency or + penta-valency. Vanadium compounds of - mono-valency to + mono-valency are unstable. Vanadium compounds of + di-valency to + tri-valency have low polymerization activity and are unusable as a radical polymerization catalyst. The + tetravalent or + pentavalent vanadium compound is selected from divanadium tetroxide (tetravalent), vanadium oxide acetylacetonate (tetravalent), vanadyl oxalate (tetravalent), vanadyl sulfate (tetravalent), oxobis(1-phenyl-1,3-butanedionate) vanadium (tetravalent), bis(maltolato) oxovanadium (tetravalent), vanadium pentoxide (pentavalent), sodium metavanadate (pentavalent) and ammonium metavanadate (pentavalent).

These + tetravalent or + pentavalent vanadium compounds can be used in combination of a plurality of kinds.

When simply "vanadium compound" is mentioned hereinafter for convenience, it indicates a + tetravalent or + pentavalent vanadium compound unless otherwise defined.

In the present radical polymerization catalyst, the amounts of individual components used are not particularly restricted and may be any amounts as long as the radical polymerization catalyst produced function as such. Preferably, the amount of the acidic compound is 0.1-100 moles and the amount of the vanadium compound is 0.0005-5 moles, both per mole of the aryl borate compound, from the standpoints of, for example, the polymerization activity of the radical polymerization catalyst produced, the properties (e.g. strength) of the cured material obtained using the catalyst, and the risk of bleeding of the components not participating in polymerization. More preferably, the amount of the acidic compound is 0.5-50 moles and the amount of the vanadium compound is 0.001-1 mole, both per mole of the aryl borate compound.

The present radical polymerization catalyst is used to polymerize and cure a radical-polymerizable monomer. Because there is used an acidic monomer as the acidic compound, the acidic monomer per se is a radical-polymerizable monomer; therefore, a curable composition is obtained by simply mixing these three components. However, it is ordinarily preferred to further use, depending upon e.g. the application purpose and usage, a radical-polymerizable monomer other than the acidic monomer, i.e. an acidic group-free radical-polymerizable monomer.

### (Curable composition and Cured material)

Next, description is made on a curable composition containing the present radical polymerization catalyst (hereinafter, the composition is referred to as the present curable composition) and a cured material obtained by curing the curable composition (hereinafter, the material is referred to as the cured material).

As the acidic group-free radical-polymerizable monomer used in the present curable composition (hereinafter, the monomer is referred to as non-acidic monomer), a known radical-polymerizable monomer can be used with no restriction.

Specific examples of the acidic group-free radical-polymerizable monomer are shown below.

As the monomer having a (meth)acryloxy group as the polymerizable unsaturated group, there are (meth)acrylate type monomers having one polymerizable unsaturated group (hereinafter, these monomers are referred to also as non-acidic monofunctional monomers), such as methyl (meth)acrylate, ethyl (meth)acrylate, isopropyl (meth)acrylate, butyl (meth)acrylate, 2-ethylhexyl (meth)acrylate, glycidyl (meth)acrylate, benzyl (meth)acrylate, polyethylene glycol mono(meth)acrylate, allyl (meth)acrylate, 2-hydroxyethyl (meth)acrylate, 3-hydroxypropyl (meth)acrylate, glyceryl mono(meth)acrylate, tetrahydrofurfuryl (meth)acrylate, 2-(meth)acryloxyethyl propionate and 2-methacryloxyethyl acetoacetate; aliphatic (meth)acrylate type monomers having a plurality of polymerizable unsaturated groups, such as ethylene glycol di(meth)acrylate, diethylene glycol di(meth)acrylate, triethylene glycol di(meth)acrylate, butylene glycol di(meth)acrylate, nonaethylene glycol di(meth)acrylate, propylene glycol di(meth)acrylate, dipropylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, 1,9-nonanediol di(meth)acrylate, trimethylolpropane tri(meth)acrylate, neopentyl glycol di(meth)acrylate, pentaerythritol tri(meth)acrylate, trimethylolmethane tri(meth)acrylate, pentaerythritol tetra(meth)acrylate, 1,6-bis[(meth)acrylethyloxycarbonylamino]-2,2,4-trimethylhexane, 1,6-bis[(meth)acrylethyloxycarbonylamino]-2,4,4-trimethylhexane and urethane (meth)acrylate; and aromatic (meth)acrylate type monomers having a plurality of polymerizable unsaturated groups, such as 2,2-bis[(meth)acryloxyphenyl]propane, 2,2-bis[4-(2-hydroxy-3-(meth)acryloxypropoxy)phenyl]propane, 2,2-bis[(4-(meth)acryloxyethoxy)phenyl]propane, 2,2-bis[(4-(meth)acryloxydiethoxy)phenyl]propane, 2,2-bis[(4-(meth)acryloxypropoxy)phenyl]propane and 2,2-bis[(4-(meth)acryloxypolyethoxy)phenyl]propane (hereinafter, the aliphatic monomers and the aromatic monomers are together referred to also as non-acidic polyfunctional monomers.

As the monomer having a (meth)acrylamide group as the polymerizable unsaturated group, there are e.g. diacetone acrylamide and N-methylol (meth)acrylamide.

As the fumaric acid ester compound, there are e.g. diethyl fumarate and diphenyl fumarate.

As the styrene derivative, there are e.g. styrene, divinylbenzene and α-methylstyrene.

As the allyl compound, there are e.g. diallyl phthalate, diallyl terephthalate, diallyl carbonate and allyl diglycol carbonate.

There can be further mentioned vinyl acetate, 4-vinylpyridine, N-vinylpyrrolidone and ethyl vinyl ether.

These non-acidic monomers can be used singly or in admixture of two or more kinds.

In the present curable composition, of the above-mentioned radical-polymerizable monomers, a compound (a monomer) having, as the radical-polymerizable group, (meth)acryl or a group derived therefrom [e.g. (meth)acryloxy, (meth)acrylamide or (meth)acrylthio] is used, from the standpoints of the rate of curing and the properties (e.g. strength) of the cured material obtained, preferably in an amount of 50 mass% or more, more preferably in an amount of 60 mass% or more of the total radical-polymerizable monomers (when the acidic monomer is used, it is included therein; the same applies hereinafter).

In particular, a compound (a monomer) having a (meth)acryloxy group as the radical-polymerizable group is used preferably in an amount of 50 mass% or more, particularly preferably in an amount of 60 mass% or more of the total radical-polymerizable monomers.

In the curable composition, the ratio of the amount of the radical-polymerizable monomer and the amount of the radical polymerization catalyst is not particularly restricted as long as the ratio is such as the radical-polymerizable monomer is cured sufficiently. The aryl borate compound constituting the radical polymerization catalyst is used generally in an amount of 0.01-20 parts by mass(pbm), preferably in an amount of 0.1-10 pbm relative to 100 pbm of the total radical-polymerizable monomers, from the standpoints of the rate of curing and the properties (e.g. mechanical strength) of the cured material obtained.

The acidic compound and the vanadium compound, which are components of the radical polymerization catalyst other than the aryl borate compound, are used in the above-mentioned amounts relative to the amount of the aryl borate compound used.

The specific amounts of these components differ depending upon the radical-polymerizable monomer used and the molecular weights (or the formula weights) of the catalyst components. Generally, however, the amount of the aryl borate compound is 0.01-20 pbm (preferably 0.1-10 pbm) relative to 100 pbm of the total radical-polymerizable monomers; the amount of the vanadium compound is 0.00005-10 pbm (preferably 0.0005-1 pbm); when the acidic compound is other than the acidic monomer, the amount of the acidic compound is 0.001-20 pbm (preferably 0.01-10 pbm); when the acidic compound is an acidic monomer, the amount of the acidic monomer is 0.01-70 mass% (preferably 0.1-50 mass%) in the total radical-polymerizable monomers. Such a composition is suitable.

In the present curable composition, known additives can be compounded depending upon the purpose and application of the composition. As the additives, there can be mentioned, for example, a polymerization catalyst other than the present radical polymerization catalyst, a filler (e.g. an inorganic filler, an organic filler or an inorganic-organic composite filler), a thickening agent, a polymerization inhibitor, a polymerization-regulator, an ultraviolet absorber, a metal compound (e.g. a metal salt or a metal complex) other than + tetravalent or + pentavalent vanadium compound, water, an organic solvent, a dye and a pigment.

As the polymerization catalyst other than the present radical polymerization catalyst (this catalyst is hereinafter referred to as other polymerization catalyst),there can be used a known thermal polymerization catalyst, a known redox polymerization catalyst (this catalyst may be referred to as chemical polymerization catalyst), and a known photopolymerization catalyst, with no restriction.

As the thermal polymerization catalyst, there can be mentioned, for example, organic peroxides such as ketone peroxide, peroxyketal, hydroperoxide, dialkyl peroxide, peroxy ester, diacyl peroxide and peroxy dicarbonate; and azo compounds such as azobisisobutyronitrile.

As the thermal polymerization catalyst, organic peroxides are preferred from the standpoints of the polymerization activity and the low harm to living body; and particularly preferred are a hydroperoxide, a ketone peroxide, a peroxy ester and a diacyl peroxide. These organic peroxides are explained in detail in the second dental adhesive kit of the present invention described later. When such a thermal polymerization catalyst is added as a component of the present curable composition, there is preferably used one having a 10-hour half-life temperature of 60°C or more, from the standpoint of storage stability.

The most preferred thermal polymerization catalyst is e.g. benzoyl peroxide, cumene hydroperoxide and 1,1,3,3-tetramethylbutyl hydroperoxide.

It is also preferred to employ a redox catalyst by adding, together with the organic peroxide or the azo compound, an amine compound such as p-dimethylaminotoluidine, p-diethylaminotoluidine or p-diethanolaminotoluidine.

As the redox catalyst, there can also be used a barbituric acid type redox catalyst such as disclosed in JP-A-5-295013.

It is also preferred to use, in combination, a photopolymerization catalyst which can give rise to polymerization upon irradiation of an ultraviolet light or a visible light. When a photopolymerization catalyst is used in combination, there can be obtained a dual cure type curable composition which can give rise to any of chemical curing and photo-curing. By using a dual cure type curable composition, rapid curing is possible by light irradiation and, moreover, even the portion of insufficient light irradiation or of no light irradiation can be cured sufficiently.

As the photopolymerization catalyst, there can be mentioned α-diketones such as diacetyl, acetyl benzoyl, benzil, 2,3-pentadione, 2,3-octadione, 4,4'-dimethoxybenzil, 4,4'-oxybenzil, camphorquinone, 9,10-phenanthrenequinone and acenaphthenequinone; benzoin alkyl ethers such as benzoin methyl ether, benzoin ethyl ether and benzoin propyl ether; thioxanthone derivatives such as 2,4-diethoxythioxanthone, 2-chlorothioxanthone and methylthioxanthone; benzophenone derivatives such as benzophenone, p,p'-dimethylaminobenzophenone and p,p'-methoxybenzophenone; acylphosphine oxide derivatives such as 2,4,6-trimethylbenzoyldiphenylphosphine oxide, bis(2,6-dimethoxybenzoyl)-2,4,4-trimethylpentylphosphine oxide and bis(2,4,6-trimethylbenzoyl)phenylphosphine oxide; and a combination of an aryl borate compound (an essential component of the present invention), a dye and a photo-induced acid-generating agent, that is, a system consisting of an aryl borate compound, a dye and a photo-induced acid-generating agent. Of these, particularly preferred are an α-diketone type photopolymerization catalyst, an acylphosphine oxide type photopolymerization catalyst and a photopolymerization catalyst which is a system consisting of an aryl borate compound, a dye and a photo-induced acid-generating agent.

As the α-diketone, preferred are camphorquinone and benzil; as the acylphosphine oxide, preferred are 2,4,6-trimethylbenzoyldiphenylphosphine oxide, bis(2,6-dimethoxybenzoyl)-2,4,4-trimethylpentylphosphine oxide and bis(2,4,6-trimethylbenzoyl)phenylphosphine oxide. These α-diketones and acylphosphine oxides show a photopolymerization activity alone; however, their combination use with an amine compound such as ethyl 4-dimethylaminobenzoate, lauryl 4-dimethylaminobenzoate or dimethylaminoethyl methacrylate is preferred because it gives a higher polymerization activity.

As the photopolymerization catalyst which is a system consisting of an aryl borate compound, a dye and a photo-induced acid-generating agent, those catalysts described in JP-A-9-3109 can be used suitably. Specifically explaining, a catalyst using, as the dye, a coumarin type dye and, as the photo-induced acid-generating agent, a halomethyl group-substituted s-triazine derivative or a diphenyliodonium salt compound can be used suitably.

Suitable as the coumarin type dye are those which have a high sensitivity to a light irradiator generally used in dental applications and which show the maximum absorption wavelength at a visible light region of 400-500 nm. As representative examples of the coumarin type dye, there can be mentioned 3-thienoylcoumarin, 3,3'-carbonylbis(7-diethylamino)coumarin, 3,3'-carbonylbis(4-cyano-7-diethylaminocoumarin, 7-hydroxy-4-methylcoumarin and 2,3,6,7-tetrahydro-9-methyl-1H,5H,11H-[1]benzopyrano[6,7,8-ij]quinolidin-11-on.

The photo-induced acid-generating agent, when exposed to a light, is decomposed to form a Brønsted acid or a Lewis acid. A known photo-induced acid-generating agent can be used with no restriction as long as it is decomposed when exposed to a visible light in the presence of a dye, to generate an acid. Preferred is a halomethyl group-substituted s-triazine derivative or a diphenyliodonium salt compound, which conducts energy transfer with the above coumarin type dye and, when exposed to a visible light, generates an acid at a high efficiency.

As specific examples of the representative halomethyl group-substituted s-triazine derivatives, there can be mentioned 2,4,6-tris(trichloromethyl)-s-triazine, 2-methyl-4,6-bis(trichloromethyl)-s-triazine and 2-(2,4-dichlorophenyl)-4,6-bis(trichloromethyl)-s-triazine.

As specific examples of the representative diphenyliodonium salt compounds, there can be mentioned bromide, tetrafluoroborate, hexafluorophosphate, hexafluoroantimonate and trifluoromethanesulfonate salts of diphenyliodonium, and p-octyloxyphenylphenyliodonium.

The above-mentioned other polymerization catalysts can be compounded singly or, as necessary, in a combination of a plurality of kinds. The amount of the other polymerization catalyst used is not particularly restricted as long as it does not hinders the meritorious effects of the present invention, and may be appropriately determined depending upon the purpose and application of the curable composition produced. The amount of α-diketone or acylphosphine oxide used is preferably 0.01-20 pbm, more preferably 0.1-10 pbm relative to 100 pbm of the total radical-polymerizable monomers; and it is preferred to add, as necessary, 0.01-20 pbm of an amine compound.

When the polymerization catalyst is a system of an aryl borate compound, a dye and a photo-induced acid-generating agent, the dye may be used in an amount of 0.001-1 pbm and the photo-induced acid-generating agent may be used in an amount of 0.01-10 pbm. In this case, the amount of the aryl borate compound used may be in the above-mentioned range in the total amount including the aryl borate compound used in the present radical polymerization catalyst.

As to the optional additives other than mentioned above, explanation is made on highly relevant additives in the later-described dental applications.

The present curable composition is used, at a final steps, in a state in which the total components are mixed. However, it is ordinarily packed stably in two packages as necessary to prevent the deterioration of components and the undesired curing, occurring during the storage. In particular, the aryl borate compound, which decomposes easily in an acidic condition, is preferred to be packed separately from the acidic compound.

There is ordinarily used, for example, a combination of a package consisting of part of a radical-polymerizable monomer, a vanadium compound and an acidic compound, and a package consisting of part of the radical-polymerizable monomer and an aryl borate compound. In this case, the above-mentioned optional components are compounded appropriately in the two packages in view of e.g. the stabilities. When a filler is used, it is preferred that a package consisting of the whole part of a radical-polymerizable monomer and a package consisting of the filler are prepared and the present radical polymerization catalyst is packed appropriately in either of the packages.

The present radical polymerization catalyst has no particular restriction as to the applications and can be used in applications of known radical polymerization catalysts. As particularly preferable applications of the present radical polymerization catalyst where the high polymerization activity thereof can be exhibited effectively, there is a dental composition.

### (Dental composition)

The present application of a dental composition are not particularly restricted but specific examples include a dental adhesive, a dental filling material and a material for denture base resin. Further, the present radical polymerization catalyst is also usable, for example, as a dental pretreatment agent used prior to the application of a curable composition.

### (1) Dental adhesive

The first dental application of the curable composition containing the present radical polymerization catalyst is a dental adhesive (hereafter referred to as the present dental adhesive). By using the present radical polymerization catalyst, good adhesivity and adhesion durability can be obtained as compared with when known radical polymerization catalysts of chemical polymerization type are used.

As representative dental adhesives, there can be mentioned an adhesive used for adhesion of a tooth and a material for direct restoration (e.g. a composite resin for filling) (this adhesive is hereinafter referred to as adhesive for direct restoration and is generally called bonding agent); and an adhesive used for adhesion of a tooth and a material for indirect restoration (e.g. crown or inlay) or for temporary fixation of a loose tooth [this adhesive is hereinafter referred to as adhesive for indirect restoration, is generally called adhesive cement, and is classified into e.g. a CR type resin cement, a MMA (methyl mehtacrylate) type cement and a resin-reinforced glass ionomer cement for luting]. When the term "dental adhesive" is simply mentioned, it means all of the adhesive for direct restoration, the adhesive for indirect restoration and adhesives used for other dental applications.

The curable composition containing the present radical polymerization catalyst is used as a dental adhesive, and uses, as the acidic compound which is a component of the radical polymerization catalyst, the above-mentioned acidic group-containing radical-polymerizable monomer (an acidic monomer) described in more detail below.

The acidic monomer functions not only as a component of the radical polymerization catalyst but also as an acid to show a decalcification effect to tooth and a high infiltrability to tooth; thereby, the adhesive obtained can have very good adhesivity to tooth. The adhesive can also have improved adhesivity to various dental metals and ceramics.

As to the acidic monomer, there is used acompound which, when made into a 10 wt.% aqueous solution or suspension, have a pH of 4.5 or less, more preferably 4 or less, and is a compound of formula (2)- 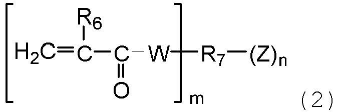 wherein
- R⁶: is H or methyl;
- R⁷: is a di- to hexavalent C₁₋₃₀-organic residue;
- W: is O, S or NH;
- Z: is -COOH, -SO₃H, -O-P(=O)(OH)₂, -P(=O)(OH)₂ or -O-P(=O)(OH)(OR⁸), wherein R⁸ is C₁₋₁₀-alkyl or aryl having 6-14 ring carbon atoms, and the alkyl group or the aryl group may be substituted by halogen, hydroxyl, cyano, nitro, C₁₋₅-alkyl, C₂₋₅-alkenyl, C₂₋₅-alkynyl, C₁₋₅-alkoxyl, C₂₋₅-acyl or C₂₋₅-acyloxy;
- m: is an integer of 1-5;
- n: is an integer of 1-5; and (m+n) corresponds to the valence of R⁷.

In formula (2) Z is -COOH, -SO₃H, -O-P(=O)(OH)₂, - P(=O)(OH)₂ or
-O-P(=O)(OH)(OR₈); and R₈ is alkyl whose main chain has 1-10, preferably 1-5, carbon atoms or aryl whose ring(s) has (have) 6-14, preferably 6-10, carbon atoms. The alkyl or aryl may be substituted by halogen, hydroxyl, nitro, cyano, C₁₋₅-alkyl, C₂₋₅-alkenyl, C₂₋₅-alkinyl, C₁₋₅-alkoxyl, C₂₋₅-acyl or C₂₋₅-acyloxy; or may be substituted by a plurality of same or different substituents.

When R₈ is alkyl whose main chain has 1-10 carbon atoms, it is exemplified by methyl, ethyl, n-propyl, n-butyl, n-octyl and n-decyl. When the alkyl is substituted by the above-mentioned substituent(s), such a substituted alkyl group is exemplified by chloromethyl, 2-chloroethyl, 2-bromoethyl, 2-hydroxyethyl, 2-hydroxypropyl, 2,3-dihydroxypropyl, 6-hydroxyhexyl, 2-cyanoethyl, i-propyl, i-butyl, t-butyl, 1-methylpropyl, 2-ethylhexyl, 2-propenyl, cis- or trans-2-butenyl, 2-propyneyl, methoxymethyl, 2-methoxyethyl, 2-ethoxyethyl, 3-methoxybutyl, 3-oxabutyl, 4-oxapentyl, 3-oxapentyl, 2-acetyloxyethyl, 3-acetyloxypropyl, 2-propionyloxyethyl, 3-acetyloxy-2-hydroxypropyl and 2-ethyl-3-hydroxypentyl.

The aryl group whose ring(s) has (have) 6-14 carbon atoms, is exemplified by phenyl, 1- or 2-naphthyl and 1-, 2- or 9-anthranyl. When the aryl is substituted by the above-mentioned substituent(s), such a substituted aryl is exemplified by o-, m- or p-chlorophenyl, o-, m- or p-bromophenyl, o-, m- or p-hydroxyphenyl, 3-hydroxy-2-naphthyl, o-, m- or p-nitrophenyl, o-, m- or p-cyanophenyl, o-, m- or p-methylphenyl, o-, m- or p-butylphenyl, 3,4-dimethylphenyl, 2,4-dimethylphenyl, o-, m- or p-styryl, o-, m- or p-(2-propinyl)phenyl, o-, m- or p-methoxyphenyl, o-, m- or p-ethoxyphenyl, 2-, 3- or 4-acetylphenyl, 2-, 3- or 4-acetyloxyphenyl, 4-hydroxy-3-methylphenyl and 4-methyl-2-nitrophenyl.

In the present dental adhesive, there can also be suitably used a compound of acid anhydride structure formed by dehydration and condensation of an acidic group such as Z. The acid anhydride may be a compound formed by intra-molecular dehydration and condensation of a compound of formula (2), or may be a compound formed by inter-molecular dehydration and condensation of two molecules selected from the compounds of formula (2), vinylsulfonic acid, vinylphosphonic acid and (meth)acrylic acid. The acid anhydride formed by inter-molecular dehydration and condensation may have a structure formed by dehydration and condensation of the same acid, or may have a structure formed by dehydration and condensation of different acids. From the easiness of synthesis and availability, the acid anhydride preferably has a structure formed by intra-molecular dehydration and condensation or a structure formed by dehydration and condensation of two molecules of the same acid.

In formula (2), R₇ is a di- to hexavalent organic residue of 1-30 carbon atoms. This organic residue is not particularly restricted and may be a known group. The organic residue may have, in the structure, a bond other than carbon-to-carbon bond, such as ether bond, ester bond, amide bond, sulfonyl bond, urethane bond or thioether bond, and further may have a substituent containing no carbon atom, such as halogen, hydroxyl, amino, cyano or nitro.

As specific examples of the organic residue, the following groups can be mentioned.

-CH₂-

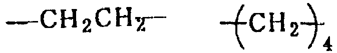

(̵CH₂)̵₆

(̵CH₂)̵₈ (̵CH₂)̵₁₀ (̵CH₂)̵₂₀

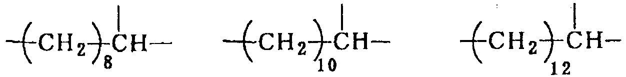

-CH₂CH₂OCH₂CH₂- -CH₂CH₂(̵OCH₂CH₂)̵₂

-CH₂CH₂(̵OCH₂CH₂)̵₆

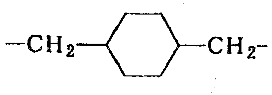

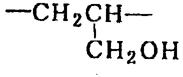

-C(CH₃)₂CH₂- 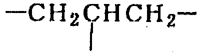 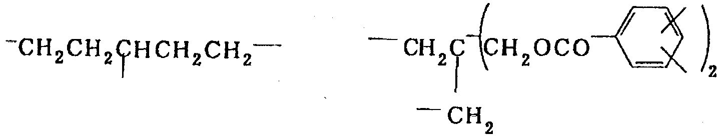 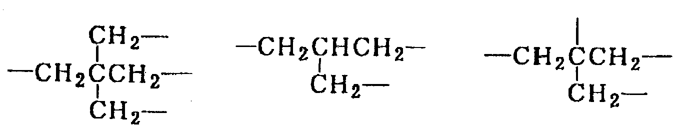 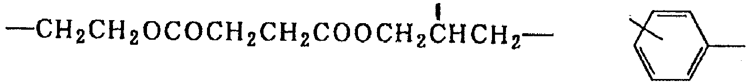 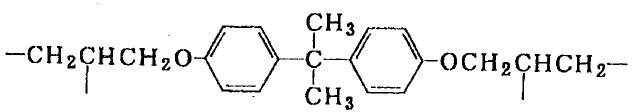 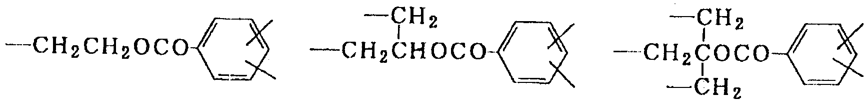 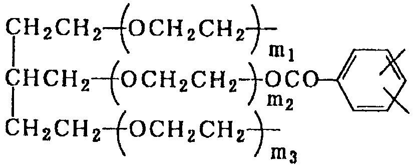 (Each m¹, m², and m³ is integer of 0-5.)

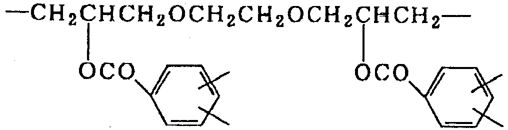

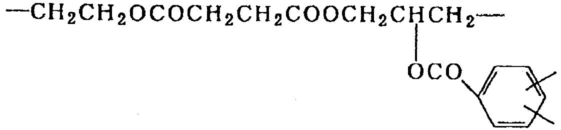

Specific examples of the acidic group-containing radical-polymerizable monomer of formula (2) are as follows (wherein R₆ is H or methyl). 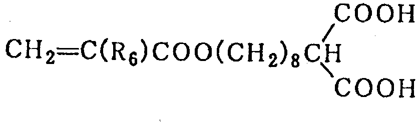 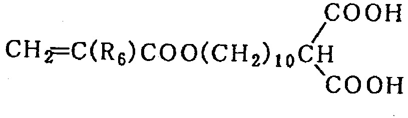 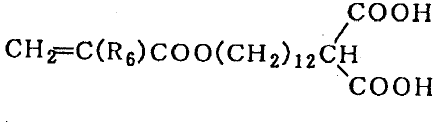 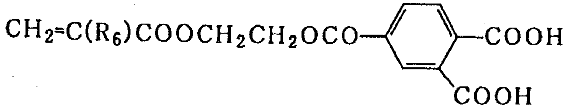 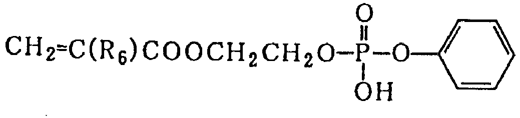 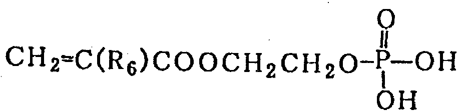 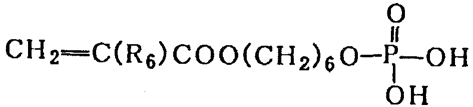 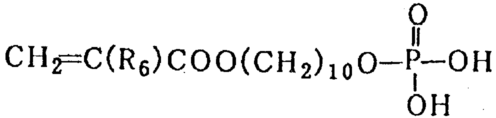 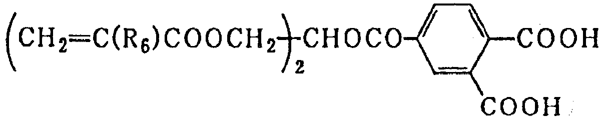 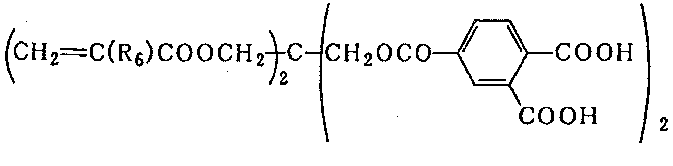 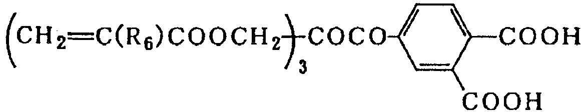 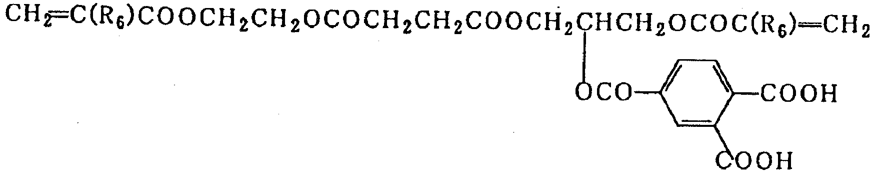 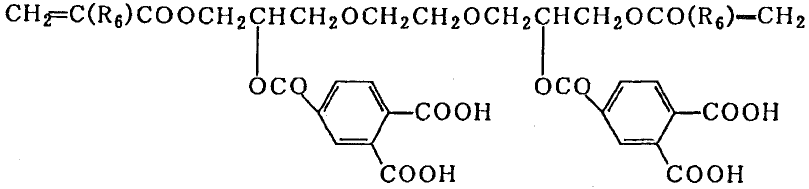 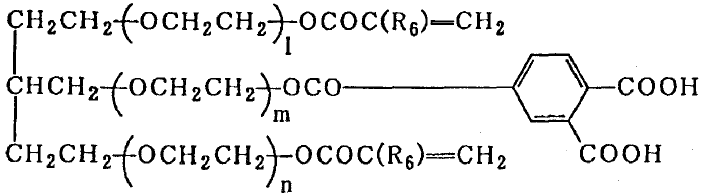 (mixed compound of l+m+n=3.5.)

CH₂-C(R₆)CONHCH₂CH₂COOH

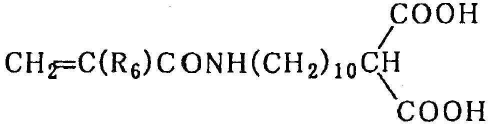

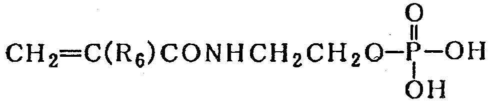

CH₂=C(R₆)COOCH₂CH₂SO₃H

CH₂=C(R₆)COO(CH₂)₆SO₃H

CH₂=C(R₆)COO(CH₂)₁₀SO₃H

CH₂=C(R₆)COOC(CH₃)₂CH₂SO₃H

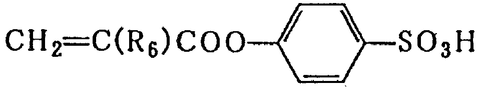

CH₂=C(R₆)CONHCH₂CH₂SO₃H

CH₂=C(R₆)CONHC(CH₃)₂CH₂SO₃H

These acidic monomers may be used alone or in admixture of two or more kinds.

The compounds of formula (2), used for formation of the above acid anhydrides are restricted to those having 2 or more as n.

It is preferred to use, from the reason that a high adhesion strength is obtained to tooth enamel and base metals, a compound of formula (2) wherein Z is -O-P(=O)(OH)₂ or -O-P(=O)(OH)(OR₈) (hereinafter, these compounds may be generically referred to as phosphoric acid type monomer).

By using, in combination with the phosphoric acid type monomer, a compound of formula (2) wherein Z is -COOH or a compound of acid anhydride structure formed by dehydration and condensation of the above -COOH (hereinafter, these compounds may be generically referred to as carboxylic acid type monomer), the resulting dental adhesive can have a higher adhesion strength to dentin and further can give a reduced fluctuation in adhesion strength, particularly in adhesion strength to dentin. The most preferred combination is a combined use of a phosphoric acid type monomer and a carboxylic acid type monomer having a plurality of -COOH groups [a compound of formula (2) wherein n is 2-5] or an acid anhydride formed by intra-molecular dehydration and condensation of the carboxylic acid type monomer.

When a phosphoric acid type monomer and a carboxylic acid type monomer are used in combination, their proportions may be appropriately determined depending upon e.g. the kinds and amounts of other components, the application and purpose of the resulting adhesive. Ordinarily, however, the phosphoric acid type monomer : the carboxylic acid type monomer is 10:90 to 90:10 in terms of mass ratio.

From reasons that the polymerizability is good and the risk of release of residual monomer from the cured material obtained is low, there is also preferred a compound wherein W, or W' and W" are an oxygen atom, that is, a compound wherein the radical-polymerizable group is a (meth)acryloxy group.

The present dental adhesive has adhesivity even when it contains only an aryl borate compound, an acidic monomer and a vanadium compound. However, a dental adhesive having even higher adhesivity can be obtained by compounding other components described below, depending upon the use purpose and the application.

In the present dental adhesive, it is preferred that in addition to the above-mentioned acidic monomer, other radical-polymerizable monomer (non-acidic monomer) is compounded. The non-acidic monomer is not particularly restricted and a known radical-polymerizable monomer can be used. As specific examples of the non-acidic monomer, there can be mentioned those monomers previously described as examples of the components of the present curable composition.

In the present dental adhesive, there is no particular restriction as to the use amounts of the aryl borate compound, the acidic monomer, the vanadium compound and the optionally added non-acidic monomer. However, in a preferred composition for obtaining high adhesivity, the amount of the aryl borate compound is 0.01-10 pbm and the amount of the + tetravalent and/or + pentavalent vanadium compound is 0.001-10 pbm, relative to 100 pbm of the total radical-polymerizable monomers including the acidic monomer, and the amount of the acidic monomer is 1-100 mass% in the total radical-polymerizable monomers. In a more preferred composition, the amount of the acidic monomer is, in the above compounding ratio, 5-70 mass%, particularly 10-50 mass% in the total radical-polymerizable monomers.

When the present dental adhesive is used as an adhesive for direct restoration, in, for example, adhesion of a composite resin for filling, it is preferred that a non-acidic polyfunctional monomer is used as the non-acidic monomer in an amount of 10-85 mass% relative to the total radical-polymerizable monomers. It is also preferred that, in addition to the non-acidic polyfunctional monomer, a water-soluble non-acidic monofunctional monomer having hydroxyl group (the monomer is hereinafter referred to as water-soluble non-acidic monomer) such as 2-hydroxyethyl (meth)acrylate, 3-hydroxypropyl (meth)acrylate or 2,3-dihydroxypropyl (meth)acrylate is used in an amount of 5-40 mass% relative to the total radical-polymerizable monomers. By their use, even higher adhesivity can be obtained.

In the adhesive for direct restoration, it is also preferred that a polyvalent metal ion-releasing filler is used as a filler. Thereby, very high adhesivity and adhesion durability can be obtained.

The polyvalent metal ion-releasing filler is an inorganic compound which reacts with an acid to release a polyvalent metal ion. By using the polyvalent metal ion-releasing filler, a chelating reaction between the acidic monomer and the polyvalent metal ion proceeds in parallel with a radical polymerization reaction, whereby the resulting cured material has a higher strength. The polyfunctional metal ion-releasing filler is not particularly restricted as long as it has the above-mentioned property. As specific examples of the polyfunctional metal ion-releasing filler usable in the present invention, there can be mentioned hydroxides such as calcium hydroxide and strontium hydroxide; zinc oxide; silicate glass; fluoroaluminosilicate glass; barium glass; and strontium. Of these, fluoroaluminosilicate glass is best in the coloring resistance of the cured material, and its use is suitable.

As the fluoroaluminosilicate glass, a known material can be used. A generally known fluoroaluminosilicate glass has such a composition that silicon is 10-33, aluminum is 4-30, alkaline earth metal is 5-36, alkali metal is 0-10, phosphorus is 0.2-16, fluorine is 2-40 and oxygen is the remainder, all in terms of ionic mass %. Besides such a composition, there can also be used those in which part or the whole of the above alkaline earth metal has been replaced by magnesium, strontium or barium. A composition in which part or the whole of the alkaline earth metal has been replaced by strontium, in particular, can be used suitably because it can provide a cured material having X-ray impermeability and a high strength.

The use amount of the polyvalent metal ion-releasing filler is preferably 1-20 pbm, more preferably 2-15 pbm relative to 100 pbm of the total radical-polymerizable monomers.

The adhesive for direct restoration preferably contains water. By using water, high adhesivity can be obtained very easily without conducting a treatment by a pretreatment agent (described later) to a tooth surface.

The use amount of water is not particularly restricted, but is preferably 2-30 pbm, more preferably 3-20 pbm relative to 100 pbm of the total radical-polymerizable monomers. By using water in an amount of 2 pbm or more in the adhesive for direct restoration, higher adhesivity can be obtained effectively even for a tooth not pretreated by a pretreatment agent. Further by using water in an amount of 30 pbm or less, the resulting cured material of the adhesive can have superior properties in e.g. initial adhesion strength and coloring resistance. Furthermore, by using a polyvalent metal ion-releasing filler in combination with water, the above-mentioned effects become higher.

When the present dental adhesive is an adhesive for direct restoration, it is preferred to use a photopolymerization catalyst in addition to the present radical polymerization catalyst. By using a photopolymerization catalyst and conducting photopolymerization by light irradiation together with the chemical polymerization according to the present invention, adhesivity can be enhanced further. The light irradiation has an advantage that curing is made possible in a desired shorter time.

As the photopolymerization catalyst, one of the above-mentioned catalysts can be used. A photopolymerization catalyst of α-diketone type, acylphosphine oxide type or aryl borate compound/dye/photo-induced acid-generating agent type is preferred.

A photopolymerization catalyst of aryl borate compound/dye/photo-induced acid-generating agent type is particularly preferred for the reason mentioned above. The use amount of the photopolymerization catalyst is as per explained in the section of the present curable composition.

When the dental adhesive is used as an adhesive for direct restoration, there can be compounded in the adhesive for direct restoration, in a range in which the meritorious effects of the present invention are not impaired, known additives mentioned above or mentioned later, such as inorganic filler, organic filler, inorganic-organic composite filler, radical-polymerizable monomer other than mentioned above (e.g. adhering-to-precious-metal monomer), thickening agent, polymerization inhibitor, polymerization-regulator, ultraviolet absorber, metal salt, organic solvent, inorganic or organic acid, dye, pigment and thermal polymerization catalyst or redox type polymerization catalyst.

When the present dental adhesive is used as an adhesive for indirect restoration, it is preferred to compound, in the adhesive for indirect restoration, a filler such as inorganic filler, organic filler or inorganic-organic composite filler, for the handleability and the strength of the cured material obtained.

Further, various components are compounded depending upon the application and purpose of the adhesive. The following compositions are preferably used as suitable forms of the adhesive.
(A) An adhesive for indirect restoration containing radical-polymerizable monomers including an acidic monomer and a non-acidic polyfunctional monomer, an aryl borate compound, a vanadium compound, and an inorganic filler and/or an inorganic-organic composite filler [hereinafter, this adhesive is referred to as CR (composite resin) type resin cement].
(B) An adhesive for indirect restoration containing radical-polymerizable monomers including an acidic monomer and methyl methacrylate, an aryl borate compound, a vanadium compound, and an organic filler (hereinafter, this adhesive is referred to as MMA type resin cement).

In the CR type resin cement, specific examples of the acidic monomer, the non-acidic polyfunctional monomer, the aryl borate compound and the vanadium compound are the same as mentioned above in the section of adhesive for direct restoration.

As the inorganic filler and/or the inorganic-organic composite filler, there can be used known fillers for dental use with no particular restriction. Such an inorganic filler can be exemplified by inorganic particles of oxides of metals such as quartz, silica, silica-alumina, silica-titania, silica-zirconia, silica-magnesia, silica-calcia, silica-barium oxide, silica-strontium oxide, silica-titania-sodium oxide, silica-titania-potassium oxide, titania, zirconia and alumina. The above-mentioned polyvalent metal ion-releasing filler can also be used as the inorganic filler.

As a suitable example of the inorganic-organic composite filler, there is a filler obtained by mixing the above-mentioned inorganic particles with a polymerizable monomer and subjecting the mixture to curing and grinding.

By compounding such an inorganic filler and/or an inorganic-organic composite filler in the adhesive for restoration, the resulting cured material can have a higher mechanical strength and higher adhesion durability.

When importance is attached to the mechanical strength of cured material, it is preferred to use inorganic particles of e.g. oxides of metals, such as quartz, silica, silica-titania, silica-zirconia, silica-titania-sodium oxide and silica-titania-potassium oxide. When importance is attached to adhesivity, it is preferred to use a polyvalent metal ion-releasing filler as the inorganic filler. By using the polyvalent metal ion-releasing filler, high adhesivity can be obtained without conducting a pretreatment to a tooth surface by a pretreatment agent.

These inorganic fillers and/or inorganic-organic composite fillers are preferably surface-treated with a silane coupling agent such as γ-methacryloxypropyltrimethoxysilane, ε-methacryloxyoctyltrimethoxysilane or vinyltrimethoxysilane.

As explained in the above section of adhesive for direct restoration, compounding of water and/or a water-soluble non-acidic monomer in the adhesive for restoration is preferred because high adhesivity is obtained. Compounding of the water and/or the water-soluble non-acidic monomer is particularly effective when the filler used mainly is a polyvalent metal ion-releasing filler.

When the present dental adhesive is used in the form of a CR type resin cement, the use amounts of individual components are preferably such that the amount of the aryl borate compound is 0.01-10 pbm, the amount of the + tetravalent and/or + pentavalent vanadium compound is 0.001-10 pbm and the amount of the inorganic filler and/or the inorganic-organic composite filler is 50-900 pbm, relative to 100 pbm of the radical-polymerizable monomers including 5-70 mass% of an acidic monomer and 10-95 mass% of a non-acidic polyfunctional monomer.

When importance is attached to the mechanical strength of cured adhesive, the use amounts of individual components are more preferably such that the amount of the aryl borate compound is 0.05-8 pbm, the amount of the + tetravalent and/or + pentavalent vanadium compound is 0.01-5 pbm and the amount of the inorganic filler composed mainly (preferably 50 mass% or more) of metal oxide inorganic particles is 100-800 pbm, relative to 100 pbm of the radical-polymerizable monomers including 10-50 mass% of an acidic monomer and 50-80 mass% of a non-acidic polyfunctional monomer.

When importance is attached to adhesivity, there is more preferred a composition in which the amount of the aryl borate compound is 0.05-8 pbm, the amount of the + tetravalent and/or + pentavalent vanadium compound is 0.001-5 pbm, the amount of the inorganic filler composed mainly (preferably 50 mass% or more) of the polyvalent metal ion-releasing filler is 100-300 pbm and the amount of water is 0-80 pbm, relative to 100 pbm of the radical-polymerizable monomers including 10-50 mass% of an acidic monomer, 10-50 mass% of a non-acidic polyfunctional monomer and 10-80 mass% of a water-soluble non-acidic monomer. An adhesive for indirect restoration containing, as the inorganic filler, mainly a polyvalent metal ion-releasing filler may be called a resin-reinforced glass ionomer cement for luting.

In the CR type resin cement, there can be compounded as necessary known additives such as radical-polymerizable monomer other than mentioned above (e.g. adhering-to-precious-metal monomer), other polymerization catalyst (for example, photopolymerization catalyst, thermal polymerization catalyst or redox catalyst), organic filler, thickening agent, polymerization inhibitor, polymerization-regulator, ultraviolet absorber, metal salt, organic solvent, inorganic or organic acid, dye and pigment.

In the MMA type resin cement, preferred specific examples of the acidic monomer, the aryl borate compound and the vanadium compound are the same as mentioned above in the section of adhesive for direct restoration.

In the case of the MMA type resin cement, the liquid component composed mainly of MMA (methyl methacrylate) and the powder component composed mainly of an organic filler are mixed with each other when they are used.

As specific examples of the organic filler, there can be mentioned a powder of (meth)acrylate type polymer such as polymethyl methacrylate, polyethyl methacrylate, methyl methacrylate-ethyl methacrylate copolymer, ethyl methacrylate-butyl methacrylate copolymer or methyl methacrylate-trimethylolpropane methacrylate copolymer; and a powder of polyvinyl chloride, polystyrene, chlorinated polyethylene, nylon, polysulfone, polyethersulfone or polycarbonate. Use of, in particular, a powder of (meth)acrylate type polymer is preferred.

As the non-acidic monomer, there can be appropriately used, besides methyl methacrylate, a non-acidic monomer mentioned in the section of the present curable composition.

A preferred composition of the MMA type resin cement is such that the amount of the aryl borate compound is 0.01-10 pbm, the amount of the vanadium compound is 0.001-10 pbm and the amount of the organic filler is 50-300 pbm, relative to 100 pbm of the radical-polymerizable monomers including 1-50 mass% of an acidic monomer and 30-99 mass% of methyl methacrylate. A more preferred composition is such that the amount of the aryl borate compound is 0.05-8 pbm, the amount of the vanadium compound is 0.001-5 pbm and the amount of the organic filler is 60-250 pbm, relative to 100 pbm of the radical-polymerizable monomers including 5-30 mass% of an acidic monomer and 50-95 mass% of methyl methacrylate.

It is effective to compound, in the MMA type resin cement, an X-ray impermeable inorganic filler (e.g. barium glass powder or silica-zirconia particles) in about the same mass amount as that of the organic filler, to allow for X-ray imaging.

In the MMA type resin cement, it is possible to compound not only the above-mentioned components but also, as necessary, known additives such as other radical-polymerizable polymerization catalyst (e.g. photopolymerization catalyst, thermal polymerization catalyst or redox catalyst), inorganic filler, inorganic-organic composite filler, adhering-to-precious-metal monomer, thickening agent, polymerization inhibitor, polymerization-regulator, ultraviolet absorber, metal salt, organic solvent, inorganic or organic acid, dye and pigment.

In each of the dental adhesives for direct restoration and indirect restoration, it is possible to add a radical-polymerizable monomer having a functional group capable of bonding to precious metals (an adhering-to-precious-metal monomer), to adhere the adhesive to, for example, a precious metal-made restoration for dental crown. As examples of the adhering-to-precious-metal monomer suitably used, there can be mentioned functional group-containing radical-polymerizable monomers such as thiouracyl derivative, triazinedithione derivative and mercaptothiadiazole derivative. Specifically, there can be mentioned radical-polymerizable monomers disclosed in JP-A-10-1409, JP-A-10-1473, JP-A-8-113763. Of these, adhering-to-precious-metal monomers shown below can be used suitably.

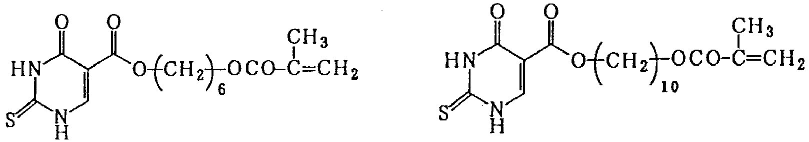

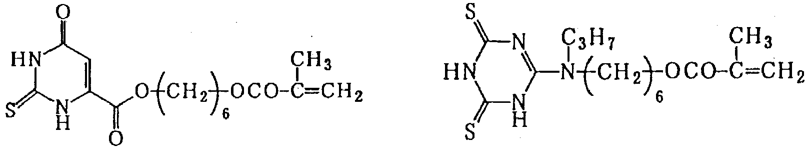

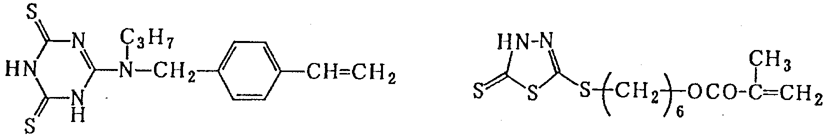

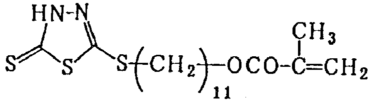

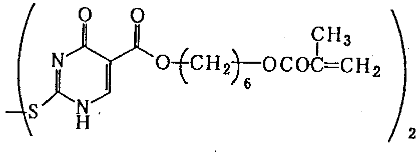

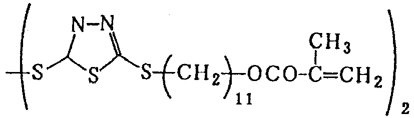

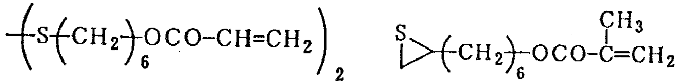

The adhering-to-precious-metal monomer is compounded particularly preferably in the adhesive for indirect restoration generally used in adhesion of precious metal-made crown and inlay. The use amount of the adhering-to-precious-metal monomer is ordinarily 0.1-50 mass%, preferably 0.2-20 mass% in the total radical-polymerizable monomers.

As to the packaging form of the present adhesive, the aryl borate compound and the acidic monomer (and other acidic compound used as necessary) are preferred to be packed in different packages to prevent the decomposition of the aryl borate compound as described previously.

In the case of, for example, the adhesive for direct restoration, there is preferred a form in which a solution composed mainly of an acidic monomer, a non-acidic monomer and a vanadium compound and a solution composed mainly of a non-acidic monomer and an aryl borate compound are packed in different packages and they are mixed right before use.

In the case of the CR type resin cement, there is a form in which two solution packages similar to those of the adhesive for direct restoration are prepared, a filler is added to each solution to prepare two pastes, and the two pastes are mixed right before use. There is also a form in which there are prepared a solution package consisting of all radical-polymerizable monomers (including an acidic monomer) and a vanadium compound and a powder package consisting of a filler and an aryl borate compound and the contents of the two packages are mixed right before use.

In the case of the MMA type resin cement, an example of a preferred packaging form is such that a solution of consisting of all radical-polymerizable monomers (including an acidic monomer) and a vanadium compound and a powder consisting of a filler and an aryl borate compound are packed in different packages. When water is compounded, it is preferred not to compound water in a package containing an acidic monomer, for good storage stability.

The above packaging forms are exemplary and other packaging forms may be taken as necessary.

The method for using the present adhesive is not particularly restricted and the present adhesive may be used according to the known method for using each of the adhesive for direct restoration and the adhesive for indirect restoration.

Ordinarily, all the components constituting the adhesive are mixed right before the application of the adhesive to an adherend surface (e.g. a tooth surface); the resulting mixture is applied on the adherend surface using a small brush or a dental sponge; light irradiation is made to the applied mixture as necessary; then, e.g. a composite resin or an inlay is adhered to the adherend surface.

In this case, the adherend surface is preferably subjected as necessary to a pretreatment by a pretreatment agent. By this pretreatment, adhesion becomes strong. The pretreatment is particularly effective when the present dental adhesive is free from water and/or polyvalent metal ion-releasing filler.

### (2) Dental restorative material

The second dental application of the curable composition containing the present radical polymerization catalyst is a dental restorative material (hereinafter referred to as the present dental restorative material). In using a conventional photo-curing type radical polymerization catalyst, it has been difficult to obtain a cured material having sufficient properties, at a site where light irradiation is not sufficient. Application to such a site becomes possible by using the present radical polymerization catalyst. When the present radical polymerization catalyst is used, as compared with when a conventional chemical polymerization type radical polymerization catalyst is used, the cured material obtained has good mechanical properties in e.g. bending strength and Knoop hardness and also has good resistance to initial color. Further, the cured material is low in risk of release of residual monomers and accordingly has a higher safety to living body.

The present dental restorative material includes a composite resin for direct restoration which is filled, shaped and cured in the mouth in, for example, the tooth damaged by dental caries; a composite resin for indirect restoration which is shaped and cured outside the mouth to be made into e.g. a crown, an inlay or a bridge and then is fitted inside the mouth; a composite resin for building of abutment tooth (hereinafter, these composite resins are referred to as dental composite resin); a dental ambient-temperature-curing type resin for forming a temporary crown, a temporary inlay, a denture base resin material, a relining material for denture (hereinafter, this resin is referred to as self-curing type resin); and a resin-reinforced type glass ionomer cement for filling. The present dental restorative material is used in the lost portion of tooth and gum and its cured material becomes a prosthesis for the lost portion.

The present dental restorative material contains the present dental adhesive and further contains a non-acidic monomer and a filler.

A non-acidic monomer is compounded in the present dental restorative material. By compounding the non-acidic monomer, the dental restorative material after curing can have good mechanical strengths and good discoloration resistance (good resistance to color development).

There is no particular restriction as to the non-acidic monomer. There can be suitably used those exemplary monomers shown in the explanation of the present curable composition, and they can be appropriately selected depending upon, for example, the later-described application of the dental restorative material.

A filler is compounded in the present dental restorative material. By compounding the filler, the dental restorative material has good operability and the cured material thereof has good mechanical properties. As the filler, any of the above-mentioned inorganic filler, organic filler and inorganic-organic composite filler may be used and a known filler may be selected appropriately depending upon the use purpose of the restorative material.

When the present dental restorative material is a dental composite resin, the non-acidic monomer is used preferably in an amount of 80 mass% or more, more preferably in an amount of 90 mass% or more in the total radical-polymerizable monomers for the high mechanical strengths and high resistance to initial color, of the cured composite resin.

Also when the present dental restorative material is a dental composite resin, the filler is particularly preferably an inorganic filler and/or an inorganic-organic composite filler. As the inorganic filler or the inorganic-organic composite filler, there can be used those described in the section of the present dental adhesive.

Of these fillers, there can be used suitably, as the inorganic filler, particles of, for example, metal oxides such as silica and alumina, and particles of composite metal oxides such as silica-titania and silica-zirconia. As the inorganic-organic composite filler, there can be suitably used fillers obtained by mixing metal oxide particles or composite metal oxide particles with a radical-polymerizable monomer and subjecting the mixture to curing and grinding.

The shape of the inorganic filler or of the inorganic filler present in the inorganic-organic composite filler is not particularly restricted and may be a known shape. However, there are preferred inorganic particles of spherical shape or nearly spherical shape (a shape judged to be nearly spherical when observed by a scanning type electron microscope), or a combination of such spherical or nearly spherical inorganic particles and irregular-shaped inorganic particles. By using spherical or nearly spherical inorganic particles, the cured material obtained has a smooth and lustrous surface and has an excellent appearance. By further using irregular-shaped inorganic particles in addition to the spherical or nearly spherical inorganic particles, the cured material obtained can have higher mechanical strengths. In this case, particularly suitable as the spherical or nearly spherical inorganic particles are those having an average primary particle diameter of 0.05-5 µm and particularly suitable as the irregular-shaped inorganic particles are those having an average particle diameter of 0.05-5 µm. These inorganic particles, particularly spherical inorganic particles may be in the form of an agglomerate. The maximum diameter of the agglomerate is preferably 10 µm or less, particularly preferably 5 µm or less.

When the inorganic-organic composite filler is used, the average particle diameter is suitably 0.1-20 µm.

It is possible to add, to the restorative material, inorganic particles having an average primary particle diameter of 0.005-0.05 µm for the adjustment of viscosity and thixotropy.

The inorganic filer or the inorganic-organic composite filler is preferably surface-treated with a surface-treating agent. The filler is preferably surface-treated specifically with a silane coupling agent such as γ-methacryloxypropyltrimethoxysilane, ε-methacryloxyoctyltrimethoxysilane or vinyltrimethoxysilane.

There is no particular restriction, either, as to the use amount of the inorganic filler and/or the inorganic-organic composite filler. However, the use amount is preferably 50-1,900 pbm, more preferably 100-1,200 pbm relative to 100 pbm of the total radical-polymerizable monomers. When the present dental composite resin is for indirect restoration or for abutment tooth building, the use amount of the inorganic filler and/or the inorganic-organic composite filler is preferably 300-1,900 pbm, particularly preferably 400-1,200 pbm relative to 100 pbm of the total radical-polymerizable monomers.

The above-mentioned photopolymerization catalyst is preferably compounded in the present dental composite resin when it is a composite resin for direct restoration or building of abutment tooth.

Also, e.g. a thermal polymerization catalyst or a redox catalyst is preferably compounded in the present dental composite resin when it is a composite resin for indirect restoration.

When the present dental restorative material is an self-curing type resin, the non-acidic monomer used is not particularly restricted and a known radical-polymerizable monomer can be used. Specifically, there can be suitably used, for example, those exemplary monomers shown in the description of the present curable composition.

The above-mentioned non-acidic monofunctional monomer and aliphatic non-acidic polyfunctional monomer can be suitably used when importance is attached to the operability of dental restorative material and the properties of the cured material obtained. When importance is attached to the high strength or low water absorption of cured material, there are preferably compounded the fumaric acid ester compound, styrene derivative and allyl compound shown as specific examples in the section of the present curable composition.

The non-acidic monomer is used preferably in an amount of 80-100 mass% in the total radical-polymerizable monomers.

As the filler, an organic filler is used particularly preferably when the present dental restorative material is an self-curing type resin. By using the organic filler, the self-curing type resin can have good operability and the cured material thereof can have good mechanical properties. As the organic filler, there can be used those explained in the section of the MMA type resin cement.

The use amount of the organic filler is 50-500 pbm, preferably 60-250 pbm relative to 100 pbm of the total radical-polymerizable monomers.

Also, the above-mentioned photopolymerization catalyst may be used. In this case, a dual cure type restorative material is obtained.

When the present dental restorative material is a resin-reinforced type glass ionomer cement for filling, there are preferably compounded in the restorative material, as the non-acidic monomer, a hydroxyl group-containing, water-soluble, non-acidic monofunctional monomer such as 2-hydroxyethyl methacrylate or 2,3-dihydroxypropyl methacrylate, and a non-acidic polyfunctional monomer. Further use of an acidic monomer in combination with the non-acidic monomer is particularly suitable.

The use amounts of these monomers are not particularly restricted. However, when importance is attached to, for example, the mechanical properties of cured material, the use amount of the water-soluble non-acidic monofunctional monomer is preferably 5-80 mass%, the use amount of the non-acidic polyfunctional monomer is preferably 5-80 mass% and the use amount of the acidic monomer is 1-50 mass%, relative to all in the total radical-polymerizable monomers.

As the filler in the resin-reinforced type glass ionomer cement for filling, there is used the above-mentioned polyvalent metal ion-releasing filler. The use amount of the polyvalent metal ion-releasing filler is not particularly restricted, either. However, it is preferably 200-1,900 pbm, more preferably 300-900 pbm relative to 100 pbm of the total radical-polymerizable monomers.

To the resin-reinforced type glass ionomer cement for filling may also be added other inorganic filler, other organic filler and other inorganic-organic composite filler. In this case, however, the use amount of the polyvalent metal ion-releasing filler is preferably 50 mass% or more in the total fillers used.

To the resin-reinforced type glass ionomer cement for filling may further be added water in an amount of 100 pbm or less (preferably 80 pbm or less) relative to 100 pbm of the total radical-polymerizable monomers.

To these dental restorative materials such as dental composite resin, dental self-curing type resin and resin-reinforced type glass ionomer cement for filling may be added, in addition to the above-mentioned components, radical-polymerizable monomers other than specifically mentioned in each of the above sections, various components mentioned in the section of the present dental adhesive, and other known addition components. As these other addition components, there can be mentioned a polymerization inhibitor, a polymerization-regulator, an ultraviolet absorber, a dye, a pigment and a perfume.

### (3) Dental pretreatment agent

As an application of the composition containing the present radical polymerization catalyst, there can be mentioned a pretreatment agent used in adhesion or bonding of various materials (the agent is hereinafter referred to as the present dental pretreatment agent). The present dental pretreatment agent comprises the present dental adhesive (comprising an aryl borate compound, a (+)-tetra- and/or - pentavalent vanadium compound and an acidic group-containing radical-polymerizable monomer as defined above) and water.

The pretreatment agent is a material used for applying a pretreatment to an adherend prior to adhesion (luting is included). An adhesive or a luting material is ordinarily used in application of e.g. a restorative material to an adherend surface and, prior thereto, a pretreatment agent is used. However, in adhesion between denture base resin and relining material or in adhesion of a restorative material having adhesivity (e.g. a resin-reinforced type ionomer for filling), there are cases that the two materials are bonded directly with a pretreatment agent.

As examples of the application of the present dental pretreatment agent, there are shown, with no particular restriction, known dental materials such as tooth surface, dental precious metal, dental base metal, dental ceramic, composite resin and denture base resin.

Of the above applications of the present pretreatment agent the pretreatment of tooth surface is particularly suitable.

The pretreatment agent containing an acidic monomer as a component of the polymerization catalyst, when applied to a tooth surface, can efficiently remove a smear layer formed on the tooth surface when e.g. dental caries have been removed, and consequently high adhesivity can be obtained. Further, by adding water to the pretreatment agent, decalcification (e.g. removal of the above smear layer) can be promoted, whereby even higher adhesivity can be obtained.

The use amount of the aryl borate compound is preferably 0.01-10 mass%, more preferably 0.05-8 mass%, particularly preferably 0.5-6 mass%, in the total components. The use amount of the (+)-tetra- and/or -pentavalent vanadium compound is preferably 0.001-10 mass%, more preferably 0.005-8 mass%, particularly preferably 0.01-5 mass%, in the total components.

The use amount of the acidic group-containing radical-polymerizable monomer in the present dental pretreatment agent is preferably 3-50 mass%, more preferably 7-40 mass% in the total components. By applying a pretreatment agent containing the acidic monomer in the above amount range, to a tooth surface, a pretreatment providing a high adhesion strength to both of the dentin and the enamel can be conducted.

The use amount of water is preferably 5-90 mass%, more preferably 20-80 mass% in the total components. In this amount range, an increase in adhesivity is particularly striking.

When the present dental pretreatment agent is applied to a tooth surface, a non-acidic monomer and an organic solvent may be used suitably, in addition to the above-mentioned components.

As to the non-acidic monomer, there is no particular restriction and a known radical-polymerizable monomer containing no acidic group can be used. Specifically, the non-acidic monomers mentioned in the section of the present curable composition can be used. As preferred examples of the non-acidic monomer, there can be mentioned water-soluble, non-acidic monofunctional monomers such as 2-hydroxyethyl methacrylate and 2,3-dihydropropyl methacrylate; and non-acidic polyfunctional monomers such as triethylene glycol dimethacrylate, trimethylolpropane trimethacrylate, 2,2-bis[4-(2-hydroxy-3-(meth)acryloxypropoxy)phenyl]propane, 2,2-bis[(4-(meth)acryloxydiethoxy)phenyl]propane, 2,2-bis[(4-methacryloyloxypolyethoxy)phenyl]propane, 1,6-bis(methacrylethyloxycarbonylamino) 2,2,4-trimethylhexane and 1,6-bis(methacrylethyloxycarbonylamino) 2,4,4-trimethylhexane. The use amount of the non-acidic monomer is not particularly restricted, but is preferably 0.1-30 mass%, more preferably 1-20 mass% in the total components.

The organic solvent used is particularly preferably a water-soluble organic solvent. As specific examples of the water-soluble organic solvent, there can be mentioned alcohols such as methanol, ethanol, 1-propanol, 2-propanol, 1-butanol, 2-butanol, 2-methyl-1-propanol, 2-methyl-2-propanol, 1-pentanol, 2-pentanol, 3-pentanol, 2-methyl-1-butanol, isopentyl alcohol, 2-methyl-2-butanol, 3-methyl-2-butanol, 2,2-dimethyl-1-propanol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 3,5,5-trimethyl-1-hexanol, allyl alcohol, propargyl alcohol, cyclohexanol, 1-methylcyclohexanol, 2-methylcyclohexanol, 3-methylcyclohexanol, 4-methylcyclohexanol, abietinol, 1,2-ethanediol, 1,2-propanediol, 1,2-butanediol, 2,3-butanediol, 2-methyl-2,4-pentanediol and 1,2,6-hexanetriol; ether compounds such as triethylene glycol monomethyl ether, triethylene glycol monoethyl ether, dipropylene glycol monomethyl ether, tripropylene glycol monomethyl ether, 1,3-dioxolane, tetrahydrofuran, dimethoxyethane, 1,2-dimethoxyethane, 1,2-diethoxyethane, bis(2-methoxyethyl) ether and bis(2-ethoxyethyl) ether; ketone compounds such as acetone and methyl ethyl ketone; phosphoric acid esters such as hexamethylphosphoric acid triamide; acid amide compounds such as dimethylformamide and dimethylacetamide; carboxylic acid compounds such as acetic acid and propionic acid; and sulfur oxide type compounds such as dimethyl sulfoxide and sulfolane.

Of these water-soluble organic solvents, most preferred are water-soluble organic solvents having high safety to living bodies, such as ethanol, isopropanol and acetone.

By compounding the water-soluble organic solvent, the pretreatment agent can be made a homogeneous solution or a long-time-stable emulsion and can very easily treat an adherend surface uniformly. The use amount of the organic solvent is not particularly restricted, either, and can be determined appropriately. However, it is preferably 1-80 mass%, more preferably 3-50 mass% in the total components.

The pretreatment agent for tooth surface is superior particularly in the enhanced adhesivity to tooth surface as mentioned above, and is usable not only for the tooth surface but also as a pretreatment agent for various other dental materials such as dental metal and dental ceramic.

Particularly, a pretreatment agent containing, as the acidic group-containing radical-polymerizable monomer, a monomer having a group derived from phosphoric acid, for example, -O-P(=O) (OH)₂ or -O-P(=O) (OH) (OR₈) (R₈ has the same definition as given above) is superior in the enhanced adhesivity to base metal, ceramic or composite resin.

By compounding the above-mentioned adhering-to-precious-metal monomer in the pretreatment agent, the pretreatment agent can have strikingly enhanced adhesivity to dental precious metal.

In producing a pretreatment agent exclusively used for a dental precious metal or for a dental ceramic, the composition may be one suitable for such an application and is not restricted to those mentioned above.

To the present dental pretreatment agent can be further added, as necessary, substances known as additives for pretreatment agent. Such additives are exemplified by thickening agent, polymerization inhibitor, polymerization-regulator, ultraviolet absorber, metal salt, inorganic or organic acid, dye, pigment, other chemical polymerization type radical polymerization catalyst and photopolymerization catalyst.

The package form and the use manner of the present dental pretreatment agent are not particularly restricted and a known package form and a known use manner may be employed. As an example of the package form, there can be mentioned a form consisting of a package containing mainly an acidic monomer, a vanadium compound and optional components such as non-acidic monomer and organic solvent, and a package containing mainly water and an aryl borate compound. As the use manner, there can be mentioned a manner in which the two packages are mixed right before use, the resulting mixture is applied on a tooth surface and allowed to stand for 1-120 seconds, then e.g. compressed air is sprayed for drying, thereafter an appropriate adhesive is applied and cured.

### (4) Dental adhesive kit

The present radical polymerization catalyst can be used in a state that the total components of the aryl borate compound, the acidic group-containing radical-polymerizable monomer and the (+)-tetra- and/or -pentavalent vanadium compound are compounded in one dental material, as shown in the above-mentioned compounding of various dental materials. It is also possible that the individual components are appropriately compounded in a plurality of different dental materials used in combination and the total components of the radical polymerization catalyst are mixed finally when the plurality of materials are actually used in combination.

As a representative example of the dental materials used in combination, there is a combination of a dental pretreatment agent and a dental adhesive (hereinafter, this combination is referred to as the present dental adhesive kit) .

Prior to the application of a dental adhesive, treatment of an adherend surface with a pretreatment agent is generally conducted, as described in detail in the section of the present dental pretreatment agent. When there is thus employed an adhesion method of using a dental pretreatment agent and a dental adhesive (they are in separate storage) in this order, it is not necessary that all the components (three components) constituting the present radical polymerization catalyst are compounded in each of the dental pretreatment agent and the dental adhesive. It is possible that the three components are compounded in either of the dental pretreatment agent and the dental adhesive separately, and the dental pretreatment agent and the dental adhesive are used in this order, whereby all of the three components of the present radical polymerization catalyst are allowed to be present together finally; thereby, high adhesivity can be obtained.

As to the distribution of the three components in the dental pretreatment agent and the dental adhesive, there is no particular restriction; however, the following modes are particularly suitable because higher adhesivity can be obtained.

### (4-1) First dental adhesive kit

The first dental adhesive kit is one constituted by a dental pretreatment agent and a dental adhesive containing an aryl borate compound and, as the acidic compound, an acidic group-containing radical-polymerizable monomer, wherein a (+)-tetra- and/or -pentavalent vanadium compound is compounded in at least either of the pretreatment agent and the adhesive. Particularly preferred is one in which the pretreatment agent further contains an acidic monomer and water.

The (+)-tetra- and/or -pentavalent vanadium compound may be compounded in any of the dental pretreatment agent and the dental adhesive, as mentioned above; however, it is preferably compounded in the pretreatment agent because high adhesivity can be obtained.

By compounding an aryl borate compound in the dental adhesive as described above, even when the dental pretreatment agent contains no aryl borate compound, there can be obtained the same high adhesivity as when the pretreatment agent contains the aryl borate compound and, moreover, the pretreatment agent has higher stability and has a longer working life.

Preferable specific compounds and use amounts of the individual components (including optional components) used in the dental pretreatment agent and the dental adhesive are the same (excluding the vanadium compound) as in the above-mentioned present dental adhesive (adhesive for direct restoration, CR type resin cement, MMA type resin cement, resin-reinforced type glass ionomer cement for luting) and dental pretreatment agent.

Preferable specific examples and use amount of the (+)-tetra- and/or -pentavalent vanadium compound are also basically the same as mentioned above. However, in the present dental adhesive kit, the vanadium compound may be used in either one of the dental pretreatment agent and the dental adhesive and no vanadium compound need not be present in other material.

The use manner of the dental adhesive kit is the same as explained above for the present dental pretreatment agent.

The dental composition containing the present radical polymerization catalyst is usable not only in the applications specifically described above but also in other known applications, for example, surface brightener for composite resin and denture base resin, without any particular restriction.

### (4-2) Second dental adhesive kit

The second dental adhesive kit is an adhesive kit constituted by a dental pretreatment agent containing an acidic group-containing radical-polymerizable monomer, a (+)-tetra- and/or -pentavalent vanadium compound and water, and a dental adhesive containing an aryl borate compound, a non-acidic monomer, an organic peroxide and a photopolymerization catalyst.

With the above mentioned adhesive kit, since the dental adhesive contains an organic peroxide and a photopolymerization catalyst, a high adhesion strength can be obtained even if no acidic monomer is used in the adhesive.

Also, containing no aryl borate compound, the pretreatment agent can be stored in one package.

Specific examples of the acidic group-containing radical-polymerizable monomer, (+)-tetra- and/or -pentavalent vanadium compound and water compounded in the dental pretreatment agent constituting the above adhesive kit are the same as specifically mentioned with respect to the pretreatment agent for treatment of tooth surface, in the section of the present dental pretreatment agent.

For the same reason as described in the section of the present dental pretreatment agent, the dental pretreatment agent of the second dental adhesive kit can compound, for various purposes, components other than mentioned above, in such amounts that the properties of the pretreatment agent are not impaired.

As described above, the dental pretreatment agent can be stored in one package because no aryl borate compound is needed therein. The dental pretreatment agent can further compound a basic compound and/or a polymerization inhibitor, whereby the storage stability of the pretreatment agent can be improved.

By compounding a basic compound in the pretreatment agent, the pretreatment agent can have strikingly improved storage stability when the agent has been stored in one package. When the amount of the basic compound added is too large, the whole portion of the acidic monomer is neutralized and the decalcification function of the acidic monomer is lost; therefore, the amount of the basic compound added is preferably such that the pH of the pretreatment agent becomes at least 4 or less, preferably 3 or less. To obtain high storage stability, the pH is preferred to be high and the basic compound is added so as to give a pH of preferably 1.5 or more, more preferably 1.6 or more.

The basic compound added for the above purpose is not particularly restricted, but is preferably an amine compound in view of that the pH control therewith is easy and, when the compound is added to the pretreatment agent, there is no reduction in adhesion strength to teeth. As specific examples of the amine compound, there can be mentioned primary amine compounds such as butylamine; secondary amine compounds such as dibutylamine; and tertiary amine compounds such as tributylamine, triethanolamine, N,N-dimethylaminoethyl methacrylate, N,N-dimethylamino-p-toluidine, N,N-diethanol-p-toluidine, N,N-dimehtylaminoacetophenone and ethyl 4-dimethylaminobenzoate. Of these, tertiary amine compounds are particularly preferred.

The preferred use amount of the amine compound differs depending upon the kinds and amounts of other components used, but is generally 0.1-10 mass%, preferably 1-5 mass% in the pretreatment agent.

The polymerization inhibitor is added to prevent the gelling of the present pretreatment agent during storage and improve the storage stability, preferably in such an amount that there is no striking adverse effect on adhesion strength. As specific examples of the polymerization inhibitor, there can be mentioned hydroquinone, hydroquinone monomethyl ether and 2,6-di-tertiarybutylphenol. The use amount of the polymerization inhibitor is generally 0.00001-5 mass%, preferably 0.0001-1 mass% in the pretreatment agent.

As described above, the pretreatment agent can be stored in one package where all the components are present in one solution, unless there is compounded no optional component (e.g. aryl borate compound against acidic monomer) which invites instability when present in the package. Very high storage stability can be obtained when in particular, the above-mentioned amine compound is compounded. Needless to say, it is possible that, for example, when a compound making single packaging difficult is used, the components are stored in two or more appropriate packages and they are mixed when used actually.

In the second dental adhesive kit, the adhesive containing the above-mentioned non-acidic monomer, aryl borate compound, organic peroxide and photopolymerization catalyst is used to an adherend surface treated with the above-mentioned pretreatment agent, whereby a high adhesion strength can be obtained.

Specific examples of the non-acidic monomer are the same as explained in the section of the present curable composition.

The kind of the non-acidic monomer used may be appropriately selected depending upon the application and purpose of the adhesive used. However, to obtain a high adhesion strength and adhesion durability, both a non-acidic polyfunctional monomer and a water-soluble non-acidic monomer mentioned in the section of the present dental adhesive are used preferably. Particularly preferably, there are used the water-soluble non-acidic monomer in an amount of 5-40 mass% and the polyfunctional non-acidic monomer in an amount of 10-85 mass% in the total radical-polymerizable monomers constituting the adhesive.

Also, the specific examples and the preferred kind of the aryl borate compound are the same as explained in the section of the present curable composition. The use amount of the aryl borate compound is not particularly restricted, but is preferably 0.01-10 pbm, more preferably 0.1-8 pbm relative to 100 pbm of the total radical-polymerizable monomers constituting the adhesive.

In the present second adhesive kit, an organic peroxide is essential as a component of the dental adhesive. The organic peroxide is not particularly restricted and a known organic peroxide can be used with no particular restriction. As representative organic peroxides, there are preferred known organic peroxides which are classified into ketone peroxide, peroxyketal, hydroperoxide, diaryl peroxide, peroxy ester, diacyl peroxide and peroxy dicarbonate.

As the ketone peroxide, there can be specifically mentioned methyl ethyl ketone peroxide, cyclohexanone peroxide, methylcyclohexanone peroxide, methylacetoacetate peroxide and acetylacetone peroxide. As the peroxyketal, there can be mentioned 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate and 2,2-bis(4,4-di-t-butylperoxycyclohexyl)propane. As the hydroperoxide, there can be mentioned p-methane hydroperoxide, diisopropylbenzene peroxide, 1,1,3,3-tetramethylbutyl hydroperoxide, cumene hydroperoxide, t-hexyl hydroperoxide and t-butyl hydroperoxide. As the dialkyl peroxide, there can be mentioned α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide and 2,5-dimethyl-2,5-bis(t-butylperoxy)hexane-3. As the diacyl peroxide, there can be mentioned isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide and benzoyl peroxide. As the peroxy carbonate, there can be mentioned di-n-propyl peroxy dicarbonate, di-isopropyl peroxy dicarbonate, bis(4-t-butylcyclohexyl) peroxy dicarbonate, di-2-ethoxyethyl peroxy dicarbonate, di-2-ethylhexyl peroxy dicarbonate, di-2-methoxybutyl peroxy dicarbonate and di(3-methyl-3-methoxybutyl) peroxy dicarbonate. As the peroxy ester, there can be mentioned α,α-bis(neodecanoyl peroxy) diisopropylbenzene, cumyl peroxy neodecanoate, 1,1,3,3-tetramethylbutyl peroxy neodecanoate, 1-cyclohexyl-1-methylethyl peroxy neodecanoate, t-hexyl peroxy neodecanoate, t-butyl peroxy neodecanoate, t-hexyl peroxy pivalate, t-butyl peroxy pivalate, 1,1,3,3-tetramethylbutyl peroxy-2-ethylhexanoate, 2,5-dimethyl-2,5-bis(2-ethylhexanoyl peroxy)hexane, 1-cyclohexyl-1-methylethyl peroxy-2-ethylhexanoate, t-hexyl peroxy-2-ethylhexanoate, t-butyl peroxy-2-ethylhexanoate, t-butyl peroxy isobutyrate, t-hexyl peroxy isopropyl monocarbonate, t-butyl peroxy maleic acid, t-butyl peroxy-3,5,5-trimethylhexanoate, t-butyl peroxy laurate, 2,5-dimethyl-2,5-bis(m-toluoyl peroxy)hexane, t-butyl peroxy isopropyl monocarbonate, t-butyl peroxy-2-ethylhexyl monocarbonate, t-hexyl peroxy benzoate, 2,5-dimethyl-2,5-bis(benzoyl peroxy)hexane, t-butyl peroxy acetate, t-butyl peroxy-m-toluoylbenzoate, t-butyl peroxy benzoate and bis(t-butyl peroxy)isophthalate.

There can also be used, as preferred organic peroxides, t-butyltrimethylsilyl peroxide and 3,3',4,4'-tetra(t-butyl peroxy carbonyl)benzophenone.

The organic peroxide used can be appropriately selected depending upon the structures and amounts of the non-acidic monomer and aryl borate compound used in combination. The organic peroxides can be used singly or in combination of two or more kinds. Of them, there are preferred ketone peroxides, peroxy esters, hydroperoxides and diacyl peroxides for the polymerization activity. Of these, particularly preferred are organic peroxides whose 10-hour half-life period temperature is 60°C or more, for the storage stability when added into the present curable composition.

The use amount of the organic peroxide is not particularly restricted and can be determined appropriately depending upon the kinds and amounts of the polymerizable monomers and the amounts of other components, but it is preferably 0.01-10 pbm, more preferably 0.1-8 pbm relative to 100 pbm of the total radical-polymerizable monomers constituting the adhesive.

A photopolymerization catalyst is compounded in the adhesive of the second adhesive kit of the present invention. By compounding the photopolymerization catalyst, the adhesive is cured by light irradiation and can exhibit adhesivity.

The photopolymerization catalyst is not particularly restricted and a known photopolymerization catalyst for radical-polymerizable monomer can be used. Specific examples thereof, preferred kinds thereof and a preferred use amount thereof are the same as explained in the section of the present curable composition.

The photopolymerization catalyst can be used in one kind or, as necessary, in combination of a plurality of kinds.

The dental adhesive in the present second adhesive kit, when combined with the above-mentioned dental pretreatment agent, functions as a photo-polymerizing type adhesive showing sufficient adhesivity to dentin as long as the adhesive contains the above-mentioned non-acidic monomer, aryl borate compound and photopolymerization catalyst. In this dental adhesive, there may be compounded, as necessary, various components described in the section of the present dental adhesive, such as acidic compound (e.g. acidic monomer), vanadium compound, organic, inorganic or organic-inorganic composite filler, polymerization inhibitor, water, organic solvent, polymerization regulator, ultraviolet absorber, metal (other than vanadium) salt, dye, pigment, anti-microbial agent, acid-multiplication agent, polymeric thickener and thermal polymerization catalyst or redox polymerization catalyst (other than organic peroxide). The kinds and use amounts of these various components are the same as described in the section of the present dental adhesive.

By adding, in particular, an acidic compound and a vanadium compound and using a package form shown below, the present second adhesive kit can become an adhesive kit having both of the function of a photo-curing type adhesive and the function of a chemical curing type adhesive.

That is, the adhesive kit having such two functions is a dental adhesive kit constituted by the above-mentioned pretreatment agent package, an adhesive package B1 containing the above-mentioned non-acidic monomer, aryl borate compound, organic peroxide, photopolymerization catalyst and optional components (e.g. polymerization inhibitor and filler), and an adhesive package B2 containing an acidic compound, a vanadium compound and optional components (e.g. polymerization inhibitor, filler and non-acidic monomer).

As described above, the adhesive kit constituted by the pretreatment agent package and the adhesive package B1 is a photo-curing type adhesive kit which is cured by light irradiation and exhibits a high adhesion strength. By mixing the adhesive packages B1 and B2 as necessary, there can be obtained an adhesive which cures with no light irradiation. This adhesive has an advantage in that even when sufficient light irradiation is impossible to, for example, a deep cavity to be restored, the adhesive cures completely and therefore is highly reliable.

Thus, adhesion is made possible only with the package B1 used as a photo-curing type adhesive when sufficient light irradiation is possible. When no sufficient light irradiation is expected, the package B1 and the package B2 are mixed and the mixture can be used as a chemical curing type adhesive. Thereby, an adhesive kit having both of the function of a photo-curing type adhesive and the function of a chemical curing type adhesive can be constituted by three packages consisting of the pretreatment agent package, the adhesive package B1 and the adhesive package B2. This is a kit of far simpler constitution as compared with when there are prepared an adhesive kit exclusively used for photo-curing and an adhesive kit exclusively used for chemical curing. The adhesive kit of this constitution is easy to manage and low in operational mistake. In dividing the adhesive into the above two packages, sufficient storage stability can be obtained when the acidic compound and the aryl borate compound, the organic peroxide and the vanadium compound are not packed in the same package. Therefore, there is no problem even if, for example, the non-acidic monomer is present in the package B2.

As to the production method for each package constituting the present second adhesive kit, there is no particular restriction. Each package can be produced according to a known method, as in the above-mentioned case of the dental pretreatment agent or the dental adhesive. The second adhesive kit can be used in the same manner as described in the section of the present dental pretreatment agent, similarly to the case of a known kit consisting of a dental pretreatment agent and an adhesive. That is, the pretreatment agent is applied on an adherend surface using e.g. a small brush or a sponge; after 5-120 seconds, e.g. air blowing is applied for drying; then, the adhesive is applied on the pretreatment agent-applied site; thereafter, a light is irradiated to the adhesive for curing. Even when no sufficient light irradiation is possible, chemical curing takes place in ten and odds seconds to several minutes by using, as the adhesive, the above-mentioned adhesive containing an acidic monomer and a vanadium compound.

As described above in detail, the present radical polymerization catalyst gives a cured material free from initial color or discoloration, is high in polymerization activity even in the presence of oxygen and an acidic compound, is easy to handle and, when used in, for example, dental adhesive, gives an appropriately sufficient operation time.

The present dental adhesive for direct restoration (bonding agent) requires no pretreatment conducted heretofore, in adhesion between teeth and dental restorative material typified by composite resin, and promises a high adhesion strength to both dentin and enamel. Moreover, in its use, no light irradiation is required and high adhesivity is obtained not only for a photo-curing type dental restorative material but also for a chemical polymerization type dental restorative material.

The present dental adhesive for indirect restoration (dental cement), as compared with conventional adhesive dental cements, can exhibit a high adhesion strength to both dentin and enamel.

The present dental restorative material is free from initial color or discoloration, has a high strength, and can conduct restoration of excellent appearance and high reliability.

Further, the present dental pretreatment agent, as compared with conventional dental pretreatment agents, can allow, in one pretreatment operation, each of a photo-curing type adhesive and a chemical polymerization type adhesive to show a high adhesion strength to the enamel and dentin of tooth.

Furthermore, in the present adhesive kit, all of the three components constituting the present radical polymerization catalyst need not be compounded in either of the dental adhesive and the dental pretreatment agent, and a high adhesion strength can be obtained as long as the three components are compounded totally in the adhesive and the pretreatment agent in any desired combination.

Moreover, in the present second adhesive kit, by appropriately distributing the three components constituting the present radical polymerization catalyst, in the dental adhesive and the dental pretreatment agent and further compounding a photopolymerization catalyst and an organic peroxide in the adhesive, it is possible to constitute an adhesive kit which can be used as a photo-curing type or as a chemical curing type depending upon the necessity, in three packages, i.e. only one pretreatment agent package and two adhesive packages.

### Examples

The present invention is specifically described below by way of Examples.

The compounds used in Examples and Comparative Examples and their abbreviations are shown in (1); the method for measurement of curing time is shown in (2); the methods for measurement of properties of each cured material are shown in (3)-(5); and the methods for measurement of adhesion strength when the present dental adhesive, dental pretreatment agent or dental adhesive kit has been used, are shown in (6)-(8).

### (1) Abbreviations and structures

### [Acidic group-containing radical-polymerizable monomers (acidic monomers)]

- PM:: a mixture of 2-methacryloyloxyethyl dihydrogen phosphate and bis(2-methacryloyloxyethyl) hydrogenphosphate (molar ratio: 1:4)
- MAC-10:: 11-methacryloyloxy-1,1-undecanedicarboxylic acid
- 4-META:: 4-methacryloyloxyethyltrimellitic acid anhydride
- MMPS:: 2-methacrylamide-2-methylpropanesulfonic acid

### [Radical-polymerizable monomers other than acidic group-containing radical-polymerizable monomers (non-acidic monomers)]

- MMA:: methyl methacrylate
- TMPT:: trimethylolpropane trimethacrylate
- BisGMA:: 2,2-bis[4-(2-hydroxy-3-methacryloyloxypropoxy) phenyl] propane
- 3G:: triethylene glycol dimethacrylate
- D2.6E:: 2,2-bis[(4-methacryloyloxypolyethoxyphenyl)propane] (a mixture wherein the average of repeating of ethoxy moiety is 2.6)
- NPG:: neopentyl glycol dimethacrylate
- UDMA:: a mixture of 1,6-bis(methacrylethyloxycarbonylamino)-2,2,4-trimethylhexane and 1,6-bis(methacrylethyloxycarbonylamino)-2,4,4-trimethylhexane
- HEMA:: 2-hydroxyethyl methacrylate
- MTU-6:: 6-methacryloyloxyhexyl-2-thiouracyl-5-carboxylate

### [Aryl borate compounds]

PhBNa: tetraphenyl borate sodium salt
PhBTEOA: tetraphenyl borate triethanolamine salt
PhBDMPT: tetraphenyl borate dimethyl-p-toluidine salt
PhBDMBE: tetraphenyl borate (ethyl dimethylaminobenzoate) salt
FPhBNa: tetrakis(p-fluorophenyl) borate sodium salt
BFPhBNa: butyl tri(p-fluorophenyl) borate sodium salt

### [Vanadium compounds]

- VOSO₄:: vanadyl (IV) sulfate
- VOAA:: vanadium (IV) oxide acetylacetonate
- OPBV:: oxobis(1-phenyl-1,3-butanedionate) vanadium (IV)
- BMOV:: bis(maltolato) oxovanadium (IV)
- V₂O₅:: vanadium (V) oxide
- VAA:: vanadium (III) acetylacetonate
- VCl₂:: vanadium (II) chloride

### [Fillers]

### • Organic fillers

- PEMA:: polyethyl methacrylate (weight-average molecular weight: 300,000, average particle diameter: 30 µm)
- PMMA:: polymethyl methacrylate (weight-average molecular weight: 400,000, average particle diameter: 25 µm)
- P(MMA-EMA):: methyl methacrylate-ethyl methacrylate copolymer (weight-average molecular weight: 400,000, average particle diameter: 30 µm)

### • Inorganic fillers

- 0.5Si-Zr:: spherical silica-zirconia, surface-treated with γ-methacryloyloxypropyltrimethoxysilane (average particle diameter: 0.5 µm)
- 0.06Si-Zr:: spherical silica-zirconia, surface-treated with γ-methacryloyloxypropyltrimethoxysilane (average particle diameter: 0.06 µm)
- 3Si-Zr:: irregular shaped silica-zirconia, surface-treated
- with: γ-methacryloyloxypropyltrimethoxysilane (average particle diameter: 3 µm)
- 0.3Si-Ti:: spherical silica-titania, surface-treated with γ-methacryloyloxypropyltrimethoxysilane (average particle diameter: 0.3 µm)
- MT10:: amorphous silica, surface-treated with monomethyltrichlorosilane (specific surface area: 120 m²/g)
- FASG:: fluoroaluminosilicate glass powder

### [Organic peroxides]

- BPO:: benzoyl peroxide
- Percumyl H:: cumene hydroperoxide
- Perocta H:: 1,1,3,3-tetramethylbutyl hydroperoxide

### [components of photopolymerization catalyst (other than amine compounds)]

- TCT:: 2,4,6-tris(trichloromethyl)-s-triazine
- CDAC:: 3,3'-carbonylbis(7-diethylamino)coumarin
- HMC:: 7-hydroxy-4-methyl-coumarin
- CM102:: 2,3,6,7-tetrahydro-9-methyl-1H,5H,11H-[1]-benzopyrano[6,7,8-ij]quinolidin-11-one
- BAPO:: bis(2,4,6-trimethylbenzoyl)-phenylphosphine oxide
- BDTPO:: bis(2,6-dimethoxybenzoyl)-2,4,4-trimethylpentylphosphine oxide
- CQ:: camphorquinone

### [Amine compounds]

- DMEM:: N,N-dimethylaminoethyl methacrylate
- DEPT:: N,N-diethanol-p-toluidine
- DMPT:: dimethylamino-p-toluidine [Polymerization inhibitors]
- BHT:: dibutylhydroxytoluene
- HQME:: hydroquinone monomethyl ether

### [Others]

- IPA:: isopropyl alcohol
- FeAA:: iron (III) acetylacetonate
- MDPB:: methacryloyloxydodecyl pyridinium

### (2) Measurement of curing time

Curing time was measured by an exothermic heat method using a thermistor thermometer. That is, 5 g of a radical-polymerizable monomer solution containing an acidic compound and a vanadium compound (a first solution) and 5 g of a radical-polymerizable monomer solution containing an aryl borate compound (a second solution) were mixed with stirring for 20 seconds to obtain a homogeneous solution. Then, the solution was poured into a teflon-made mold of 2 cm x 2 cm x 1 cm having a hole of 6 mm in diameter at the center. A thermistor thermometer was inserted into the solution; there was measured a time from the start of mixing to the recording of the maximum temperature; and the time was taken as the curing time of the solution mixture. The measurement was conducted in a thermostatic chamber of 23°C.

### (3) Evaluation of curability

Curability was evaluated based on the degree of curing and surface stickiness of the whole portion of cured material. A curable composition was prepared in the same manner as above, was poured into the same mold, and was cured in the air at 23°C for 15 minutes. The hardness and surface stickiness of the cured material obtained were evaluated each in 5 levels. That is, a composition which gave a cured material having a sufficient hardness and no surface stickiness, was rated as ⊚; a composition which gave a cured material cured as a whole, having a sufficient hardness, but sticky only at the surface, was rated as ○; a composition which gave a cured material cured in a jelly state as a whole and having residual monomers at the surface, was rated as Δ; a composition which gave a cured material being partially in a jelly state and having uncured portions, was rated as X; and a composition which was not cured at all, was rated as XX.

### (4) Tests for initial color and discoloration resistance of cured material

A test for discoloration resistance of a cured material was conducted as follows. First, individual components were mixed in given proportions and kneaded for 20 seconds. Then, the kneaded material was poured into a mold of 10 mm x 10 mm x 2 mm and cured at 37°C for 24 hours. The cured material obtained was visually evaluated for initial color, in 3 levels shown below.
Score 1: colorless and transparent
Score 2: yellow
Score 3: brown

The cured material was stored in water of 80°C for 60 days, and the discoloration of the cured material after storage was rated according to the following standard.
Score 1: no discoloration
Score 2: only cloudiness
Score 3: slight discoloration into yellow
Score 4: discoloration into yellow
Score 5: discoloration into brown

### (5) Measurement of bending strength and hardness

The bending strength of a cured material was measured by the following method. First, individual components were mixed homogeneously in given proportions, then poured into a mold of 25 mm x 4 mm x 2 mm, and cured at 37°C for 24 hours. The cured material obtained was subjected to a failure-in-bending test at a distance between fulcra, of 20 mm. The crosshead speed was 1 mm/min. In the measurement of hardness, the surface of the above cured material was subjected to buff polishing and the Knoop hardness of the resulting material was measured under a load of 10 g and 20 seconds using a small hardness tester produced by Matsuzawa Seiki. The measurement was conducted in a thermostat chamber of 23°C.

### (6) Measurement method 1 for adhesion strength (for direct restoration)

Foreteeth of the underjaw of cattle were extracted within 24 hours after slaughter. The flat surface of enamel or dentin was prepared by shaving the foreteeth with a #800 emery paper under injection water, so that the enamel or dentin surface became parallel to the lip surface. To the resulting surface was sprayed compressed air for 10 seconds, to dry the surface. Then, onto the dried surface was fixed a double face adhesive tape having a hole of 3 mm in diameter, to specify an adhesion area. Then, a wax of 1 mm in thickness having a hole of 8 mm in diameter was attached in a state that the center of the hole of the double face adhesive tape and the center of the hole of the wax were coincided with each other, whereby a mock cavity was formed. Onto this mock cavity was applied a dental adhesive for direct restoration prepared right before use, and the adhesive was allowed to stand for 20 seconds. When necessary, a pretreatment agent was applied onto the mock cavity prior to the applying of the adhesive and allowed to stand for 20 seconds; then, compressed air was sprayed for drying; thereby, the pretreatment of dental surface was conducted.

When a photo-curing type composite resin was used, there was filled, into the mock cavity applied with the dental adhesive, a photo-curing type composite resin (Palfique Estelite produced by Tokuyama K.K.); the composite resin was covered with a polypropylene-made sheet; a light was irradiated on the sheet for 30 seconds using Power Light (produced by Tokuyama K.K.) to polymerize and cure the composite resin to produce a test piece. When a chemical curing type composite resin was used, a chemical polymerization type composite resin (Palfique produced by Tokuyama K.K.) was filled in the same manner, and cured to produce a test piece.

The above-produced test piece was immersed in water of 37°C for 24 hours and then subjected to a tensile test using a tensile tester (Autograph AG 5000 produced by Shimadzu Corporation) at a crosshead speed of 1 mm/min. In one test, 8 adhesion test pieces were used and the average measurement value thereof was taken as adhesion strength.

### (7) Measurement method 2 for adhesion strength (for indirect restoration)

An adhesion area was specified in the same manner as in the measurement method 1 for adhesion strength. Then, for a case of conducting a treatment of tooth surface, a pretreatment agent was applied in a thin film state, the applied tooth was allowed to stand for 20 seconds, then, compressed air was sprayed for 5 seconds for drying.

Onto the tooth surface subjected or not subjected to the above pretreatment was applied a present adhesive for indirect restoration, prepared right before use; a stainless steel-made attachment of 8 mm in diameter was pressure-bonded to the applied adhesive to produce a test piece. The test piece was kept for 1 hour in an atmosphere of 37°C and 100% humidity, then immersed in water of 37°C for 24 hours, and subjected to a tensile test using a tensile tester (Autograph AG 5000 produced by Shimadzu Corporation) at a crosshead speed of 1 mm/min. In one test, 8 adhesion test pieces were used and the average measurement value thereof was taken as adhesion strength.

### (8) Measurement method 3 for adhesion strength (for direct restoration)

An adhesion area was specified in the same manner as in the measurement method 1 for adhesion strength. Then, a pretreatment agent was applied in a thin film state, the applied tooth was allowed to stand for 20 seconds, then, compressed air was sprayed for 5 seconds for drying. An adhesive was applied onto the pretreated tooth surface. Successively, a light was irradiated to the applied adhesive for 20 seconds using Power Light.

Successively, a composite resin was filled and cured to produce a test piece in the same manner as in the measurement method 1 for adhesion strength. The test piece was measured for various properties.

First, the present radical polymerization catalyst was evaluated for polymerization-initiating ability and the cured material obtained was evaluated for basic properties.

### Example 1

To 100 pbm of a MMA/TMPT (90 wt.%/10 wt.%) solution were added 5 pbm of PM (an acidic compound) and 0.005 pbm of VOAA (a vanadium compound) to prepare a homogeneous solution (a first solution). Separately, 3 pbm of PhBNa (an aryl borate compound) was added to a MMA/TMPT (90 wt.%/10 wt.%) solution to prepare a homogeneous solution (a second solution). The two solutions were mixed at a 1:1 mass ratio until the mixture became homogeneous, after which the mixture was evaluated for curing time, curability and surface stickiness when cured. Further, the cured material was evaluated for initial color and discoloration resistance. The results are shown in Table 1.

**Table 1**

| | Aryl borate compound | | Acidic compound | | Vanadium compound | | Other additive | | Curing time | Curability | Initial color | Resistance to discoloration |
|---|---|---|---|---|---|---|---|---|---|---|---|---|
| | | pbm | | pbm | | pbm | | pbm | | | | |
| Example 1 | PhBNa | 3 | PM | 5 | VOAA | 0.005 | - | - | 3'10" | ⊚ | 1 | 1 |
| Example 2 | PhBNa | 1 | PM | 5 | VOAA | 0.005 | - | - | 6'40" | ⊚ | 1 | 1 |
| Example 3 | PhBDMFT | 3 | PM | 5 | VOAA | 0.005 | - | - | 3'20" | ⊚ | 1 | 2 |
| Example 4 | PhBDMBE | 3 | PM | 5 | VOAA | 0.005 | - | - | 2'50" | ⊚ | 1 | 1 |
| Example 5 | FPhBNa | 3 | PM | 5 | VOAA | 0.005 | - | - | 3'10" | ⊚ | 1 | 1 |
| Example 6 | BFPhBNa | 3 | PM | 5 | VOAA | 0.005 | - | - | 2'40" | ⊚ | 1 | 1 |
| Example 7 | PhBTEOA | 3 | PM | 5 | VOAA | 0.005 | - | - | 2'40" | ⊚ | 1 | 1 |
| Example 8 | PhBNa | 3 | Phoshonic acid | 3 | VOAA | 0.005 | - | - | 3'00" | ○ | 1 | 1 |
| Example 9 | PhBNa | 3 | Nitric acid | 3 | VOAA | 0.005 | - | - | 2'40" | ○ | 1 | 1 |
| Example 10 | PhBNa | 3 | MMPS | 5 | VOAA | 0.005 | - | - | 2'50" | ⊚ | 1 | 1 |
| Example 11 | PhBNa | 3 | PM | 1 | VOAA | 0.005 | - | - | 6'30" | ⊚ | 1 | 1 |
| Example 12 | PhBNa | 3 | PM | 5 | VOAA | 0.01 | - | - | 1'50" | ⊚ | 1 | 1 |
| Example 13 | PhBNa | 3 | PM | 5 | VOSO₄ | 0.005 | - | - | 3'40" | ⊚ | 1 | 1 |
| Example 14 | PhBNa | 3 | PM | 5 | OPBV | 0.005 | - | - | 3'10" | ⊚ | 1 | 1 |
| Example 15 | PhBNa | 3 | PM | 5 | BMOV | 0.005 | - | - | 3'00" | ⊚ | 1 | 1 |
| Example 16 | PhBNa | 3 | PM | 5 | V₂O₅ | 0.005 | - | - | 2'50" | ⊚ | 1 | 1 |
| Example 17 | PhBTEOA | 3 | PM | 5 | BMOV | 0.005 | - | - | 2'40" | ⊚ | 1 | 1 |
| Example 18 | PhBTEOA | 3 | PM | 5 | BMOV | 0.005 | Percumyl H | 2 | 2'20" | ⊚ | 1 | 1 |
| Example 19 | PhBTEOA | 3 | PM | 5 | BMOV | 0.005 | Perocta H | 2 | 2'30" | ⊚ | 1 | 1 |

| | | pbm | | pbm | | part by mass | | pbm | | | | |
|---|---|---|---|---|---|---|---|---|---|---|---|---|
| Comparative Example 1 | - | - | PM | 5 | VOAA | 0.005 | - | - | No curing | - | - | - |
| Comparative Example 2 | PhBNa | 3 | - | - | VOAA | 0.005 | - | - | No curing | - | - | - |
| Comparative Example 3 | PhBNa | 3 | PM | 5 | - | - | - | - | >60'00" | × | 1 | - |
| Comparative Example 4 | PhBNa | 3 | PM | 6 | VCl₂ | 0.005 | - | - | >60'00" | × | 1 | - |
| Comparative Example 5 | PhBNa | 3 | PM | 5 | VAA | 0.005 | - | - | 7'00" | Δ | 1 | - |
| Comparative Example 6 | BPO (4) /DEPT (2) | | | | | | | | 3'00" | ○ | 2 | 5 |

### Examples 2-19 and Comparative Examples 1-6

There were prepared MMA/TMPT (90 wt.%/10 wt.%) solutions containing a radical polymerization catalyst shown in Table 1. The solutions were cured in the same manner as in Example 1. Each cured material was measured for properties. The results are shown in Table 1. The amounts of individual components in Table 1 are the respective amounts in the first or second solution. Percumyl H and Perocta H were compounded in the second solution, BPO was compounded in the first solution, and DEPT was compounded in the second solution.

In each of Examples 1-19, the curable composition containing a present radical polymerization catalyst was evaluated for curing speed, curability and initial color. As is clear from the above Table 1, in all the Examples each using a present radical polymerization catalyst, good curability was seen. Further, there was no initial color of cured material and the discoloration after the discoloration test was none or slight.

There was no surface stickiness in the cured materials of Examples (other than Examples 8 and 9) each using an acidic group-containing radical-polymerizable monomer, as compared with the cured materials of Examples 8 and 9 using, as an acidic compound, phosphoric acid or nitric acid (both are non-polymerizable). Therefore, they had better curability.

In Comparative Examples 1-3, either one of the essential components of the present radical polymerization catalyst was not used. In Comparative Example 1 or 2 containing no aryl borate compound or no acidic compound, the composition showed no curing. Further, in Comparative Example 3 containing no + tetravalent and/or + pentavalent vanadium compound, or in Comparative Example 4 using a + divalent vanadium compound, the composition became gel-like only partially after 1 hour and showed very low curability. In Comparative Example 5 using a + trivalent vanadium compound, the composition cured as a whole but the cured material was jelly. It is appreciated from this that curability was considerably inferior when a + trivalent vanadium compound is used, as compared with when a + tetravalent or + pentavalent vanadium compound is used.

In Comparative Example 6, a known chemical polymerization type (BPO/amine type) radical polymerization catalyst was used. In this case, the cured material had initial color and showed high discoloration after discoloration resistance test.

### Example 20

0.005 pbm of VOAA (a vanadium compound) was added to 200 pbm of PM to form a homogeneous solution. Thereto was added 3 pbm of PhBNa (an aryl borate compound), followed by mixing for 20 seconds. The resulting composition was evaluated for curability, initial color and discoloration resistance. As a result, the curability was O, the initial color was score 1, and the discoloration resistance was score 2.

### Comparative Example 7

4 pbm of BPO was added to 200 pbm of PM to form a homogeneous solution. Thereto was added 2 pbm of DMPT, followed by mixing for 20 seconds. The resulting composition was evaluated for curability, initial color and discoloration resistance. As a result, the curability was Δ, the initial color was score 2, and the discoloration resistance was score 5.

Next, compositions each containing a present radical polymerization catalyst were evaluated for properties when used as a dental adhesive for direct restoration.

### Example 21

There was prepared an adhesive A for direct restoration consisting of a first solution and a second solution each having a composition shown in Table 2 (the value of each component in Table 2 indicates pbm. The same applies in all Tables which follow.). The two solutions were mixed right before use to obtain an adhesive having compounding proportions shown in Table 2. Using this adhesive, an adhesion strength when a photo-curing type composite resin was used, was measured according to the measurement method 1 for adhesion strength. As shown in Table 3, the adhesion strength to enamel was 13.9 (2.2) MPa and the adhesion strength to dentin was 12.3 (2.6) MPa [( ) indicates a standard deviation].

**Table 2**

| | Composition of first solution | | | | | | Composition of second solution | | | | | |
|---|---|---|---|---|---|---|---|---|---|---|---|---|
| | Acidic monomer(s) | | Non-acidic monomers | | Vanadium compound | | Non-acidic monomers | | Aryl borate compound | | Other components | |
| A | PM | 25 | D2.6E | 15 | VOAA | 0.005 | HEMA | 30 | PhBTEOA | 3 | - | |
| | | | TMPT | 10 | | | MMA | 17 | | | | |
| B | PM | 25 | D2.6E | 15 | VOAA | 0.005 | HEMA | 30 | PhBTEOA | 3 | FASG | 7 |
| | | | TMPT | 10 | | | MMA | 10 | | | | |
| C | PM | 25 | D2.6E | 15 | VOAA | 0.005 | HEMA | 30 | PhBTEOA | 3 | Water | 5 |
| | | | TMPT | 10 | | | MMA | 12 | | | | |
| D | PM | 25 | D2.6E | 15 | VOAA | 0.005 | HEMA | 30 | PhBTEOA | 3 | FASG | 7 |
| | | | TMPT | 10 | | | MMA | 5 | | | Water | 5 |
| E | PM | 20 | D2.6E | 15 | VOAA | 0.005 | HEMA | 30 | PhBTEOA | 3 | FASG | 7 |
| | MAC-10 | 5 | TMPT | 10 | | | MMA | 5 | | | Water | 5 |
| F | PM | 20 | D2.6E | 15 | VOAA | 0.005 | HEMA | 30 | PhBTEOA | 3 | FASG | 7 |
| | 4-META | 5 | TMPT | 10 | | | MMA | 5 | | | Water | 5 |
| G | PM | 20 | D2.6E | 15 | VOAA | 0.005 | HEMA | 30 | PhBTEOA | 3 | FASG | 7 |
| | MAC-10 | 5 | TMPT | 5 | | | MMA | 5 | | | Water | 5 |
| | | | MMA | 5 | | | | | | | | |
| H | PM | 20 | BisGMA | 10 | VOAA | 0.005 | HEMA | 30 | PhBTEOA | 3 | FASG | 7 |
| | MAC-10 | 5 | 3G | 10 | | | MMA | 5 | | | Water | 5 |
| | | | MMA | 5 | | | | | | | | |
| I | PM | 20 | D2.6E | 15 | OPBV | 0.005 | HEMA | 30 | PhBTEOA | 3 | FASG | 7 |
| | MAC-10 | 5 | TMPT | 10 | | | MMA | 5 | | | Water | 5 |
| J | PM | 20 | D2.6E | 15 | BMOV | 0.005 | HEMA | 30 | PhBTEOA | 3 | FASG | 7 |
| | MAC-10 | 5 | TMPT | 10 | | | MMA | 5 | | | Water | 5 |
| K | PM | 20 | D2.6E | 15 | VOAA | 0.005 | HEMA | 30 | PhBNa | 3 | FASG | 7 |
| | MAC-10 | 5 | TMPT | 10 | | | MMA | 5 | | | Water | 5 |
| L | PM | 20 | D2.6E | 15 | VOAA | 0.005 | HEMA | 30 | PhBDMPT | 3 | FASG | 7 |
| | MAC-10 | 5 | TMPT | 10 | | | MMA | 5 | | | Water | 5 |

| | Acidic monomer(s) | | Non-acidic monomers | | Vanadium compound | | Non-acidic monomers | | Aryl borate compound | | Other component | |
|---|---|---|---|---|---|---|---|---|---|---|---|---|
| M | PM | 20 | D2.6E | 15 | VOAA | 0.005 | HEMA | 30 | PhBTEOA | 3 | FASG | 7 |
| | MAC-10 | 5 | TMPT | 10 | | | MMA | 4 | | | Water | 5 |
| | | | | | | | | | | | Perocta H | 1 |
| N | PM | 20 | D2.6E | 15 | VOAA | 0.005 | HEMA | 30 | PhBTEOA | 3 | - | |
| | MAC-10 | 5 | TMPT | 10 | | | MMA | 17 | | | | |
| O | PM | 20 | D2.6E | 15 | VOAA | 0.005 | HEMA | 30 | PhBTEOA | 3 | FASG | 7 |
| | MAC-10 | 5 | TMPT | 10 | | | MMA | 10 | | | | |
| P | PM | 20 | D2.6E | 15 | VOAA | 0.005 | HEMA | 30 | PhBTEOA | 3 | Water | 5 |
| | MAC-10 | 5 | TMPT | 10 | | | MMA | 12 | | | | |
| Q | - | | D2.6E | 30 | VOAA | 0.005 | HEMA | 30 | PhBTEOA | 3 | FASG | 7 |
| | | | TMPT | 10 | | | MMA | 5 | | | Water | 5 |
| | | | MMA | 10 | | | | | | | | |
| R | PM | 20 | D2.6E | 15 | - | | HEMA | 30 | PhBTEOA | 3 | FASG | 7 |
| | MAC-10 | 5 | TMPT | 10 | | | MMA | 5 | | | Water | 5 |
| S | PM | 20 | D2.6E | 15 | VOAA | 0.005 | HEMA | 30 | - | | FASG | 7 |
| | MAC-10 | 5 | TMPT | 10 | | | MMA | 5 | | | Water | 5 |
| T | PM | 20 | D2.6E | 15 | BPO | 1 | HEMA | 30 | - | | FASG | 7 |
| | MAC-10 | 5 | TMPT | 10 | | | MMA | 8 | | | Water | 5 |
| | | | | | | | | | | | DMPT | 1 |

| | | | | | | | | | | | | |
|---|---|---|---|---|---|---|---|---|---|---|---|---|
| *FASG is a polyvalent metal ion-releasing filler. | | | | | | | | | | | | |

**Table 3**

| | Direct restorative adhesive used | Direct filling restorative material used | Adhesion strength /MPa (S.D.) | |
|---|---|---|---|---|
| | | | Enamel | Dentin |
| Example 21 | A | Photo-curing type | 13.9 (2.2) | 12.3 (2.6) |
| Example 22 | B | Photo-curing type | 14.2 (3.3) | 15.9 (3.6) |
| Example 23 | C | Photo-curing type | 17.2 (2.9) | 14.8 (3.0) |
| Example 24 | D | Photo-curing type | 20.3 (2.8) | 18.0 (2.8) |
| Example 25 | E | Photo-curing type | 20.2 (1.9) | 20.1 (3.0) |
| Example 26 | F | Photo-curing type | 20.1 (2.3) | 20.5 (3.2) |
| Example 27 | G | Photo-curing type | 21.1 (1.3) | 20.6 (2.9) |
| Example 28 | H | Photo-curing type | 20.9 (2.6) | 19.9 (3.2) |
| Example 29 | I | Photo-curing type | 21.3 (2.6) | 20.8 (2.9) |
| Example 30 | J | Photo-curing type | 21.0 (1.8) | 21.0 (1.8) |
| Example 31 | K | Photo-curing type | 20.0 (2.5) | 19.8 (2.1) |
| Example 32 | L | Photo-curing type | 19.8 (2.7) | 19.0 (1.7) |
| Example 33 | M | Photo-curing type | 21.2 (2.8) | 20.9 (4.1) |
| Example 34 | N | Chemical curing type | 12.3 (2.3) | 11.9 (3.0) |
| Example 35 | O | Chemical curing type | 14.0 (3.1) | 14.3 (2.1) |
| Example 36 | P | Chemical curing type | 16.2 (2.2) | 14.0 (2.6) |
| Example 37 | E | Chemical curing type | 19.2 (3.0) | 17.8 (2.9) |
| Example 38 | H | Chemical curing type | 19.0 (2.3) | 17.9 (3.0) |
| Example 39 | I | Chemical curing type | 20.1 (2.1) | 18.1 (3.6) |
| Comparative Example 8 | Q | Photo-curing type | 0 | 0 |
| Comparative Example 9 | R | Photo-curing type | 2.3 (0.9) | 0 |
| Comparative Example 10 | S | Photo-curing type | 0 | 0 |
| Comparative Example 11 | T | Photo-curing type | 8.5 (1.29 | 5.5 (2.2) |
| Comparative Example 12 | Q | Chemical curing type | 0 | 0 |
| Comparative Example 13 | R | Chemical curing type | 1.9 (0.3) | 0 |
| Comparative Example 14 | S | Chemical curing type | 0 | 0 |
| Comparative Example 15 | T | Chemical curing type | 7.9 (2.1) | 5.0 (1.8) |

### Examples 22-39 and Comparative Examples 8-15

There were prepared adhesives B to T for direct restoration, all consisting of a first solution and a second solution each having a composition shown in Table 2. The two solutions were mixed right before use to obtain adhesives having compounding proportions shown in Table 2. Using each adhesive, the adhesion strength was measured according to the measurement method 1 for adhesion strength. The kinds of the composite resins used and the measurement results of adhesion strengths are shown in Table 3.

As is clear from Table 3, adhesives for direct restoration each containing a present radical polymerization catalyst showed high adhesion strengths to both the enamel and the dentin without conducting any pretreatment. Further, higher adhesion strengths were obtained when a polyvalent metal ion-releasing filler and/or water was added.

As shown in Comparative Examples, adhesives not using either of the components of the present polymerization catalyst, i.e. an aryl borate compound, an acidic compound (an acidic monomer) and a vanadium compound showed each a very low adhesion strength.

Also, in Comparative Examples 11 and 15 using a polymerization catalyst of BPO/amine type, the adhesives per se cured but gave very low adhesion strengths.

### Example 40

There was prepared a dual cure type adhesive U for direct restoration, containing a present radical polymerization catalyst. The composition is shown in Table 4. The adhesive was applied onto a mock cavity and allowed to stand for 20 seconds. Using Power Light, a light was irradiated to the adhesive from a distance of 10 cm from the cavity for 10 seconds. Immediately thereafter, a photo-curing type composite resin was filled and cured. At this time, the light intensity near the adhesive surface was 50 mW/cm². The adhesion strength between tooth and composite resin was measured and the result is shown in Table 5.

**Table 4**

| | Composition of first solution | | | | | | | | Composition of second solution | | | | | |
|---|---|---|---|---|---|---|---|---|---|---|---|---|---|---|
| | Acidic monomers | | Non-acidic monomers | | Vanadium compound | | Other component(s) | | Non-acidic monomers | | Aryl borate compound | | Other components | |
| U | PM | 20 | D2.6E | 15 | VOAA | 0.005 | TCT | 1 | HEMA | 30 | PhBTEOA | 3 | FASG | 7 |
| | MAC-10 | 4 | TMPT | 10 | | | | | MMA | 5 | | | Water | 5 |
| | | | | | | | | | | | | | CDAC | 0.01 |
| V | PM | 20 | D2.6E | 15 | VOAA | 0.005 | CQ | 0.5 | HEMA | 30 | PhBTEOA | 3 | FASG | 7 |
| | MAC-10 | 4 | TMPT | 10 | | | DMBE | 0.5 | MMA | 5 | | | Water | 5 |
| W | PM | 20 | D2.6E | 15 | - | | TCT | 1 | HEMA | 30 | PhBTEOA | 3 | FASG | 7 |
| | MAC-10 | 4 | TMPT | 10 | | | BPO | 2 | MMA | 5 | | | Water | 5 |
| | | | | | | | | | | | | | CDAC | 0.01 |
| | | | | | | | | | | | | | DMPT | 2 |
| X | PM | 20 | D2.6E | 15 | - | | TCT | 1 | HEMA | 30 | PhBTEOA | 3 | FASG | 7 |
| | MAC-10 | 4 | TMPT | 10 | | | | | MMA | 5 | | | Water | 5 |
| | | | | | | | | | | | | | CDAC | 0.01 |

| | | | | | | | | | | | | | | |
|---|---|---|---|---|---|---|---|---|---|---|---|---|---|---|
| TCT 2,4,6-Tris(chloromethyl)-s-triazine (a photo-induced acid-generating agent) CQ Campherquinone (α-diketone compound) DMBE Ethyl 4-dimethylaminobenzoate (an amine compound) BPO Benzoyl peroxide (an organic peroxide) FASG Fluoroaluminosilicate glass (a polyvalent ion-releasing filler) CDAC 3,3'-Carbonyl-bis (7-diethylamino) coumarin DMPT Dimethilamino-p-toluidine (an amine compound) | | | | | | | | | | | | | | |

**Table 5**

| | Direct restorative adhesive used | Intensity of light irradiated to adhesive | Adhesion strength /MPa(S.D.) | |
|---|---|---|---|---|
| | | | Enamel | Dentin |
| Example 40 | U | 50 | 21.1 (2.8) | 20.9 (3.5) |
| Example 41 | V | 50 | 21.0 (2.1) | 20.6(4.1) |
| Comparative Example 16 | W | 50 | 8.8 (2.9) | 5.7 (1.5) |
| Comparative Example 17 | X | 50 | 8.0 (2.2) | 5.1 (1.9) |
| Reference Example 1 | X | 700 | 20.8 (3.0) | 19.0 (3.1) |

### Example 41 and Comparative Examples 16-17

Adhesives V, W and X for direct restoration were prepared in compositions shown in Table 4. Using these adhesives, adhesion strengths between tooth and composite resin were measured in the same manner as in Example 40. The results are shown in Table 5.

### Reference Example 1

Using the adhesive X for direct restoration, a test was conducted in the same manner as in Example 40 except that the distance of light irradiation was changed to 1 mm (700 mW/cm²). The result is shown in Table 5.

The adhesives U and V used in the above Examples are each a dual cure type adhesive for direct restoration containing a present radical polymerization catalyst (chemical polymerization type) and a photopolymerization catalyst; the adhesive W used in Comparative Example 16 is a dual cure type adhesive for direct restoration containing a conventional chemical polymerization catalyst and a photopolymerization catalyst; and the adhesive X is a photopolymerization type adhesive containing only a photopolymerization catalyst. The light intensity of 50 mW/cm² employed in the present Examples and Comparative Examples was used in consideration of such adhesion as is conducted in e.g. the mouth with insufficient light irradiation.

As is clear from the comparison of Examples 40 and 41 with Comparative Examples 16 and 17, dual cure type adhesives containing a present radical polymerization catalyst could give, even under insufficient light irradiation, at least the same high adhesion strengths as when sufficient light irradiation was made (Reference Example 1). Thus, when the present adhesive is used in dental treatment, insufficient adhesion caused by insufficient light irradiation can be avoided.

Subsequently, adhesives for indirect restoration each containing a present radical polymerization catalyst were evaluated for performances.

### Example 42

There was prepared an adhesive CR-1 for indirect restoration (a CR type resin cement) consisting of a first paste and a second paste each having a composition shown in Table 6.

A pretreatment agent having the following composition was prepared in two solutions, i.e. a first solution and a second solution. The two solutions were mixed in equal masses right before use, and a treatment of tooth surface was conducted. Incidentally, ( ) indicates pbm.

### First solution:

PM (15)
MAC-10 (5)
bis-GMA (5)
Acetone (10)
Isopropyl alcohol (6)

### Second solution:

Water (38)
Acetone (19)
Sodium p-toluenesulfinate (2)

Successively, the first paste and second paste constituting the CR-1 were mixed in equal masses right before use, and an adhesion strength was measured in accordance with the measurement method 2 for adhesion strength. The result is shown in Table 7.

**Table 6**

| | Composition of first paste | | | | | | | | Composition of second paste | | | | | | | |
|---|---|---|---|---|---|---|---|---|---|---|---|---|---|---|---|---|
| | Aryl borate compound | | Inorganic filler(s) | | Non-acidic monomers | | Other component | | Vanadium compound | | Inorganic fillers | | Acidic monomers | | Non-acidic monomers | |
| CR-1 | PhBNa | 2 | 3Si-Zr | 50 | BisGMA | 12 | - | | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-2 | PhBTEOA | 2 | 3Si-Zr | 50 | BisGMA | 12 | - | | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-3 | FPhBNa | 2 | 3Si-Zr | 50 | BisGMA | 12 | - | | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-4 | PhBTEOA | 2 | 3Si-Zr | 50 | D2.6E | 15 | - | | VOAA | 0.05 | 3Si-Zr | 50 | PM | 10 | D2.6E | 12 |
| | | | 0.3Si-Ti | 50 | NPG | 15 | | | | | 0.3Si-Ti | 50 | MAC-10 | 2 | 3G | 6 |
| CR-5 | PhBTEOA | 2 | 3Si-Zr | 50 | BisGMA | 12 | - | | VOAA | 0.05 | 3Si-Zr | 50 | PM | 10 | D2.6E | 12 |
| | | | 0.3Si-Ti | 50 | 3G | 17 | | | | | 0.3Si-Ti | 50 | MAC-10 | 2 | 3G | 6 |
| | | | | | MTU-6 | 1 | | | | | | | | | | |
| CR-6 | PhBTEOA | 2 | 3Si-Zr | 50 | BisGMA | 12 | - | | OPBV | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-7 | PhBTEOA | 2 | 3Si-Zr | 50 | BisGMA | 12 | - | | BMOV | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-8 | PhBTEOA | 2 | 3Si-Zr | 50 | BisGMA | 12 | - | | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | NPG | 6 |
| CR-9 | PhBTEOA | 2 | 3Si-Zr | 50 | BisGMA | 12 | BAPO | 0.5 | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-10 | PhBTEOA | 2 | 3Si-Zr | 50 | BisGMA | 12 | BDTPO | 0.5 | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-11 | PhBTEOA | 2 | 3Si-Zr | 50 | BisGMA | 12 | Perocta H | 0.5 | VOAA | 0.05 | 3Si-Zr | 50 | PM | 10 | D2.6E | 12 |
| | | | 0.3Si-Ti | 50 | 3G | 18 | | | | | 0.3Si-Ti | 50 | MAC-10 | 2 | 3G | 6 |
| CR-12 | - | | 3Si-Zr | 50 | BisGMA | 12 | DMPT | 2 | (Organic peroxide) | | 3Si-Zr | 50 | PM | 10 | D2.6E | 12 |
| | | | 0.3Si-Ti | 50 | 3G | 18 | | | BPO | 1 | 0.3Si-Ti | 50 | MAC-10 | 2 | 3G | 6 |
| CR-13 | PhBTEOA | 2 | 3Si-Zr | 50 | BisGMA | 12 | DMPT | 2 | (Organic peroxide) | | 3Si-Zr | 50 | PM | 10 | D2.6E | 12 |
| | | | 0.3Si-Ti | 50 | 3G | 18 | | | BPO | 1 | 0.3Si-Ti | 50 | MAC-10 | 2 | 3G | 6 |
| GI-1 | PhBTEOA | 2 | FASG | 100 | D2.6E | 15 | - | | VOAA | 0.05 | 3Si-Zr | 50 | PM | 10 | D2.6E | 12 |
| | | | | | NPG | 15 | | | | | 0.3Si-Ti | 50 | MAC-10 | 2 | 3G | 6 |
| GI-2 | PhBTEOA | 2 | FASG | 100 | D2.6E | 15 | - | | OPBV | 0.05 | 3Si-Zr | 50 | PM | 10 | D2.6E | 12 |
| | | | | | NPG | 15 | | | | | 0.3Si-Ti | 50 | MAC-10 | 2 | 3G | 6 |
| GI-3 | - | | FASG | 100 | D2.6E | 15 | DMPT | 2 | (Organic peroxide) | | 3Si-Zr | 50 | PM | 10 | D2.6E | 12 |
| | | | | | NPG | 15 | | | BPO | 1 | 0.3Si-Ti | 50 | MAC-10 | 2 | 3G | 6 |

**Table 7**

| | Indirect restoractive adhesive used | Adhesion strength /MPa (S.D.) | |
|---|---|---|---|
| | | Enamel | Dentin |
| Example 42 | CR-1 | 19.8 (3.0) | 18.9 (2.3) |
| Example 43 | CR-2 | 20.3 (1.9) | 20.2 (2.6) |
| Example 44 | CR-3 | 21.0 (2.4) | 20.3 (2.2) |
| Example 45 | CR-4 | 22.0 (3.3) | 21.0 (2.9) |
| Example 46 | CR-5 | 20.9 (3.6) | 20.0 (2.3) |
| Example 47 | CR-6 | 22.3 (3.6) | 21.2 (3.0) |
| Example 48 | CR-7 | 22.2 (1.9) | 21.6 (3.6) |
| Example 49 | CR-8 | 21.9 (3.2) | 20.5 (2.7) |
| Example 50 | CR-9 | 20.9 (3.4) | 20.1 (4.1) |
| Example 51 | CR-10 | 21.5(3.1) | 20.3 (3.7) |
| Example 52 | CR-11 | 22.6 (1.5) | 21.5 (1.9) |
| Comparative Example 18 | CR-12 | 12.0 (1.8) | 5.9 (2.1) |

### Examples 43-52 and Comparative Example 18

There were prepared adhesives CR-2 to CR-12 for indirect restoration each consisting of a first paste and a second paste each having a composition shown in Table 6. Adhesion strengths were measured in the same manner as in Example 42 except that the above adhesives were used. The results are shown in Table 7.

### Example 53 and Comparative Example 19

The adhesives CR-2 and CR-12 were measured for the bending strength and discoloration resistance of cured material. The results are shown in Table 8.

**Table 8**

| | Used adhesive for indirect restoration | Bending strength MPa/cm² | Initial color | Discoloration resistance |
|---|---|---|---|---|
| Example 53 | CR-2 | 95 | 1 | 1 |
| Comparative Example 19 | CR-12 | 80 | 1 | 4 |

CR-1 to CR-11 are present adhesives for indirect restoration (CR type resin cements) and contain metal oxide inorganic particles as the inorganic filler. In these adhesives, importance is attached to mechanical properties after curing.

As is clear from the comparison of Examples 42-52 with Comparative Example 18, adhesives for indirect restoration each containing a present radical polymerization catalyst, as compared with an adhesive for indirect restoration containing a known BPO/amine type polymerization catalyst, can give very high adhesion strengths. Further, as is seen from Example 53 and Comparative Example 19, present adhesives for indirect restoration have high mechanical strengths after curing and are also superior in discoloration resistance. It is appreciated from these results that present adhesives for indirect restoration are more suitable for long-term use in the mouth.

### Example 54

There was prepared an adhesive GI-1 for indirect restoration consisting of a first past and a second paste each having a composition shown in Table 6.

Using GI-1, the adhesion strength was measured in the same manner as in Example 42 except that there was no pretreatment of tooth using a pretreatment agent. The result is shown in Table 9.

**Table 9**

| | Used adhesive for indirect restoration | Adhesion strength /MPa (S.D.) | |
|---|---|---|---|
| | | Enamel | Dentin |
| Example 54 | GI-1 | 16.3 (2.8) | 14.9 (2.2) |
| Example 55 | GI-2 | 16.5 (2.6) | 15.3 (2.6) |
| Comparative Example 20 | GI-3 | 6.1 (2.2) | 4.0 (2.3) |

### Example 55 and Comparative Example 20

Adhesion strengths were measured in the same manner as in Example 54 except that there were used, adhesives for indirect restoration, GI-2 and GI-3 each having a composition shown in Table 6. The results are shown in Table 9.

### Example 56

0.005 pbm of VOAA was dissolved, by mixing, in 20 pbm of PM, 50 pbm of HEMA, 12 pbm of BisGMA and 18 pbm of 3G, to obtain a homogeneous solution (a liquid component). 3 pbm of PhBNa were homogeneously mixed into 100 pbm of FASG to obtain a powder component. The liquid component and the powder component were mixed at a mass ratio of 1:2.3 right before use to prepare an adhesive GI-4 for indirect restoration. Using this adhesive, the adhesion strength was measured in the same manner as in Example 54. The result is shown in Table 11.

**Table 10**

| | Composition of powder components | | | | Composition of liquid components | | | | | |
|---|---|---|---|---|---|---|---|---|---|---|
| | Aryl borate compound | | Polyvalent metal ion-releasing filler | | Acidic monomer(s) | | Non-acidic monomers | | Vanadium compound | |
| GI-4 | PhBNa | 3 | FASG | 100 | PM | 20 | HEMA | 50 | VOAA | 0.005 |
| | | | | | | | BisGMA | 12 | | |
| | | | | | | | 3G | 18 | | |
| GI-5 | PhBTEOA | 3 | FASG | 100 | PM | 20 | HEMA | 50 | VOAA | 0.005 |
| | | | | | | | BisGMA | 12 | | |
| | | | | | | | 3G | 18 | | |
| GI-6 | PhBTEOA | 3 | FASG | 100 | PM | 20 | HEMA | 50 | VOAA | 0.005 |
| | | | | | | | BisGMA | 12 | | |
| | | | | | | | 3G | 18 | | |
| GI-7 | PhBTEOA | 3 | FASG | 100 | PM | 20 | HEMA | 45 | VOAA | 0.005 |
| | | | | | MAC-10 | 5 | BisGMA | 12 | | |
| | | | | | | | 3G | 18 | | |
| GI-8 | PhBTEOA | 3 | FASG | 100 | PM | 20 | HEMA | 50 | OPBV | 0.005 |
| | | | | | | | BisGMA | 12 | | |
| | | | | | | | 3G | 18 | | |
| GI-9 | (Organic peroxide) | | FASG | 100 | PM | 20 | HEMA | 45 | (Amine compound) | |
| | BPO | 1 | | | MAC-10 | 5 | BisGMA | 12 | DMPT | 3 |
| | | | | | | | 3G | 18 | | |

**Table 11**

| | Indirect restorative adhesive used | Adhesion strength /MPa (S.D.) | |
|---|---|---|---|
| | | Enamel | Dentin |
| Example 56 | GI-4 | 15.3 (2.3) | 14.3 (2.4) |
| Example 57 | GI-5 | 15.6 (3.6) | 14.0 (3.6) |
| Example 58 | GI-6 | 15.7(1.7) | 14.1 (2.1) |
| Example 59 | GI-7 | 15.5 (3.1) | 15.3 (2.6) |
| Example 60 | GI-8 | 15.2 (3.2) | 14.8 (2.7) |
| Comparative Example 21 | GI-9 | 6.0 (2.3) | 4.5 (1.4) |

### Examples 57-60 and Comparative Example 21

There were prepared adhesives GI-5 to GI-9 for indirect restoration each consisting of a powder component and a liquid component both shown in Table 10. Using these adhesives, adhesion strengths were measured in the same manner as in Example 56. The results are shown in Table 11.

GI-1 to GI-9 are each an adhesive for indirect restoration containing, as the filler, a polyvalent metal ion-releasing filler (a resin-reinforced type glass ionomer cement for luting).

As is clear from Examples 54-60, GI-1 to GI-2 and GI-4 to GI-8 each containing a present radical polymerization catalyst showed high adhesion strengths to both the enamel and the dentin. GI-3 and GI-9 each containing a BPO/amine type polymerization catalyst showed no sufficient adhesion strength to the enamel or the dentin.

### Example 61

There were prepared a powder component consisting of 3 pbm of PhBNa, 5 pbm of PEMA and 95 pbm of P (MMA-EMA), and a liquid component which was a homogeneous solution consisting of 5 pbm of PM, 5 pbm of MAC-10, 65 pbm of MMA, 20 pbm of HEMA, 3 pbm of BisGMA, 2 pbm of 3G and 0.005 pbm of VOAA. Using the same pretreatment agent as used in Example 42, a tooth surface was pretreated under the same conditions. Then, to the pretreated tooth surface was applied an adhesive MMA-1 for indirect restoration prepared by kneading the above liquid component and the above powder component at a mass ratio of 1:1.4, and the adhesion strength was measured according to the measurement method 2 for adhesion strength. The result is shown in Table 13.

**Table 12**

| | Composition of Powder components | | | | | | Composition of liquid components | | | | | |
|---|---|---|---|---|---|---|---|---|---|---|---|---|
| | Aryl borate compound | | Organic fillers | | Other component | | Acidic monomers | | Non-acidic monomers | | Vanadium compound | |
| MMA-1 | PhBNa | 3 | PEMA | 5 | | | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-2 | PhBTEOA | 3 | PEMA | 5 | | | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-3 | FPhBNa | 3 | PEMA | 5 | | | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-4 | PhBDMBE | 3 | PEMA | 5 | | | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-5 | PhBNa | 3 | PMMA | 5 | | | PM | 5 | MMA | 65 | VOAA | 0.005 |
| | | | PEMA | 5 | | | MAC-10 | 5 | HEMA | 20 | | |
| | | | P(MMA-EMA) | 90 | | | | | BisGMA | 3 | | |
| | | | | | | | | | 3G | 2 | | |
| MMA-6 | PhBTEOA | 3 | PMMA | 5 | | | PM | 5 | MMA | 65 | VOAA | 0.005 |
| | | | PEMA | 5 | | | MAC-10 | 5 | HEMA | 20 | | |
| | | | P(MMA-EMA) | 90 | | | | | BisGMA | 3 | | |
| | | | | | | | | | 3G | 2 | | |
| MMA-7 | PhBTEOA | 3 | PEMA | 5 | | | PM | 5 | MMA | 65 | VOAA | 0.005 |
| | | | P(MMA-EMA) | 95 | | | 4-META | 5 | HEMA | 20 | | |
| | | | | | | | | | BisGMA | 3 | | |
| | | | | | | | | | 3G | 2 | | |

| | Composition of Powder component | | | | | | Composition of liquid components | | | | | |
|---|---|---|---|---|---|---|---|---|---|---|---|---|
| | Aryl borate compound | | Organic fillers | | Other component | | Acidic monomers | | Non-acidic monomers | | Vanadium compound | |
| MMA-8 | PhBTEOA | 3 | PEMA | 5 | | | PM | 10 | MMA | 65 | VOAA | 0.005 |
| | | | P(MMA-EMA) | 95 | | | MAC-10 | 5 | HEMA | 20 | | |
| MMA-9 | PhBTEOA | 3 | PEMA | 5 | | | PM | 10 | MMA | 65 | OPBV | 0.005 |
| | | | P(MMA-EMA) | 95 | | | MAC-10 | 5 | HEMA | 20 | | |
| MMA-10 | PhBTEOA | 3 | PEMA | 5 | | | PM | 10 | MMA | 65 | BMOV | 0.005 |
| | | | P(MMA-EMA) | 95 | | | MAC-10 | 5 | HEMA | 20 | | |
| MMA-11 | PhBTEOA | 3 | PEMA | 5 | BAPO | 2 | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-12 | PhBTEOA | 3 | PEMA | 5 | BDTPO | 2 | PM | 5 | MMA | 65 | VOAA | 0.005 |
| | | | P(MMA-EMA) | 95 | | | MAC-10 | 5 | HEMA | 20 | | |
| | | | | | | | | | BisGMA | 3 | | |
| | | | | | | | | | 3G | 2 | | |
| MMA-13 | PhBTEOA | 3 | PEMA | 5 | | | PM | 10 | MMA | 65 | BMOV | 0.005 |
| | | | P(MMA-EMA) | 95 | | | MAC-10 | 5 | HEMA | 19 | | |
| | | | | | | | | | MTU-6 | 1 | | |
| MMA-14 | - | | PEMA | 5 | BPO | 1 | PM | 10 | MMA | 65 | (Amine compound) | |
| | | | P(MMA-EMA) | 95 | | | MAC-10 | 5 | HEMA | 20 | DMPT | 3 |
| MMA-15 | - | | PEMA | 5 | BPO | 1 | MAC-10 | 5 | MMA | 65 | (Amine compound) | |
| | | | P(MMA-EMA) | 95 | | | | | HEMA | 20 | DMPT | 3 |
| | | | | | | | | | BisGMA | 6 | | |
| | | | | | | | | | 3G | 4 | | |

**Table13**

| | Indirect restorative adhesive used | Adhesion strength /MPa (S.D.) | |
|---|---|---|---|
| | | Enamel | Dentin |
| Example 61 | MMA-1 | 20.1 (3.8) | 18.1 (2.9) |
| Example 62 | MMA-2 | 21.0 (3.2) | 19.5 (3.4) |
| Example 63 | MMA-3 | 19.7 (2.7) | 18.0 (3.3) |
| Example 64 | MMA-4 | 19.8 (2.6) | 18.3 (2.6) |
| Example 65 | MMA-5 | 20.3 (4.0) | 19.7 3.6 |
| Example 66 | MMA-6 | 20.2 (2.9) | 20.2 (2.8) |
| Example 67 | MMA-7 | 21.2 (3.0) | 20.8 (4.1) |
| Example 68 | MMA-8 | 20.9 (2.8) | 21.0 (3.9) |
| Example 69 | MMA-9 | 22.3 (3.6) | 21.2 (3.2) |
| Example 70 | MMA-10 | 22.0 (2.6) | 21.3 (1.9) |
| Example 71 | MMA-11 | 21.9 (2.3) | 20.9 (3.2) |
| Example 72 | MMA-12 | 20.8 (3.3) | 19.3 (3.0) |
| Example 73 | MMA-13 | 20.3 (3.6) | 19.8 (4.3) |
| Comparative Example 22 | MMA-14 | 12.2 (1.8) | 6.0 (1.8) |

### Examples 62-73 and Comparative Example 22

Adhesion strengths were measured in the same manner as in Example 63 except that there were used, as adhesives for indirect restoration, MMA-2 to MMA-14 each consisting of a powder component and a liquid component each having a composition shown in Table 12. The results are shown in Table 13.

MMA-1 to MMA-14 are each an adhesive for indirect restoration containing a liquid component composed mainly of MMA (methyl methacrylate) and an organic filler (a MMA type resin cement).

As is clear from a comparison between Examples 61-73 and Comparative Example 22, adhesives for indirect restoration each containing a present radical polymerization catalyst showed far higher adhesion strengths than an adhesive for indirect restoration containing a conventional BPO/amine type radical polymerization catalyst.

There were prepared dental restorative materials each containing a present radical polymerization catalyst, and their properties were evaluated.

### Example 74

There were added, as fillers, 70 pbm of 0.5Si-Zr and 30 pbm of 0.06Si-Zr to a liquid obtained by homogeneously mixing 35 pbm of BisGMA, 23 pbm of 3G, 5 pbm of PM and 0.01 part by mass of VOAA. The mixture was kneaded homogeneously in a mortar to obtain a first paste. Separately, there were added, as fillers, 70 pbm of 0.5Si-Zr and 30 pbm of 0.06Si-Zr to a liquid obtained by homogeneously mixing 40 pbm of BisGMA, 27 pbm of 3G and 3 pbm of PhBTEOA. The mixture was homogeneously kneaded in a mortar to obtain a second paste. A dental composite resin COM-1 consists of the first paste and the second paste.

The first paste and the second paste were kneaded at a mass ratio of 1:1, after which a curing time and the bending strength and Knoop hardness of the cured material obtained were measured and a discoloration resistance test for the cured material was conducted. The results are shown in Table 15.

**Table 14**

| | First paste | | | | | | | | Second paste | | | | | | | |
|---|---|---|---|---|---|---|---|---|---|---|---|---|---|---|---|---|
| | Fillers | | Non-acidic monomers | | Vanadium compound | | Acidic compound | | Fillers | | Non-acidic monomers | | Aryl borate compound | | Other additive | |
| COM-1 | 0.5Si-Zr | 70 | BisGMA | 35 | VOAA | 0.01 | PM | 5 | 0.5Si-Zr | 70 | BisGMA | 40 | PhBTEOA | 3 | - | |
| | 0.06Si-Zr | 30 | 3G | 23 | | | | | 0.06Si-Zr | 30 | 3G | 27 | | | | |
| COM-2 | 0.5Si-Zr | 70 | BisGMA | 35 | VOAA | 0.01 | PM | 5 | 0.5Si-Zr | 70 | BisGMA | 40 | FPhBNa | 3 | - | |
| | 0.06Si-Zr | 30 | 3G | 23 | | | | | 0.06Si-Zr | 30 | 3G | 27 | | | | |
| COM-3 | 0.5Si-Zr | 70 | BisGMA | 35 | BMOV | 0.01 | PM | 5 | 0.5Si-Zr | 70 | BisGMA | 40 | PhBTEOA | 3 | - | |
| | 0.06Si-Zr | 30 | 4G | 23 | | | | | 0.06Si-Zr | 30 | 4G | 27 | | | | |
| COM-4 | 0.5Si-Zr | 70 | BisGMA | 35 | OPBV | 0.01 | PM | 5 | 0.5Si-Zr | 70 | BisGMA | 40 | PhBTEOA | 3 | - | |
| | 0.06Si-Zr | 30 | 5G | 23 | | | | | 0.06Si-Zr | 30 | 5G | 27 | | | | |
| COM-5 | 0.5Si-Zr | 70 | BisGMA | 35 | VOAA | 0.01 | PM | 5 | 0.5Si-Zr | 70 | BisGMA | 40 | PhBTEOA | 3 | Perocta H | 2 |
| | 0.06Si-Zr | 30 | 6G | 23 | | | | | 0.06Si-Zr | 30 | 6G | 27 | | | | |
| COM-6 | 0.5Si-Zr | 70 | BisGMA | 35 | (Organic peroxide) | | - | | 0.5Si-Zr | 70 | BisGMA | 40 | (Amine compound) | | - | |
| | 0.06Si-Zr | 30 | 3G | 23 | BPO | 2 | | | 0.06Si-Zr | 30 | 3G | 27 | DEPT | 3 | | |

**Table 15**

| | Composite resin evaluated | Curing time | Bending strength | Knoop hardness | Score of discoloration resistance |
|---|---|---|---|---|---|
| | | sec | MPa/cm² | Hk | |
| Example 74 | COM-1 | 3'00" | 118 | 36 | 1 |
| Example 75 | COM-2 | 2'50" | 120 | 36 | 1 |
| Example 76 | COM-3 | 3'10" | 122 | 37 | 1 |
| Example 77 | COM-4 | 3'10" | 120 | 35 | 1 |
| Example 78 | COM-5 | 2'40" | 126 | 38 | 1 |
| Comparative Example 23 | COM-6 | 2'40" | 100 | 35 | 4 |

### Examples 75-78 and Comparative Example 23

In the same manner as in Example 74, there were prepared dental composite resins COM-2 to COM-6 each having a composition shown in Table 14. The properties of these resins were evaluated. The results are shown in Table 15.

As is clear from Tables 14 and 15, dental composite resins each containing a present radical polymerization catalyst, as compared with a composite resin containing a BPO/amine type radical polymerization catalyst, showed high bending strengths and were low in discoloration and accordingly were particularly excellent composite resins.

### Example 79

There was prepared a resin-reinforced type glass ionomer cement for filling, consisting of a powder component obtained by homogeneously mixing 100 pbm of FASG and 3 pbm of PhBTEOA and a liquid component obtained by dissolving 0.005 pbm of VOAA in a solution consisting of 20 pbm of PM, 30 pbm of HEMA, 22 pbm of BisGMA and 28 pbm of 3G. The powder component and the liquid component were mixed at a 4:1 mass ratio to prepare a cured material. The cured material was evaluated for bending strength and discoloration resistance. As a result, the bending strength was 65 MPa and the discoloration score after discoloration test was 1, and the resin-reinforced type glass ionomer cement for filling had excellent properties.

### Comparative Example 24

A resin-reinforced type glass ionomer cement for filling was prepared in the same manner as in Example 79 except that 3 pbm of PhBTEOA in the powder component of Example 79 were changed to 1 part by mass of BPO and 0.005 pbm of VOAA in the liquid component of Example 79 was changed to 3 pbm of DMPT. The properties thereof were evaluated. The bending strength was 51 MPa and the discoloration score was 4, both of which were insufficient.

### Example 80

There was prepared an self-curing type resin consisting of a powder component containing 10 pbm of PEMA, 90 pbm of P(MMA-EMA) and 3 pbm of PhBTEOA and a liquid component obtained by dissolving 0.005 pbm of VOAA in a solution consisting of 85 pbm of MMA, 10 pbm of TMPT and 5 pbm of PM. The powder component and the liquid component were mixed at a 2:1 mass ratio. The resulting cured material was measured for bending strength, Knoop hardness, discoloration resistance and the amount of residual monomers. As a result, the bending strength was 84 MPa, the Knoop hardness was 14.9 kg/mm², the discoloration resistance score was 1, and the amount of residual monomers was 1.1%.

### Example 81

A cured material was obtained in the same manner as in Example 80 except that there was used, as the liquid components, a solution consisting of 50 pbm of 1,9-nonanediol methacrylate, 45 pbm of 2-(methacryloxy)ethyl acetoacetate, 5 pbm of PM and 0.005 pbm of VOAA. The cured material was evaluated. As a result, the bending strength was 75 MPa, the Knoop hardness was 13.8 kg/mm², the discoloration resistance score was 1, and the amount of residual monomers was 1.4%.

### Comparative Example 25

A cured material was obtained in the same manner as in Example 80 except that the PhBTEOA in the powder component of Example 80 was changed to 1 pbm of BPO and the VOAA in the liquid component of Example 80 was changed to 3 pbm of DMPT. The cured material was evaluated. As a result, the bending strength was 71 MPa, the Knoop hardness was 14.4 kg/mm², the discoloration resistance score was 4, and the amount of residual monomers was 3.7%.

Example 80 is a dental self-curing type resin of general use, and Example 81 is a composition suitably usable as a relining material for denture base resin. As is appreciated from the results of Examples 80-81 and Comparative Example 25, compositions each containing a present radical polymerization catalyst cure at ambient temperature and become a resin superior in discoloration resistance and small in amounts of residual monomers.

Next, evaluation was made on the properties of dental pretreatment agents each containing a present radical polymerization catalyst.

### Example 82

There was prepared an adhesive MMA-15 for indirect restoration consisting of a powder component obtained by mixing 1 pbm of BPO into 5 pbm of PMMA and 95 pbm of P(MMA-EMA) and a liquid component obtained by dissolving 3 pbm of DMPT in a solution consisting of 5 pbm of MAC-10, 65 pbm of MMA, 20 pbm of HEMA, 6 pbm of BisGMA and 4 pbm of 3G. MMA-15 is a dental adhesive containing no present radical polymerization catalyst.

There was prepared a dental pretreatment agent consisting of a first solution obtained by dissolving 0.1 pbm of VOAA in 20 pbm of PM and a second solution obtained by dissolving 2 pbm of PhBNa in 77.9 pbm of water. The first solution and the second solution were mixed at a mass ratio of 20.1:79.9. Using the mixture, an adhesion strength was measured in accordance with the measurement method 2 for adhesion strength. In the adhesive MMA-15 used, the powder component and the liquid component were mixed at a mass ratio of 1:1.4.

The adhesion strength to enamel was 17.3 MPa and the adhesion strength to dentin was 16.8 MPa.

### Examples 83-91 and Comparative Examples 26-28

There were prepared dental pretreatment agents each consisting of a first solution and a second solution, shown in Table 16. Each pretreatment agent was evaluated in the same manner as in Example 82 except that the first solution and the second solution were mixed at a mass ratio of 40:60. The results are shown in Table 16.

**Table 16**

| | Composition of pretreatment agent (pbm) | | | | | | Adhesive | Adhesion strength /MPa(S.D.) | |
|---|---|---|---|---|---|---|---|---|---|
| | First solution | | | Second solution | | | | | |
| | Acidic monomer(s) | Vanadium compound | Other component(s) | Aryl borate compound | Water | Other component | | Enamel | Dentin |
| Example 82 | PM (20) | VOAA (0.1) | - | PhBNa (2) | 77.9 | - | MMA-15 | 17.3(2.3) | 16.8(3.3) |
| Example 83 | PM (20) | VOAA (0.1) | IPA (19.9) | PhBTEOA (2) | 40 | Acetone (18) | MMA-15 | 18.8 (3.9) | 18.0 (2.9) |
| Example 84 | PM (20) | VOAA (0.1) | IPA (14.9) | PhBTEOA (2) | 40 | Acetone (18) | MMA-15 | 19.0 (3.6) | 19.8 (4.1) |
| | MAC-10 (5) | | | | | | | | |
| Example 85 | MMPS (5) | VOAA (0.1) | IPA (24.9) | PhBTEOA (2) | 40 | Acetone (18) | MMA-15 | 20.8 (2.9) | 19.1 (3.5) |
| Example 86 | PM (20) | VOSO4 (0.1) | IPA (19.9) | PhBTEOA (2) | 40 | Acetone (18) | MMA-15 | 20.5 (3.4) | 20.3 (2.7) |
| Example 87 | PM (20) | VOC2H4 (0.1) | IPA (19.9) | PhBTEOA (2) | 40 | Acetone (18) | MMA-15 | 20.1 (2.5) | 20.1 (3.4) |
| Example 88 | PM (20) | BMOV (0.1) | IPA (19.9) | PhBTEOA (2) | 40 | Acetone (18) | MMA-15 | 21.5 (3.7) | 20.9 (2.8) |
| Example 89 | PM (20) | VOAA (0.1) | UDMA (5) | PhBTEOA (2) | 40 | Acetone (18) | MMA-15 | 21.9 (2.5) | 21.3 (3.8) |
| | | | IPA (14.9) | | | | | | |
| Example 90 | PM (20) | VOAA (0.1) | IPA (19.9) | FphBNa (3) | 40 | Acetone (17) | MMA-15 | 20.3 (2.2) | 19.7 (2.0) |
| Example 91 | PM (20) | VOAA (0.1) | IPA (19.9) | PhBTEOA (2) | 40 | Acetone (18) | MMA-15 | 20.0 (3.8) | 19.5(2.1) |
| Example 92 | PM (20) | VOAA (0.1) | IPA (19.9) | PhBTEOA (2) | 40 | Acetone (18) | MACBOND 2 | 22.3 (3.6) | 20.3 (4.0) |
| Example 93 | PM (20) | VOAA (0.1) | IPA (19.9) | PhBTEOA (2) | 40 | Acetone (18) | Bistite 2 | 21.6 (2.9) | 19.0 (3.1) |
| Comparative | - | VOAA (0.1) | UDMA (5) | PhBTEOA (2) | 40 | Acetone (18) | MMA-15 | 0 | 0 |
| Example 26 | | | IPA (14.9) | | | | | | |
| Comparative | PM (20) | - | UDMA (5) | PhBTEOA (2) | 40 | Acetone (18) | MMA-15 | 1.9 (0.8) | 2.0(2.1) |
| Example 27 | | | IPA (15) | | | | | | |
| Comparative | PM (20) | VOAA (0.1) | UDMA (5) | - | 40 | Acetone (20) | MMA-15 | 8.9 (2.3) | 2.3 (1.6) |
| Example 28 | | | IPA (14.9) | | | | | | |

### Example 92

An adhesion strength was measured according to the measurement method 1 for adhesion strength, using Mac-Bond 2 [a commercial adhesive for direct restoration (a bonding agent) produced by Tokuyama K.K.] and the pretreatment agent used in Example 83. The result is shown in Table 16. Mac-Bond 2 is a photo-curing type adhesive containing MAC-10 as an acidic monomer and camphorquinone as a photopolymerization catalyst, but contains neither aryl borate compound nor vanadium compound. The adhesive was cured by 20-seconds light irradiation from a distance of 1 mm using Power Light.

### Example 93

An adhesion strength was measured according to the measurement method 2 for adhesion strength, using cement pastes A and B [a commercial adhesive for indirect restoration (Bistite 2) produced by Tokuyama K.K.] and the pretreatment agent used in Example 83. The result is shown in Table 16. The cement pastes (Bistite 2) are a chemical curing type adhesive containing MAC-10 as an acidic monomer and a BPO/amine type polymerization catalyst, but contain neither aryl borate compound nor vanadium compound. The cement was prepared by pushing out the pastes A and B in equal amounts from respective syringes according to the use instruction of the pastes, and mixing them.

As is appreciated from the results of Examples 82-93, a high adhesion strength is obtained to both the enamel and the dentin by using a dental pretreatment agent containing a present radical polymerization catalyst, even when the adhesive used (for direct restoration or indirect restoration) contains no present radical polymerization catalyst.

Evaluation was further made on first dental adhesive kits of the present invention.

### Example 94

There was prepared a dental pretreatment agent consisting of a first solution obtained by dissolving 0.1 pbm of VOAA in 20 pbm of PM and a second solution containing only water. The first solution and the second solution were mixed at a mass ratio of 20.1:79.9. Using the mixture, an adhesion strength was measured according to the adhesion strength measurement method for adhesive for indirect restoration. The adhesive used was CR-13 mentioned above.

As a result, the adhesion strength to enamel was 24.3 MPa and the adhesion strength to dentin was 22.5 MPa.

### Examples 95-97 and Comparative Example 29

There were prepared dental pretreatment agents each consisting of a first solution and a second solution and having a composition shown in Table 17. Adhesion strengths were evaluated in the same manner as in Example 94 except that the first solution and the second solution were mixed at a mass ratio of 40:60. The results are shown in Table 17.

In each of Examples 94 and 95, the pretreatment agent contains a vanadium compound and the adhesive contains no vanadium compound; however, the adhesive kit showed a good adhesion strength.

In Example 96 wherein both the pretreatment agent and the adhesive contain a vanadium compound and also in Example 97 wherein both the pretreatment agent and the adhesive contain an aryl borate compound, high adhesion strengths were obtained. In the adhesive kit of Comparative Example 29 wherein no vanadium compound was compounded in any of the pretreatment agent and the adhesive, the adhesion strength was low.

**Table 17**

| | Composition of pretreatment agent (pbm) | | | | | | Adhesive | Adhesion strength /MPa(S.D.) | |
|---|---|---|---|---|---|---|---|---|---|
| | First solution | | | Second solution | | | | | |
| | Acidic monomer(s) | Vanadium compound | Other component | Aryl borate compound | Water | Other components | | Enamel | Dentin |
| Example 94 | PM (20) | VOAA (0.1) | - | - | 79.9 | - | CR-13 | 24.3(2.3) | 22.5(3.2) |
| Example 95 | PM (20) | VOAA (0.1) | IPA (14.9) | - | 40 | Acetone (15) | CR-13 | 24.8 (2.9) | 23.5 (3.6) |
| | MAC-10 (5) | | | | | UDMA (5) | | | |
| Example 96 | PM (20) | VOAA (0.1) | IPA (14.9) | - | 40 | Acetone (15) | CR-6 | 25.5 (2.5) | 24.9 (3.2) |
| | MAC-10 (5) | | | | | UDMA (5) | | | |
| Example 97 | PM (20) | VOAA (0.1) | IPA(14.9) | PhBTEOA (2) | 40 | Acetone (13) | CR-6 | 25.0 (3.2) | 24.8 (2.0) |
| | MAC-10 (5) | | | | | UDMA (5) | | | |
| Comparative Example 29 | PM (20) | - | IPA (15) | - | 40 | Acetone (15) | CR-13 | 14.5(2.9) | 9.1(1.9) |
| | MAC-10 (5) | | | | | UDMA (5) | | | |

Successively, evaluation was made on second dental adhesive kits of the present invention.

### Example 98

In a solution consisting of 2.0 g of PM, 3.0 g of water, 1.0 g of IPA, 3.5 g of acetone and 0.2 g of D2.6E were dissolved 0.02 g of a vanadium compound (BMOV), 0.03 g of a polymerization inhibitor (BHT) and 0.3 g of an amine compound (DMEM), to obtain a homogeneous solution. This solution was named as pretreatment agent P1. The pretreatment agent P1 had a pH of 2.0.

Separately, to a monomer solution consisting of 1.6 g of HEMA, 4.6 g of D2.6E and 2.4 g of 3G were added, under light shield, 0.3 g of PhBTEOA (an aryl borate compound), 0.4 g of Perocta H (an organic peroxide), 0.1 g of BDTPO (a photopolymerization catalyst), 0.01 g of BHT (a polymerization inhibitor) and 0.005 g of HQME (a polymerization inhibitor), to obtain a homogeneous solution. To this solution was added 0.6 g of MT-10, after which mixing was made until a homogeneous solution is obtained. This solution was named as photo-curing type adhesive L1.

Using P1 (a pretreatment agent) and L1 (an adhesive), an adhesion strength was measured according to the measurement method 3 for adhesion strength. The composite resin used was a photo-curing type.

As a result, the adhesion strength to enamel was 21.2 (2.3) MPa and the adhesion strength to dentin was 18.9 (2.3) MPa [( ) indicates a standard deviation. The same applies hereinafter.].

### Examples 99-120 and Comparative Examples 30-40

There were prepared pretreatment agents having compositions shown in Table 18 and adhesives having compositions shown in Table 19 or 20, in the same manners as in Example 98. Adhesion strengths were measured. The kinds of the composite resins used and the adhesion strengths obtained are shown in Table 21. In Tables 18-20, each value shown after each component used indicates the amount of the component expressed in mass parts. The same applies also in Tables 22, 24 and 25 shown later.

**Table 18**

| | Composition of pretreatment agent solution | | | | | | | | | | | | | | pH |
|---|---|---|---|---|---|---|---|---|---|---|---|---|---|---|---|
| | Acidic monomer(s) | | Vanadium compound | | Water | | Amine compound | | Water-soluble organic solvent | | Non-acidic monomer | | Other component | | |
| P1 | PM | 20 | BMOV | 0.2 | Water | 30 | DMEM | 3 | IPA | 10 | D2.6E | 2 | | | 2.0 |
| | | | | | | | | | Acetone | 35 | | | | | |
| P2 | PM | 20 | BMOV | 0.05 | Water | 33 | | | IPA | 10 | D2.6E | 2 | | | 1.4 |
| | | | | | | | | | Acetone | 35 | | | | | |
| P3 | PM | 10 | BMOV | 0.2 | Water | 43 | | | IPA | 10 | | | MDPB | 2 | 1.4 |
| | | | | | | | | | Acetone | 35 | | | | | |
| P4 | PM | 20 | VOAA | 0.2 | Water | 20 | DMEM | 3 | IPA | 10 | Bis-GMA | 2 | | | 1.8 |
| | | | | | | | | | Acetone | 45 | | | | | |
| P5 | PM | 20 | OPBV | 0.2 | Water | 30 | DMEM | 3 | IPA | 10 | TMPT | 2 | | | 2.0 |
| | 4-META | 5 | | | | | | | Acetone | 30 | | | | | |
| P6 | PM | 20 | BMOV | 0.4 | Water | 25 | DMEM | 3 | IPA | 10 | MMA | 2 | | | 2.0 |
| | MAC-10 | 5 | | | | | | | Acetone | 35 | | | | | |
| P7 | PM | 30 | BMOV | 0.2 | Water | 20 | | | Acetone | 38 | | | MT10 | 2 | 1.4 |
| | | | | | | | | | IPA | 10 | | | | | |
| P8 | PM | 20 | BMOV | 0.2 | Water | 31 | DMEM | 3 | IPA | 10 | | | BDTPO | 1 | 2.0 |
| | | | | | | | | | Acetone | 35 | | | | | |
| P9 | PM | 20 | BMOV | 0.2 | Water | 30 | DMEM | 3 | IPA | 10 | | | Perocta H | 2 | 2.0 |
| | | | | | | | | | Acetone | 35 | | | | | |
| P10 | | | BMOV | 0.2 | Water | 50 | DMEM | 3 | IPA | 10 | D2.6E | 2 | | | 2.0 |
| | | | | | | | | | Acetone | 35 | | | | | |
| P11 | PM | 20 | | | Water | 30 | DMEM | 3 | IPA | 10 | D2.6E | 2 | | | 2.0 |
| | | | | | | | | | Acetone | 35 | | | | | |
| P12 | PM | 30 | BMOV | 0.2 | | | DMEM | 3 | IPA | 20 | D2.6E | 2 | | | 2.0 |
| | | | | | | | | | Acetone | 45 | | | | | |
| P13 | PM | 20 | (FeAA | 0.2) | Water | 30 | DMEM | 3 | IPA | 10 | D2.6E | 2 | | | 2.0 |
| | | | | | | | | | Acetone | 35 | | | | | |

| | | | | | | | | | | | | | | | |
|---|---|---|---|---|---|---|---|---|---|---|---|---|---|---|---|
| *In P13, an iron compound (FeAA) was used in place of a vanadium compound. *All the pretreatment agents contained 0.3 pbm of BHT as the polymerization inhibitor. | | | | | | | | | | | | | | | |

**Table19**

| | Composition of adhesive | | | | | | | | | |
|---|---|---|---|---|---|---|---|---|---|---|
| | Non-acidic monomers | | Aryl borate compound | | Organic peroxide | | Photo polymerization initiator(s) | | Other component(s) | |
| L1 | HEMA | 16 | PhBTEOA | 3 | Perocta H | 4 | BDTPO | 1 | MT10 | 6 |
| | D2.6E | 46 | | | | | | | | |
| | 3G | 24 | | | | | | | | |
| L2 | HEMA | 27 | PhBTEOA | 2 | Perocta H | 2 | BDTPO | 1 | MT10 | 6 |
| | D2.6E | 41 | | | | | | | | |
| | 3G | 17 | | | | | | | | |
| | MMA | 4 | | | | | DMEM | | | |
| L3 | HEMA | 20 | PhBTEOA | 3 | Percumyl H | 4 | BDTPO | 1 | | |
| | D2.6E | 32 | | | | | | | | |
| | TMPT | 10 | | | | | | | | |
| | 3G | 30 | | | | | | | | |
| L4 | HEMA | 20 | PhBTEOA | 3 | Perocta H | 4 | TCT | 1.7 | MT10 | 6 |
| | D2.6E | 35 | | | | | CM102 | 0.02 | | |
| | 3G | 30 | | | | | | | | |
| L5 | HEMA | 20 | PhBTEOA | 3 | Perocta H | 4 | TCT | 1.7 | MT10 | 6 |
| | Bis-GMA | 35 | | | | | HMC | 1 | | |
| | 3G | 30 | | | | | | | | |
| L6 | HEMA | 20 | PhBTEOA | 3 | Perocta H | 4 | BDTPO | 1 | FASG | 6 |
| | D2.6E | 31 | | | | | | | MDPB | 5 |
| | 3G | 30 | | | | | | | | |
| L7 | HEMA | 20 | PhBTEOA | 3 | Perocta H | 4 | BDTPO | 1 | MT10 | 6 |
| | D2.6E | 35 | | | | | CQ | 0.5 | | |
| | 3G | 30 | | | | | | | | |
| L8 | HEMA | 20 | PhBTEOA | 3 | Perocta H | 4 | BDTPO | 1 | MT10 | 6 |
| | D2.6E | 33 | | | | | TCT | 1.7 | | |
| | 3G | 30 | | | | | CDAC | 1 | | |

| | | | | | | | | | | |
|---|---|---|---|---|---|---|---|---|---|---|
| *All the adhesives contained, as the polymerization inhibitor, 0.1 pbm of BHT and 0.05 pbm of HQME. *In L4, L5 and L8, the aryl borate compound is also a component constituting the photopolymerization initiator. | | | | | | | | | | |

**Table 20**

| | Composition of adhesive | | | | | | | | | |
|---|---|---|---|---|---|---|---|---|---|---|
| | Non-acidic monomers | | Aryl borate compound | | Organic peroxide | | Photopolymerization initiator | | Other component(s) | |
| L9 | HEMA | 20 | PhBTEO A | 1 | Perocta H | 4 | BDTPO | 1 | MT 10 | 6 |
| | D2.6E | 38 | | | | | | | | |
| | 3G | 30 | | | | | | | | |
| L10 | HEMA | 20 | PhBTEO A | 3 | Perocta H | 4 | BAPO | 1 | MT 10 | 6 |
| | Bis-GMA | 36 | | | | | DMEM | | | |
| | 3G | 30 | | | | | | | | |
| L11 | HEMA | 20 | PhBDMP T | 3 | Perocta H | 4 | BDTPO | 1 | MT 10 | 6 |
| | D2.6E | 36 | | | | | | | | |
| | 3G | 30 | | | | | | | | |
| L12 | HEMA | 20 | PhBNa | 3 | Perocta H | 4 | BDTPO | 1 | MT 10 | 6 |
| | D2.6E | 36 | | | | | | | | |
| | 3G | 30 | | | | | | | | |
| L13 | HEMA | 20 | PhBTEO A | 3 | Perocta H | 4 | BDTPO | 1 | MT 10 | 6 |
| | D2.6E | 36 | | | | | | | BM OV | 0.005 |
| | 3G | 30 | | | | | | | | |
| L14 | HEMA | 20 | | | Perocta H | 4 | BDTPO | 1 | MT 10 | 6 |
| | D2.6E | 39 | | | | | | | | |
| | 3G | 30 | | | | | | | | |
| L15 | HEMA | 20 | PhBDMP T | 3 | | | BDTPO | 1 | MT 10 | 6 |
| | D2.6E | 40 | | | | | | | | |
| | 3G | 30 | | | | | | | | |
| L16 | HEMA | 20 | PhBDMP T | 3 | Perocta H | 4 | | | MT 10 | 6 |
| | D2.6E | 37 | | | | | | | | |
| | 3G | 30 | | | | | | | | |

| | | | | | | | | | | |
|---|---|---|---|---|---|---|---|---|---|---|
| *All the adhesives contained, as the polymerization inhibitor, 0.1 pbm of BHT and 0.05 pbm of HQME. | | | | | | | | | | |

**Table 21**

| | Pretreatment agent used | Adhesive used | Composite resin used | Adhesion strength /MPa(S.D.) | |
|---|---|---|---|---|---|
| | | | | Enamel | Dentin |
| Example 98 | P1 | L1 | Photo-curing type | 21.2(2.3) | 18.9 (2.3) |
| Example 99 | P2 | L1 | Photo-curing type | 19.0 (1.9) | 20.2 (2.6) |
| Example 100 | P3 | L1 | Photo-curing type | 18.7 (3.7) | 20.3 (2.2) |
| Example 101 | P4 | L1 | Photo-curing type | 19.2 (3.3) | 19.5 (3.4) |
| Example 102 | P5 | L1 | Photo-curing type | 20.5 (4.4) | 18.0 (3.3) |
| Example 103 | P6 | L1 | Photo-curing type | 21.4 (2.3) | 18.3 (2.6) |
| Example 104 | P7 | L1 | Photo-curing type | 20.1 (2.6) | 19.7(3.6) |
| Example 105 | P8 | L1 | Photo-curing type | 19.4 (2.7) | 20.2 (2.8) |
| Example 106 | P1 | L2 | Photo-curing type | 21.0(3.1) | 20.8(4.1) |
| Example 107 | P1 | L3 | Photo-curing type | 19.5 (1.8) | 21.0 (3.9) |
| Example 108 | P1 | L4 | Photo-curing type | 18.8 (4.5) | 21.2 (3.2) |
| Example 109 | P1 | L5 | Photo-curing type | 18.2 (3.8) | 20.3 (4.0) |
| Example 110 | P1 | L6 | Photo-curing type | 19.6 (2.2) | 20.2 (2.9) |
| Example 111 | P1 | L7 | Photo-curing type | 19.8 (2.9) | 21.2 (3.0) |
| Example 112 | P1 | L8 | Photo-curing type | 21.3 (1.8) | 20.9 (2.8) |
| Example 113 | P1 | L9 | Photo-curing type | 20.0 (2.6) | 22.3 (3.6) |
| Example 114 | P1 | L10 | Photo-curing type | 18.2 (2.7) | 21.0 (2.4) |
| Example 115 | P1 | L11 | Photo-curing type | 17.6 (3.1) | 22.0 (3.3) |
| Example 116 | P1 | L12 | Photo-curing type | 20.0 (4.4) | 20.9 (3.6) |
| Example 117 | P1 | L1 | Chemical curing type | 21.4 (2.7) | 22.3 (3.6) |
| Example 118 | P1 | L3 | Chemical curing type | 24.3 (4.7) | 22.2 (1.9) |
| Example 119 | P5 | L7 | Chemical curing type | 18.4 (2.0) | 21.9 (3.2) |
| Example 120 | P8 | L2 | Chemical curing type | 19.1 (1.9) | 19.4 (2.3) |
| Comparative Example 30 | P10 | L1 | Photo-curing type | 0 | 0 |
| Comparative Example 31 | P10 | L1 | Chemical curing type | 0 | 0 |
| Comparative Example 32 | P11 | L1 | Photo-curing type | 0 | 0 |
| Comparative Example 33 | P11 | L6 | Photo-curing type | 2.3(0.9) | 3.4(2.1) |
| Comparative Example 34 | P11 | L13 | Photo-curing type | 8.5(3.8) | 11.6(3.9) |
| Comparative Example 35 | P12 | L1 | Photo-curing type | 0 | 0 |
| Comparative Example 36 | P1 | L14 | Photo-curing type | 0 | 0 |
| Comparative Example 37 | P1 | L15 | Photo-curing type | 0 | 0 |
| Comparative Example 38 | P1 | L16 | Photo-curing type | 0 | 0 |
| Comparative Example 39 | P9 | L15 | Photo-curing type | 3.4(3.2) | 3.5(3.8) |
| Comparative Example 40 | P13 | L1 | Photo-curing type | 6.7(3.3) | 10.1 (5.6) |

Examples 98-116 are cases each using a photo-curing type composite resin, and Examples 117-120 are cases each using a chemical curing type composite resin. In any of these cases, high adhesion strengths to both enamel and dentin are obtained by using a second dental adhesive kit of the present invention.

Comparative Examples 30 and 31 are cases each containing no acidic monomer in the pretreatment agent, and Comparative Example 35 is a case containing no water in the pretreatment agent. In any of these cases, there was no adhesion to enamel or dentin. Comparative Examples 32-34 are cases each containing no vanadium compound in the pretreatment agent. In these cases, there was slight adhesion but the adhesion strengths were far lower than in the above Examples. In Comparative Example 34, there was no vanadium compound in the pretreatment agent, but the adhesive contained a vanadium compound. Even in this case, the adhesion strength was considerably low and it is appreciated that when no acidic monomer is used in an adhesive, no high adhesion strength is obtained unless a vanadium compound is used not only in the adhesive but also in a pretreatment agent.

Comparative Example 36 is a case containing no aryl borate compound in the adhesive; Comparative Example 37 is a case containing no organic peroxide in the adhesive; and Comparative Example 38 is a case containing no photopolymerization catalyst in the adhesive. In any case, there was no adhesion to dentin.

Comparative Example 39, similarly to Comparative Example 38, is a case containing no organic peroxide in the adhesive. In this Comparative Example, an organic peroxide is compounded in the pretreatment agent. Even in this case, the adhesion strength is very low and the importance of compounding an organic peroxide in the adhesive is appreciated.

Comparative Example 40 is a case an iron compound is compounded in place of a vanadium compound. The adhesion strength is fairly low in comparison with the above Examples, and the importance of vanadium compound is appreciated from this result.

### Example 121

0.001 g of OPBV, 0.03 g of BHT and 0.6 g of MT-10 were added to a monomer solution consisting of 1.5 g of PM, 1.5 g of MAC-10, 3.0 g of D2.6E and 3.4 g of 3G, to obtain a homogeneous solution. This solution was named as adhesive package D1.

This D1 was mixed with the above-mentioned photo-curing type adhesive L7 in equal volumes to prepare an adhesive. A test was conducted according to the measurement method 1 for adhesion strength, using P1 as a pretreatment agent and a chemical curing type composite resin.

As a result, the adhesion strength to enamel was 20.9 (4.4) MPa and the adhesion strength to dentin was 20.3 (2.8) MPa.

### Examples 122-126

There were prepared adhesive packages D2-D5 having compositions shown in Table 22, in the same manner as in Example 121. These D2-D5 were mixed with the above-mentioned photo-curing type adhesives L8 to L10 and L12, in equal volumes to obtain adhesives. Using these adhesives, adhesion strengths were measured in the same manner as in Example 121. The pretreatment agents used, the combinations of adhesives used, and the adhesion strengths obtained are shown in Table 23.

**Table 22**

| | Composition of second adhesive solution | | | | | |
|---|---|---|---|---|---|---|
| | Acidic monomers | | Vanadium compound | | Other components | |
| D1 | PM | 15 | OPBV | 0.01 | D2.6E | 30 |
| | MAC-10 | 15 | | | 3G | 34 |
| | | | | | MT10 | 6 |
| D2 | PM | 15 | VOAA | 0.01 | D2.6E | 30 |
| | MAC-10 | 10 | | | 3G | 34 |
| | MTU-6 | 5 | | | MT10 | 6 |
| D3 | PM | 10 | BMOV | 0.01 | D2.6E | 40 |
| | | | | | 3G | 34 |
| | | | | | 0.6Si-Zr | 6 |
| D4 | PM | 15 | BMOV | 0.01 | D2.6E | 30 |
| | MAC-10 | 15 | | | 3G | 34 |
| | | | | | FASG | 6 |
| D5 | PM | 15 | BMOV | 0.01 | D2.6E | 30 |
| | MAC-10 | 15 | | | 3G | 30 |
| | | | | | HEMA | 10 |

| | | | | | | |
|---|---|---|---|---|---|---|
| *All contained 0.3pbm of BHT as the polymerization inhibitor. | | | | | | |

**Table 23**

| | Pretreatment agent used | Combination of adhesives used | | | Adhesion strength /MPa(S.D.) | |
|---|---|---|---|---|---|---|
| | | | | | Enamel | Dentin |
| Example 121 | P1 | L7 | + | D1 | 20.9 (4.4) | 20.3 (2.8) |
| Example 122 | P1 | L8 | + | D2 | 20.1 (2.6) | 19.4 (4.1) |
| Example 123 | P1 | L9 | + | D3 | 22.4 (3.8) | 21.5 (3.9) |
| Example 124 | P1 | L10 | + | D4 | 19.3 (1.7) | 19.8 (3.4) |
| Example 125 | P1 | L12 | + | D5 | 17.4 (2.4) | 22.3 (2.9) |
| Example 126 | P5 | L7 | + | D1 | 22.6 (5.6) | 19.6 (2.8) |

As is understood from the above results, an adhesive very superior also as a chemical curing type can be obtained by adding, to a photo-curing type adhesive showing a high adhesion strength, a composition containing an acidic monomer and a vanadium compound (a case of using a pretreatment agent in the second adhesive kit of the present invention). Therefore, an adhesive kit having both of a function of photo-curing type and a function of chemical curing type can be constituted by a small number of packages.

### Examples 127-142 and Comparative Examples 41-46

Adhesive solutions having compositions shown in Table 24 and Table 25 were prepared in the same manner as in Example 121, and their adhesion strengths were measured. In some Examples, there was used the measurement method 3 for adhesion strength of irradiating a light after adhesive coating. The pretreatment agents used, the combinations of adhesives used, use or no use of light irradiation, the kinds of composite resins used, and the adhesion strengths obtained are shown in Table 26

**Table 24**

| | Composition | | | | | | | | | |
|---|---|---|---|---|---|---|---|---|---|---|
| | Non-acidic monomers | | Aryl borate compound | | Organic peroxide | | Photopolymerization initiator(s) | | Other component | |
| L17 | HEMA | 20 | PhBTEOA | 3 | Perocta H | 4 | BDTPO | 1 | MT10 | 6 |
| | D2.6E | 36 | | | | | | | | |
| | 3G | 30 | | | | | | | | |
| L18 | HEMA | 30 | PhBTEOA | 2 | Perocta H | 2 | BDTPO | 1 | MT10 | 6 |
| | D2.6E | 34 | | | | | | | | |
| | 3G | 25 | | | | | | | | |
| L19 | HEMA | 20 | FPhBNa | 3 | Perocta H | 4 | DMEM | 1 | | |
| | D2.6E | 42 | | | | | | | | |
| | 3G | 30 | | | | | | | | |
| L20 | HEMA | 20 | PhBTEOA | 3 | Percumyl H | 4 | TCT | 1.7 | MT10 | 6 |
| | D2.6E | 35 | | | | | CM102 | 0.02 | | |
| | 3G | 30 | | | | | | | | |
| L21 | HEMA | 20 | PhBDMBE | 3 | Perocta H | 4 | TCT | 1.7 | MT10 | 6 |
| | D2.6E | 35 | | | | | HMC | 1 | | |
| | 3G | 30 | | | | | | | | |
| L22 | HEMA | 20 | BFPhBNa | 3 | Perocta H | 4 | BDTPO | 1 | FASG | 6 |
| | D2.6E | 36 | | | | | | | | |
| | 3G | 30 | | | | | | | | |
| L23 | HEMA | 20 | | | Perocta H | 4 | BDTPO | 1 | MT10 | 6 |
| | D2.6E | 39 | | | | | | | | |
| | 3G | 30 | | | | | | | | |

| | | | | | | | | | | |
|---|---|---|---|---|---|---|---|---|---|---|
| *All the adhesives contained, as the polymerization inhibitor, 0.1 pbm of BHT and 0.05 pbm of HOME. *In L20 and L21, the aryl borate compound is also a component constituting the photopolymerization initiator. | | | | | | | | | | |

**Table 25**

| | Composition | | | | | |
|---|---|---|---|---|---|---|
| | Acidic monomer(s) | | Vanadium compound | | Other components | |
| D6 | PM | 15 | BMOV | 0.01 | D2.6E | 30 |
| | MAC-10 | 15 | | | 3G | 34 |
| | | | | | MT10 | 6 |
| D7 | PM | 20 | BMOV | 0.01 | D2.6E | 35 |
| | | | | | 3G | 39 |
| | | | | | MT10 | 6 |
| D8 | PM | 15 | BMOV | 0.01 | D2.6E | 30 |
| | MAC-10 | 15 | | | 3G | 40 |
| D9 | PM | 15 | BMOV | 0.01 | Bis-GMA | 30 |
| | MAC-10 | 15 | | | 3G | 40 |
| D10 | PM | 15 | BMOV | 0.01 | UDMA | 29 |
| | MAC-10 | 15 | | | 3G | 40 |
| | | | | | PEMA | 1 |
| D11 | PM | 15 | BMOV | 0.01 | Bis-GMA | 30 |
| | 4-META | 15 | | | 3G | 34 |
| | | | | | MT10 | 6 |

| | | | | | | |
|---|---|---|---|---|---|---|
| *All contained 0.3 pbm of BHT as the polymerization inhibitor. | | | | | | |

**Table 26**

| | Pretreatment agent used | Combination of adhesives used | | | Light irradiation to adhesive | Composite resin used | Adhesion strength /MPa(S.D.) | |
|---|---|---|---|---|---|---|---|---|
| | | | | | | | Enamel | Dentin |
| Example 127 | P1 | L17 | + | D6 | Yes | Photo-curing type | 18.4 (2.1) | 21.2 (2.1) |
| Example 128 | P2 | L17 | + | D6 | Yes | Photo-curing type | 21.4 (2.8) | 19.3 (3.7) |
| Example 129 | P3 | L17 | + | D6 | No | Photo-curing type | 20.0 (3.5) | 21.3 (3.4) |
| Example 130 | P4 | L17 | + | D6 | Yes | Photo-curing type | 22.6 (3.2) | 22.5 (3.3) |
| Example 131 | P5 | L17 | + | D6 | Yes | Photo-curing type | 24.5 (3.8) | 20.6 (2.6) |
| Example 132 | P6 | L17 | + | D6 | Yes | Photo-curing type | 19.3 (4.0) | 19.4 (2.8) |
| Example 133 | P7 | L17 | + | D6 | Yes | Photo-curing type | 21.4 (2.9) | 18.9 (4.3) |
| Example 134 | P8 | L17 | + | D6 | Yes | Photo-curing type | 19.8 (5.4) | 17.6 (3.4) |
| Example 135 | P1 | L18 | + | D7 | No | Photo-curing type | 20.2 (3.4) | 19.4 (1.7) |
| Example 136 | P1 | L19 | + | D8 | No | Photo-curing type | 21.8 (3.3) | 21.4 (4.3) |
| Example 137 | P1 | L20 | + | D9 | No | Chemical curing type | 23.1 (3.8) | 18.5 (2.5) |
| Example 138 | P1 | L21 | + | D10 | No | Chemical curing type | 19.8 (3.9) | 19.4 (3.1) |
| Example 139 | P1 | L22 | + | D11 | No | Chemical curing type | 18.4 (4.1) | 21.1 (2.3) |
| Example 140 | P1 | L17 | + | D6 | No | Chemical curing type | 19.8 (3.2) | 21.2 (3.4) |
| Example 141 | P1 | L19 | + | D8 | No | Chemical curing type | 24.3 (4.5) | 20.1 (3.7) |
| Example 142 | P8 | L18 | + | D7 | Yes | Chemical curing type | 23.3 (4.9) | 19.8 (3.9) |
| Comparative Example 41 | P10 | L17 | + | D6 | Yes | Photo-curing type | 0 | 0 |
| Comparative Example 42 | P10 | L17 | + | D6 | Yes | Chemical curing type | 0 | 0 |
| Comparative Example 43 | P12 | L17 | + | D6 | Yes | Photo-curing type | 0 | 1.1(0.8) |
| Comparative Example 44 | P12 | L17 | + | D6 | Yes | Chemical curing type | 0 | 0 |
| Comparative Example 45 | P1 | L23 | + | D11 | Yes | Photo-curing type | 0 | 0 |
| Comparative Example 46 | P1 | L23 | + | D11 | Yes | Chemical curing type | 0 | 0 |

### Example 143

A pretreatment agent P1 was stored at 23°C for one day. Then, the P1 was stored at 50°C for one month. Using the stored P1 and an adhesive L1, adhesion strengths were measured in the same manner as in Example 98. As a result, in the case of one day storage at 23°C, the adhesion strength to enamel was 21.2 MPa (2.3) and the adhesion strength to dentin was 18.9 MPa (2.3). In the case of one month storage at 50°C, the adhesion strength to enamel was 18.2 MPa (3.4) and the adhesion strength to dentin was 17.3 MPa (4.1).

The storage of one month at 50°CZcorresponds to about 3 years of storage in refrigerator at 4°C and it is appreciated that the P1 has particularly excellent storage stability.

### Example 144

A pretreatment agent P2 was evaluated for storage stability in the same manner as in Example 143. As a result, when there was used the P2 stored at 23°C for one day, the adhesion strength to enamel was 19.0 MPa (1.9) and the adhesion strength to dentin was 20.2 MPa (2.6). When there was used the P2 stored at 50°C for one month, the adhesion strength to enamel was 9.4 MPa (4.5) and the adhesion strength to dentin was 10.7 MPa (5.2).

### Comparative Example 47

There was prepared a pretreatment agent consisting of two solutions, i.e. a solution a consisting of 2.0 g of PM, 0.1 g of Percumyl H, 0.5 g of Bis-GMA and 1.5 g of IPA and a solution b consisting of 0.3 g of PhBTEOA, 4.0 g of water and 1.6 g of IPA.

Separately, there was obtained a photo-curing type adhesive by compounding 0.05 g of camphorquinone and 0.05 g of ethyl p-dimethylaminobenzoate to a monomer solution consisting of 1.0 g of MAC-10, 3.5 g of Bis-GMA, 3.5 g of 3G and 2.0 g of HEMA.

The solution a and the solution b were mixed at a weight ratio of 41:59 right before use to obtain a pretreatment agent. Using this pretreatment agent, a pretreatment was conducted. The adhesion strength when using the above-mentioned photo-curing type adhesive was measured. As a result, the adhesion strength to enamel was 22.7 MPa (3.3) and the adhesion strength to dentin was 20.4 MPa (3.1).

A mixed solution of the solution a and the solution b was stored at 23°C for one day, causing gelling. Therefore, the mixed solution after storage was unusable as a pretreatment agent.

As understood from the comparison of Comparative Example 47 with Examples 143 and 144, the pretreatment agent in the present adhesive kit has high storage stability even when stored in one package.

Further, as understood from the comparison of Example 143 and Example 144, storage stability is increased strikingly by using an amine compound to control pH in a range of 1.5-3.

## Claims

1. A dental adhesive containing a chemical radical polymerization catalyst comprising
(i) an aryl borate compound,
(ii) a tetravalent or pentavalent vanadium compound selected from divanadium tetroxide, vanadium oxide acetylacetonate, vanadyl oxalate, vanadyl sulfate, oxo-bis(1-phenyl-1,3-butanedionate) vanadium, bis(maltolato) oxo vanadium, vanadium pentoxide, sodium metavanadate, and ammonium metavanadate; and
(iii) an acidic group-containing radical-polymerizable monomer which, when present in form of a 10 wt.-% aqueous solution or suspension, has a pH of ≤ 4.5, and which is a compound of formula (2): 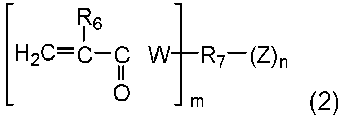 wherein
R₆ is H or methyl;
R₇ is a di- to hexavalent C₁₋₃₀-organic residue;
W is 0, S or NH;
Z is -COOH, -SO₃H, -O-P(=O)(OH)₂, -P(=O)(OH)₂ or -O-P(=O)(OH)(OR₈), wherein R₈ is C₁₋₁₀-alkyl or aryl having 6-14 ring carbon atoms, and the alkyl group or the aryl group may be substituted by halogen, hydroxyl, cyano, nitro, C₁₋₅-alkyl, C₂₋₅-alkenyl, C₂₋₅-alkynyl, C₁₋₅-alkoxyl, C₂₋₅-acyl or C₂₋₅-acyloxy;
m is an integer of 1-5;
n is an integer of 1-5; and (m+n) corresponds to the valence of R₇.

2. A dental restorative material comprising the dental adhesive of claim 1 and further
(iv) a radical-polymerizable monomer not containing an acidic group; and
(v) a filler.

3. A dental pretreatment agent comprising the dental adhesive of claim 1 and (vi) water.

4. A dental adhesive kit constituted by
(1) a dental pretreatment agent containing
(iii) an acidic group-containing radical-polymerizable monomer which, when present in form of a 10 wt.-% aqueous solution or suspension, has a pH of ≤ 4.5; and which is a compound of formula (2): 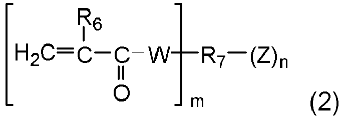 wherein
R₆ is H or methyl;
R₇ is a di- to hexavalent C₁₋₃₀-organic residue;
W is O, S or NH;
Z is -COOH, -SO₃H, -O-P(=O)(OH)₂, -P(=O)(OH)₂ or -O-P(=O)(OH)(OR₈), wherein R₈ is C₁₋₁₀-alkyl or aryl having 6-14 ring carbon atoms, and the alkyl group or the aryl group may be substituted by halogen, hydroxyl, cyano, nitro, C₁₋₅-alkyl, C₂₋₅-alkenyl, C₂₋₅-alkynyl, C₁₋₅-alkoxyl, C₂₋₅-acyl or C₂₋₅-acyloxy;
m is an integer of 1-5;
n is an integer of 1-5; and (m+n) corresponds to the valence of R₇; and
(vi) water, and
(2) a dental adhesive containing
(i) an aryl borate compound and
(iii) an acidic group-containing radical-polymerizable monomer which, when present in form of a 10 wt.-% aqueous solution or suspension, has a pH of ≤ 4.5; which is a compound of formula (2) as defined above;
and at least one of (1) and (2) contains (ii) tetravalent or pentavalent vanadium compound selected from divanadium tetroxide, vanadium oxide acetylacetonate, vanadyl oxalate, vanadyl sulfate, oxobis(1-phenyl-1,3-butanedionate) vanadium, bis(maltolato) oxo vanadium, vanadium pentoxide, sodium metavanadate, and ammonium metavanadate.

5. A dental adhesive kit constituted by
(1') a pretreatment agent containing
(ii) a tetravalent or pentavalent vanadium compound selected from divanadium tetroxide, vanadium oxide acetylacetonate, vanadyl oxalate, vanadyl sulfate, oxo-bis(1-phenyl-1,3-butanedionate) vanadium, bis(maltolato) oxo vanadium, vanadium pentoxide, sodium metavanadate, and ammonium metavanadate;
(iii) an acidic group-containing radical-polymerizable monomer which, when present in form of a 10 wt.-% aqueous solution or suspension, has a pH of ≤ 4.5; and which is a compound of formula (2): 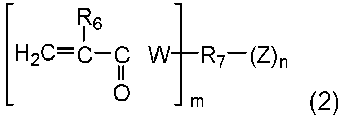 wherein
R₆ is H or methyl;
R₇ is a di- to hexavalent C₁₋₃₀-Organic residue;
W is O, S or NH;
Z is -COOH, -SO₃H, -O-P(=O)(OH)₂, -P(=O)(OH)₂ or -O-P(=O)(OH)(OR_{S}), wherein R₈ is C₁₋₁₀-alkyl or aryl having 6-14 ring carbon atoms, and the alkyl group or the aryl group may be substituted by halogen, hydroxyl, cyano, nitro, C₁₋₅-alkyl, C₂₋₅-alkenyl, C₂₋₅-alkynyl, C₁₋₅-alkoxyl, C₂₋₅-acyl or C₂₋₅-acyloxy;
m is an integer of 1-5;
n is an integer of 1-5; and (m+n) corresponds to the valence of R₇; and
(vi) water, and
(3) an adhesive containing
(i) an aryl borate compound,
(iv) a radical-polymerizable monomer not containing an acidic group,
(vii) an organic peroxide and
(viii) a photopolymerization catalyst.

6. The adhesive kit of Claim 5, wherein component (3) contains the components (ii) and (iii) of the catalyst of claim 1.

7. The adhesive kit of Claim 6, wherein component (3) is packed in two packages consisting of
(B1) a package containing the components (i), (vi), (vii) and (viii) defined in claim 6; and
(B2) a package containing the components (ii) and (iii).

8. The adhesive kit of Claim 5, wherein component (1') further contains an amine compound.

## Patentansprüche

1. Dentaladhäsiv, enthaltend einen chemischen radikalischen Polymerisationskatalysator, enthaltend
(i) eine Arylboratverbindung,
(ii) eine tetravalente oder pentavalente Vanadiumverbindung, ausgewählt aus Divanadiumtetroxid, Vanadiumoxidacetylacetonat, Vanadyloxalat, Vanadylsulfat, Oxo-bis(1-Phenyl-1,3-butandionat)vanadium, Bis(maltolato)oxovanadium, Vanadiumpentoxid, Natriummetavanadat und Ammoniummetavanadat und
(iii) ein radikalisch polymerisierbares Monomer mit einer sauren Gruppe, das, wenn es in Form einer 10 Gew-%-igen wässrigen Lösung oder Suspension vorhanden ist, einen pH von ≤ 4,5 hat und das eine Verbindung der Formel (2) ist: 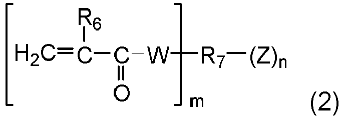
worin R₆ H oder Methyl ist,
R₇ ein di- bis hexavalenter C₁₋₃₀-organischer Rest ist,
W O, S oder NH ist,
Z -COOH, -SO₃H, -O-P(=O)(OH)₂, -P(=O)(OH)₂ oder -O-P(=O) (OH) (OR₈) ist, worin R₈ C₁₋₁₀-Alkyl oder Aryl mit 6-14 Ringkohlenstoffatomen ist und die Alkylgruppe oder Arylgruppe durch Halogen, Hydroxyl, Cyano, Nitro, C₁₋₅-Alkyl, C₂₋₅-Alkenyl, C₂₋₅-Alkinyl, C₁₋₅-Alkoxyl, C₂₋₅-Acyl oder C₂₋₅-Acyloxy substituiert sein kann,
m eine ganze Zahl von 1-5 ist,
n eine ganze Zahl von 1 - 5 ist und (m+n) der Valenz von R₇ entspricht.

2. Restaurierendes Zahnmaterial, enthaltend das Dentaladhäsiv gemäß Anspruch 1 und weiterhin
(iv) ein radikalisch polymerisierbares Monomer, das eine Säuregruppe enthält, und
(v) einen Füllstoff.

3. Zahlvorbehandlungsmittel, enthaltend das Dentaladhäsiv gemäß Anspruch 1 und (vi) Wasser.

4. Dentaladhäsivkit, konstituiert durch
(1) ein Zahlvorbehandlungsmittel, enthaltend
(iii) ein radikalisch polymerisierbares Monomer mit einer sauren Gruppe, das, wenn es in Form einer 10 Gew.-%-igen wässrigen Lösung oder Suspension vorhanden ist, einen pH von ≤ 4,5 hat und das eine Verbindung der Formel (2) ist: 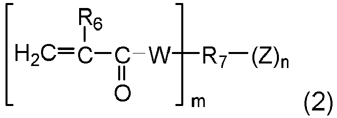
worin R₆ H oder Methyl ist,
R₇ ein di- bis hexavalenter C₁₋₃₀-organischer Rest ist,
W O, S oder NH ist,
Z -COOH, -SO₃H, -O-P(=O)(OH)₂, -P(=O) (OH)₂ oder -O-P(=O) (OH) (OR₈) ist, worin R₈ C₁₋₁₀-Alkyl oder Aryl mit 6-14 Ringkohlenstoffatomen ist und die Alkylgruppe oder Arylgruppe durch Halogen, Hydroxyl, Cyano, Nitro, C₁₋₅-Alkyl, C₂₋₅-Alkenyl, C₂₋₅-Alkinyl, C₁₋₅-Alkoxyl, C₂₋₅-Acyl oder C₂₋₅-Acyloxy substituiert sein kann,
m eine ganze Zahl von 1-5 ist,
n eine ganze Zahl von 1 - 5 ist und (m+n) der Valenz von R₇ entspricht und
(vi) Wasser, und
(2) ein Dentaladhäsiv, enthaltend
(i) eine Arylboratverbindung und
(iii) ein radikalisch polymerisierbares Monomer mit einer sauren Gruppe, das, wenn es in Form einer 10 Gew.-%-igen wässrigen Lösung oder Suspension vorhanden ist, einen pH von ≤ 4,5 hat, das eine Verbindung der Formel (2) wie oben definiert ist,
und worin zumindest eines von (1) und (2) (ii) eine tetravalente und/oder pentavalente Vanadiumverbindung enthält, ausgewählt aus Divanadiumtetroxid, Vanadiumoxidacetylacetonat, Vanadyloxalat, Vanadylsulfat, Oxobis(1-phenyl-1,3-butandionat)vanadium, Bis(maltolato)oxovanadium, Vanadiumpentoxid, Natriummetavanadat und Ammoniummetavanadat.

5. Dentaladhäsivkit, konstituiert durch
(1') ein Vorbehandlungsmittel, enthaltend
(ii) eine tetravalente und/oder pentavalente Vanadiumverbindung, ausgewählt aus Divanadiumtetroxid, Vanadiumoxidacetylacetonat, Vanadyloxalat, Vanadylsulfat, Oxobis(1-phenyl-1,3-butandionat) vanadium, Bis(maltolato)oxovanadium, Vanadiumpentoxid, Natriummetavanadat und Ammoniummetavanadat,
(iii) ein radikalisch polymerisierbares Monomer mit einer sauren Gruppe, das, wenn es in Form einer 10 Gew.-%-igen wässrigen Lösung oder Suspension vorhanden ist, einen pH von ≤ 4,5 hat und das eine Verbindung der Formel (2) ist: 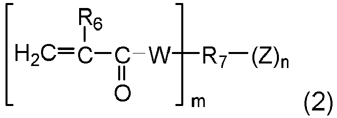
worin R₆ H oder Methyl ist,
R₇ ein di- bis hexavalenter C₁₋₃₀-organischer Rest ist,
W O, S oder NH ist,
Z -COOH, -SO₃H, -O-P(=O)(OH)₂, -P(=O) (OH)₂ oder -O-P(=O) (OH) (OR₈) ist, worin R₈ C₁₋₁₀-Alkyl oder Aryl mit 6-14 Ringkohlenstoffatomen ist und die Alkylgruppe oder Arylgruppe durch Halogen, Hydroxyl, Cyano, Nitro, C₁₋₅-Alkyl, C₂₋₅-Alkenyl, C₂₋₅-Alkinyl, C₁₋₅-Alkoxyl, C₂₋₅-Acyl oder C₂₋₅-Acyloxy substituiert sein kann,
m eine ganze Zahl von 1-5 ist,
n eine ganze Zahl von 1 bis 5 ist und (m+n) der Valenz von R₇ entspricht, und
(vi) Wasser und
(3) ein Adhäsiv, enthaltend
(i) eine Arylboratverbindung,
(iv) ein radikalisch polymerisierbares Monomer, das keine Säuregruppe enthält,
(vii) ein organisches Peroxid und
(viii) einen Fotopolymerisationskatalysator.

6. Adhäsivkit gemäß Anspruch 5, worin die Komponente (3) die Komponenten (ii) und (iii) des Katalysators gemäß Anspruch 1 enthält.

7. Adhäsivkit gemäß Anspruch 6, worin die Komponente (3) in zwei Packungen abgepackt ist, bestehend aus
(B1) einer Packung mit den Komponenten (i), (vi), (vii) und (viii), wie in Anspruch 6 definiert, und
(B2) eine Packung, die die Komponenten (ii) und (iii) enthält.

8. Adhäsivkit gemäß Anspruch 5, worin die Komponente (1') weiterhin eine Aminverbindung enthält.

## Revendications

1. Adhésif dentaire contenant un catalyseur de polymérisation radicalaire chimique comprenant
(i) un composé de borate d'aryle,
(ii) un composé de vanadium tétravalent ou pentavalent choisi parmi tétroxyde de divanadium, acétylacétonate d'oxyde de vanadium, oxalate de vanadyle, sulfate de vanadyle, oxo-bis(1-phényl-1,3-butanedionate) vanadium, bis(maltolato) oxo vanadium, pentoxyde de vanadium, métavanadate de sodium et métavanadate d'ammonium ;
(iii) un monomère polymérisable par voie radicalaire contenant un groupe acide qui, lorsqu'il est présent sous forme d'une solution ou suspension aqueuse à 10 % en poids, présente un pH ≤ 4,5, et qui est un composé de formule (2) : 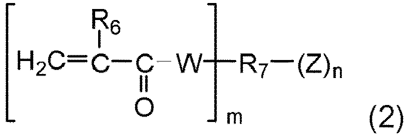 dans laquelle
R₆ est H ou méthyle ;
R₇ est un résidu organique en C₁₋₃₀ di- à hexavalent ;
West O, S ou NH ;
Z est -COOH, -SO₃H, -O-P(=O)(OH)₂, -P(=O)(OH)₂ ou -O-P(=O)(OH)(OR₈), dans lequel R₈ est alkyle ou aryle en C₁₋₁₀ présentant 6 à 14 atomes de carbone cycliques, et le groupe alkyle ou le groupe aryle peut être substitué par halogène, hydroxyle, cyano, nitro, alkyle en C₁₋₅, alcényle en C₂₋₅, alcynyle en C₂₋₅, alcoxyle en C₁₋₅, acyle en C₂₋₅ ou acyloxy en C₂₋₅ ;
m est un entier de 1 à 5 ;
n est un entier de 1 à 5 ; et (m + n) correspond à la valence de R₇.

2. Matériau de restauration dentaire comprenant l'adhésif dentaire selon la revendication 1 et en outre
(iv) un monomère polymérisable par voie radicalaire ne contenant pas de groupe acide ; et
(v) une charge.

3. Agent de prétraitement dentaire comprenant l'adhésif dentaire selon la revendication 1 et
(vi) de l'eau.

4. Ensemble adhésif dentaire constitué par
(1) un agent de prétraitement dentaire contenant
(iii) un monomère polymérisable par voie radicalaire contenant un groupe acide qui, lorsqu'il est présent sous forme d'une solution ou suspension aqueuse à 10 % en poids, présente un pH ≤ 4,5 ; et qui est un composé de formule (2) : 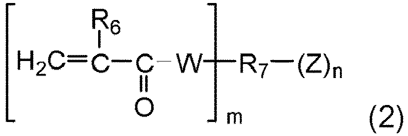 dans laquelle
R₆ est H ou méthyle ;
R₇ est un résidu organique en C₁₋₃₀ di- à hexavalent ;
W est O, S ou NH ;
Z est -COOH, -SO₃H, -O-P(=O)(OH)₂, -P(=O)(OH)₂ ou -O-P(=O)(OH)(OR₈), dans lequel R₈ est alkyle ou aryle en C₁₋₁₀ présentant 6 à 14 atomes de carbone cycliques, et le groupe alkyle ou le groupe aryle peut être substitué par halogène, hydroxyle, cyano, nitro, alkyle en C₁₋₅, alcényle en C₂₋₅, alcynyle en C₂₋₅, alcoxyle en C₁₋₅, acyle en C₂₋₅ ou acyloxy en C₂₋₅ ;
m est un entier de 1 à 5 ;
n est un entier de 1 à 5 ; et (m + n) correspond à la valence de R₇ ; et
(vi) de l'eau, et
(2) un adhésif dentaire contenant
(i) un composé de borate d'aryle et
(iii) un monomère polymérisable par voie radicalaire contenant un groupe acide qui, lorsqu'il est présent sous forme d'une solution ou suspension aqueuse à 10 % en poids, présente un pH ≤ 4,5 ; et qui est un composé de formule (2) telle que définie plus haut ;
et au moins un de (1) et (2) contient (ii) un composé de vanadium tétravalent ou pentavalent choisi parmi tétroxyde de divanadium, acétylacétonate d'oxyde de vanadium, oxalate de vanadyle, sulfate de vanadyle, oxo-bis(1-phényl-1,3-butanedionate) vanadium, bis(maltolato) oxo vanadium, pentoxyde de vanadium, métavanadate de sodium et métavanadate d'ammonium.

5. Ensemble adhésif dentaire constitué par
(1') un agent de prétraitement contenant
(ii) un composé de vanadium tétravalent ou pentavalent choisi parmi tétroxyde de divanadium, acétylacétonate d'oxyde de vanadium, oxalate de vanadyle, sulfate de vanadyle, oxo-bis(1-phényl-1,3-butanedionate) vanadium, bis(maltolato) oxo vanadium, pentoxyde de vanadium, métavanadate de sodium et métavanadate d'ammonium ;
(iii) un monomère polymérisable par voie radicalaire contenant un groupe acide qui, lorsqu'il est présent sous forme d'une solution ou suspension aqueuse à 10 % en poids, présente un pH ≤ 4,5 ; et qui est un composé de formule (2) : 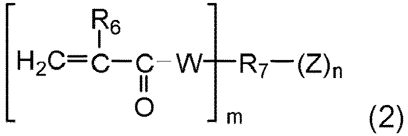 dans laquelle
R₆ est H ou méthyle ;
R₇ est un résidu organique en C₁₋₃₀ di- à hexavalent ;
W est O, Sou NH ;
Z est -COOH, -SO₃H, -O-P(=O)(OH)₂, -P(=O)(OH)₂ ou -O-P(=O)(OH)(OR₈), dans lequel R₈ est alkyle ou aryle en C₁₋₁₀ présentant 6 à 14 atomes de carbone cycliques, et le groupe alkyle ou le groupe aryle peut être substitué par halogène, hydroxyle, cyano, nitro, alkyle en C₁₋₅, alcényle en C₂₋₅, alcynyle en C₂₋₅, alcoxyle en C₁₋₅, acyle en C₂₋₅ ou acyloxy en C₂₋₅ ;
m est un entier de 1 à 5 ;
n est un entier de 1 à 5 ; et (m + n) correspond à la valence de R₇ ; et
(vi) de l'eau, et
(3) un adhésif contenant
(i) un composé de borate d'aryle,
(iv) un monomère polymérisable par voie radicalaire ne contenant pas de groupe acide,
(vii) un péroxyde organique et
(viii) un catalyseur de photopolymérisation.

6. Ensemble adhésif selon la revendication 5, dans lequel le composant (3) contient les composants (ii) et (iii) du catalyseur selon la revendication 1.

7. Ensemble adhésif selon la revendication 6, dans lequel le composant (3) est conditionné dans deux conditionnements consistant en
(B1) un conditionnement contenant les composants (i), (vi), (vii) et (viii) définis dans la revendication 6 ; et
(B2) un conditionnement contenant les composants (ii) et (iii).

8. Ensemble adhésif selon la revendication 5, dans lequel le composant (1') contient en outre un composé d'amine.
